# EUROPEAN PATENT APPLICATION

(11) **EP 4 636 085 A2**
(43) Date of publication of application: **22.10.2025**
(21) Application number: 25166107.0
(22) Date of filing: 03.03.2016
(51) Int. Cl.: C12N 15/113, A61K 31/7125, A61K 48/00, A61P 25/00

(54) **COMPOSITIONS FOR MODULATING MECP2 EXPRESSION**

(30) Priority: 03.03.2015 US 201562127682 P
(62) Divisional of application: 16759530.5
(71) Applicant: Ionis Pharmaceuticals, Inc., Carlsbad, CA 92010 (US)
(72) Inventor: FREIER, Susan M, Carlsbad, CA 92010 (US)
(74) Representative: Carpmaels & Ransford LLP

(57) **Abstract**

Disclosed herein are compounds and methods for decreasing MECP2 mRNA and protein expression. Such compounds and methods are useful to treat, prevent, or ameliorate MECP2 associated disorders and syndromes. Such MECP2 associated disorders include MECP2 duplication syndrome.

## Description

### Statement of Government Support

This invention was made with government support under P30HD024064 and 5R01NS057819 awarded by the National Institutes of Health. The government has certain rights in the invention.

### Sequence Listing

The present application is being filed along with a Sequence Listing in electronic format. The Sequence Listing is provided as a file entitled BIOL0264WOSEQ_ST25.txt created March 2, 2016, which is 180 Kb in size. The information in the electronic format of the sequence listing is incorporated herein by reference in its entirety.

### Field

Provided are compositions and methods for modulating expression of methyl CpG binding protein 2 (MECP2) mRNA and protein in an animal. Such methods are useful to treat, prevent, or ameliorate neurological disorders, including MECP2 duplication syndrome, by reducing expression and amount of MECP2 mRNA and protein in an animal.

### Background

Methyl CpG binding protein 2 (MECP2) is located on chromosome Xq28 and plays a fundamental role in epigenetics, controlling chromatin states, and expression of thousands of genes (Chahrour et al. , Science, 2008, 320:1224-1229; Nan et al., Nature, 1998, 393:386-389; Jones et al., Nat. Genet., 1998, 19:187-191). MECP2 expression must be maintained within a fairly narrow range to assure proper gene expression and neuronal function (Nan et al., Nature, 1988, 393:386-389). MECP2 duplication syndrome caused by overexpression of MECP2 is characterized by autism, intellectual disability, motor dysfunction, anxiety, epilepsy, recurrent respiratory tract infections, and early death, typically in males (Ramocki et al., Am J Med Genet A, 2010, 152A:1079-1088). Underexpression of MECP2 is associated with Rett Syndrome, which is characterized by normal early growth and development followed by a slowing of development, loss of purposeful use of the hands, distinctive hand movements, slowed brain and head growth, problems with walking, seizures, and intellectual disability, typically in females (Weaving et al., J Med Genet, 2005, 42:1-7).

Currently there is a lack of acceptable options for treating such neurological disorders. It is therefore an object herein to provide compositions for the treatment of such disorders.

### Summary

Provided herein are compositions and methods for modulating expression and amount of methyl CpG binding protein 2 (MECP2) mRNA and protein. In certain embodiments, compounds useful for modulating expression and amount of MECP2 mRNA and protein are antisense compounds. In certain embodiments, the antisense compounds are modified antisense oligonucleotides. In certain embodiments, the antisense compounds are single-stranded antisense oligonucleotides. In certain embodiments, the antisense compounds are not siRNA compounds.

In certain embodiments, modulation can occur in a cell or tissue. In certain embodiments, the cell or tissue is in an animal. In certain embodiments, the animal is a human. In certain embodiments, MECP2 mRNA levels are reduced. In certain embodiments, MECP2 protein levels are reduced. Such reduction can occur in a time-dependent manner or in a dose-dependent manner.

Also provided are compositions useful for preventing, treating, and ameliorating disorders and syndromes associated with MECP2 overexpression. In certain embodiments, a disorder associated with MECP2 overexpression is a neurological disorder. In certain embodiments, the neurological disorder is MECP2 duplication syndrome. In certain embodiments, MECP2 duplication syndrome is characterized by having additional copies of MECP2, which leads to overexpression of MECP2.

In certain embodiments, MECP2 duplication syndrome is characterized by autism, intellectual disability, motor dysfunction, anxiety, epilepsy, recurrent respiratory tract infections, and early death. In certain embodiments, MECP2 duplication syndrome is inherited in an X-linked pattern.

In certain embodiments, methods of treatment include administering a MECP2 antisense compound to an individual in need thereof. In certain embodiments, methods of treatment include administering a MECP2 modified antisense oligonucleotide to an individual in need thereof.

In certain embodiments, MECP2 levels are reduced sufficiently to prevent, treat, and ameliorate symptoms of MECP2 duplication syndrome, but not enough to cause symptoms of Rett Syndrome.

### Brief Description of the Drawings

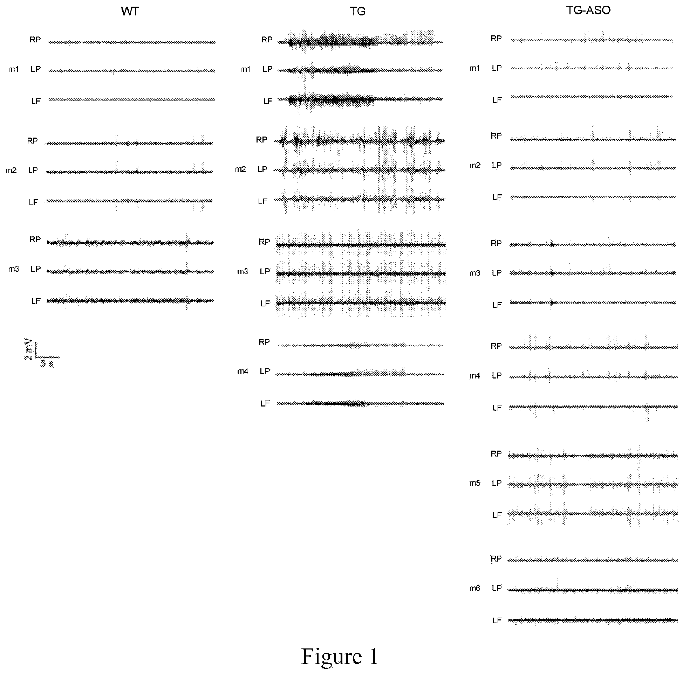

**Figure 1** displays representative EEG traces for WT mice, MECP2-TG1 mice without Isis No. 628785 treatment, and MECP2-TG1 mice that received treatment with Isis No. 628785.

### Detailed Description

It is to be understood that both the foregoing general description and the following detailed description are exemplary and explanatory only and are not restrictive of the invention, as claimed. Herein, the use of the singular includes the plural unless specifically stated otherwise. As used herein, the use of "or" means "and/or" unless stated otherwise. Additionally, as used herein, the use of "and" means "and/or" unless stated otherwise. Furthermore, the use of the term "including" as well as other forms, such as "includes" and "included", is not limiting. Also, terms such as "element" or "component" encompass both elements and components comprising one unit and elements and components that comprise more than one subunit, unless specifically stated otherwise.

The section headings used herein are for organizational purposes only and are not to be construed as limiting the subject matter described. All documents, or portions of documents, cited in this disclosure, including, but not limited to, patents, patent applications, published patent applications, articles, books, treatises, and GENBANK Accession Numbers and associated sequence information obtainable through databases such as National Center for Biotechnology Information (NCBI) and other data referred to throughout in the disclosure herein are hereby expressly incorporated by reference for the portions of the document discussed herein, as well as in their entirety.

### Definitions

Unless specific definitions are provided, the nomenclature utilized in connection with, and the procedures and techniques of, analytical chemistry, synthetic organic chemistry, and medicinal and pharmaceutical chemistry described herein are those well known and commonly used in the art. Standard techniques may be used for chemical synthesis, and chemical analysis.

Unless otherwise indicated, the following terms have the following meanings:
"2'-O-methoxyethyl" (also 2'-MOE and 2'-OCH₂CH₂-OCH₃ and MOE) refers to an O-methoxyethyl modification of the 2' position of a furanose ring. A 2'-O-methoxyethyl modified sugar is a modified sugar.
"2'-MOE nucleoside" (also 2'-O-methoxyethyl nucleoside) means a nucleoside comprising a 2'-MOE modified sugar moiety.
"2'-substituted nucleoside" means a nucleoside comprising a substituent at the 2'-position of the furanose ring other than H or OH. In certain embodiments, 2' substituted nucleosides include nucleosides with bicyclic sugar modifications.
"5-methylcytosine" means a cytosine modified with a methyl group attached to the 5 position. A 5-methylcytosine is a modified nucleobase.

"Administered concomitantly" refers to the co-administration of two pharmaceutical agents in any manner in which the pharmacological effects of both are manifest in the patient at the same time. Concomitant administration does not require that both pharmaceutical agents be administered in a single pharmaceutical composition, in the same dosage form, or by the same route of administration. The effects of both pharmaceutical agents need not manifest themselves at the same time. The effects need only be overlapping for a period of time and need not be coextensive.

"Administering" means providing a pharmaceutical agent to an animal, and includes, but is not limited to administering by a medical professional and self-administering.

"Amelioration" refers to a lessening, slowing, stopping, or reversing of at least one indicator of the severity of a syndrome or condition. The severity of indicators may be determined by subjective or objective measures, which are known to those skilled in the art.

"Animal" refers to a human or non-human animal, including, but not limited to, mice, rats, rabbits, dogs, cats, pigs, and non-human primates, including, but not limited to, monkeys and chimpanzees.

"Antibody" refers to a molecule characterized by reacting specifically with an antigen in some way, where the antibody and the antigen are each defined in terms of the other. Antibody may refer to a complete antibody molecule or any fragment or region thereof, such as the heavy chain, the light chain, Fab region, and Fc region.

"Antisense activity" means any detectable or measurable activity attributable to the hybridization of an antisense compound to its target nucleic acid. **In** certain embodiments, antisense activity is a decrease in the amount or expression of a target nucleic acid or protein encoded by such target nucleic acid.

"Antisense compound" means an oligomeric compound that is capable of undergoing hybridization to a target nucleic acid through hydrogen bonding. Examples of antisense compounds include single-stranded and double-stranded compounds, such as, antisense oligonucleotides, siRNAs, shRNAs, ssRNAs, and occupancy-based compounds.

"Antisense inhibition" or "inhibition" means reduction of target nucleic acid levels in the presence of an antisense compound complementary to a target nucleic acid compared to target nucleic acid levels or in the absence of the antisense compound.

"Antisense mechanisms" are all those mechanisms involving hybridization of a compound with a target nucleic acid, wherein the outcome or effect of the hybridization is either target degradation or target occupancy with concomitant stalling of the cellular machinery involving, for example, transcription or splicing.

"Antisense oligonucleotide" means a single-stranded oligonucleotide having a nucleobase sequence that permits hybridization to a corresponding segment of a target nucleic acid.

"Base complementarity" refers to the capacity for the precise base pairing of nucleobases of an antisense oligonucleotide with corresponding nucleobases in a target nucleic acid (i.e., hybridization), and is mediated by Watson-Crick, Hoogsteen or reversed Hoogsteen hydrogen binding between corresponding nucleobases.

"Bicyclic sugar" means a furanose ring modified by the bridging of two atoms. A bicyclic sugar is a modified sugar.

"Bicyclic nucleoside" (also BNA) means a nucleoside having a sugar moiety comprising a bridge connecting two carbon atoms of the sugar ring, thereby forming a bicyclic ring system. In certain embodiments, the bridge connects the 4'-carbon and the 2'-carbon of the sugar ring.

"Cap structure" or "terminal cap moiety" means chemical modifications, which have been incorporated at either terminus of an antisense compound.

"cEt" or "constrained ethyl" means a bicyclic nucleoside having a sugar moiety comprising a bridge connecting the 4'-carbon and the 2'-carbon, wherein the bridge has the formula: 4'-CH(CH₃)-O-2'.

"Constrained ethyl nucleoside" (also cEt nucleoside) means a nucleoside comprising a bicyclic sugar moiety comprising a 4'-CH(CH₃)-O-2' bridge.

"Chemically distinct region" refers to a region of an antisense compound that is in some way chemically different than another region of the same antisense compound. For example, a region having 2'-O-methoxyethyl nucleosides is chemically distinct from a region having nucleosides without 2'-O-methoxyethyl modifications.

"Chimeric antisense compound" means an antisense compound that has at least two chemically distinct regions, each position having a plurality of subunits.

"Co-administration" means administration of two or more pharmaceutical agents to an individual. The two or more pharmaceutical agents may be in a single pharmaceutical composition, or may be in separate pharmaceutical compositions. Each of the two or more pharmaceutical agents may be administered through the same or different routes of administration. Co-administration encompasses parallel or sequential administration.

"Complementarity" means the capacity for pairing between nucleobases of a first nucleic acid and a second nucleic acid.

"Comprise," "comprises," and "comprising" will be understood to imply the inclusion of a stated step or element or group of steps or elements but not the exclusion of any other step or element or group of steps or elements.

"Contiguous nucleobases" means nucleobases immediately adjacent to each other.

"Designing" or "designed to" refer to the process of designing an oligomeric compound that specifically hybridizes with a selected nucleic acid molecule.

"Diluent" means an ingredient in a composition that lacks pharmacological activity, but is pharmaceutically necessary or desirable. For example, in drugs that are injected, the diluent may be a liquid, e.g. saline solution.

"Dose" means a specified quantity of a pharmaceutical agent provided in a single administration, or in a specified time period. In certain embodiments, a dose may be administered in one, two, or more boluses, tablets, or injections. For example, in certain embodiments where subcutaneous administration is desired, the desired dose requires a volume not easily accommodated by a single injection, therefore, two or more injections may be used to achieve the desired dose. In certain embodiments, the pharmaceutical agent is administered by infusion over an extended period of time or continuously. Doses may be stated as the amount of pharmaceutical agent per hour, day, week, or month.

"Effective amount" in the context of modulating an activity or of treating or preventing a condition means the administration of that amount of pharmaceutical agent to an individual in need of such modulation, treatment, or prophylaxis, either in a single dose or as part of a series, that is effective for modulation of that effect, or for treatment or prophylaxis or improvement of that condition. The effective amount may vary among individuals depending on the health and physical condition of the individual to be treated, the taxonomic group of the individuals to be treated, the formulation of the composition, assessment of the individual's medical condition, and other relevant factors.

"Efficacy" means the ability to produce a desired effect.

"Expression" includes all the functions by which a gene's coded information is converted into structures present and operating in a cell. Such structures include, but are not limited to the products of transcription and translation.

"Fully complementary" or "100% complementary" means each nucleobase of a first nucleic acid has a complementary nucleobase in a second nucleic acid. In certain embodiments, a first nucleic acid is an antisense compound and a target nucleic acid is a second nucleic acid.

"Gapmer" means a chimeric antisense compound in which an internal region having a plurality of nucleosides that support RNase H cleavage is positioned between external regions having one or more nucleosides, wherein the nucleosides comprising the internal region are chemically distinct from the nucleoside or nucleosides comprising the external regions. The internal region may be referred to as a "gap" and the external regions may be referred to as the "wings."

"Hotspot region" is a range of nucleobases on a target nucleic acid amenable to antisense compounds for reducing the amount or activity of the target nucleic acid as demonstrated in the examples hereinbelow.

"Hybridization" means the annealing of complementary nucleic acid molecules. **In** certain embodiments, complementary nucleic acid molecules include, but are not limited to, an antisense compound and a target nucleic acid. In certain embodiments, complementary nucleic acid molecules include, but are not limited to, an antisense oligonucleotide and a nucleic acid target.

"Identifying an animal having a MECP2 associated disorder" means identifying an animal having been diagnosed with a MECP2 associated disorder or predisposed to develop a MECP2 associated disorder. Individuals predisposed to develop a MECP2 associated disorder include those having one or more risk factors for developing a MECP2 associated disorder, including, having a personal or family history or genetic predisposition to one or more MECP2 associated disorders. Such identification may be accomplished by any method including evaluating an individual's medical history and standard clinical tests or assessments, such as genetic testing.

"Immediately adjacent" means there are no intervening elements between the immediately adjacent elements.

"Individual" means a human or non-human animal selected for treatment or therapy.

"Inhibiting MECP2" means reducing the level or expression of a MECP2 mRNA and/or protein. In certain embodiments, MECP2 mRNA and/or protein levels are inhibited in the presence of an antisense compound targeting MECP2, including an antisense oligonucleotide targeting MECP2, as compared to expression of MECP2 mRNA and/or protein levels in the absence of a MECP2 antisense compound, such as an antisense oligonucleotide.

"Inhibiting the expression or activity" refers to a reduction or blockade of the expression or activity and does not necessarily indicate a total elimination of expression or activity.

"Internucleoside linkage" refers to the chemical bond between nucleosides.

"Linked nucleosides" means adjacent nucleosides linked together by an internucleoside linkage. "MECP2 antisense compound" means an antisense compound targeting MECP2.

"MECP2" means the mammalian gene methyl CpG binding protein 2 (MECP2), including the human gene methyl CpG binding protein 2 (MECP2). Human MECP2 has been mapped to human chromosome Xq28.

"MECP2 associated disorder" means any disorder or syndrome associated with any MECP2 nucleic acid or expression product thereof. Such disorders may include a neurological disorder. Such neurological disorders may include MECP2 duplication syndrome.

"MECP2 nucleic acid" means any nucleic acid encoding MECP2. For example, in certain embodiments, a MECP2 nucleic acid includes a DNA sequence encoding MECP2, an RNA sequence transcribed from DNA encoding MECP2 (including genomic DNA comprising introns and exons), and an mRNA sequence encoding MECP2.

"MECP2 mRNA" means any messenger RNA expression product of a DNA sequence encoding MECP2.

"MECP2 protein" means the polypeptide expression product of a MECP2 nucleic acid.

"Mismatch" or "non-complementary nucleobase" refers to the case when a nucleobase of a first nucleic acid is not capable of pairing with the corresponding nucleobase of a second or target nucleic acid.

"Modified internucleoside linkage" refers to a substitution or any change from a naturally occurring internucleoside bond (*i.e.,* a phosphodiester internucleoside bond).

"Modified nucleobase" means any nucleobase other than adenine, cytosine, guanine, thymidine, or uracil. An "unmodified nucleobase" means the purine bases adenine (A) and guanine (G), and the pyrimidine bases thymine (T), cytosine (C), and uracil (U).

"Modified nucleoside" means a nucleoside having, independently, a modified sugar moiety and/or modified nucleobase.

"Modified nucleotide" means a nucleotide having, independently, a modified sugar moiety, modified internucleoside linkage, and/or modified nucleobase.

"Modified antisense oligonucleotide" means an oligonucleotide comprising at least one modified internucleoside linkage, modified sugar, and/or modified nucleobase.

"Modified sugar" means substitution and/or any change from a natural sugar moiety.

"Monomer" means a single unit of an oligomer. Monomers include, but are not limited to, nucleosides and nucleotides, whether naturally occurring or modified.

"Motif" means the pattern of unmodified and modified nucleosides in an antisense compound.

"Natural sugar moiety" means a sugar moiety found in DNA (2'-H) or RNA (2'-OH).

"Naturally occurring internucleoside linkage" means a 3' to 5' phosphodiester linkage.

"Non-complementary nucleobase" refers to a pair of nucleobases that do not form hydrogen bonds with one another or otherwise support hybridization.

"Nucleic acid" refers to molecules composed of monomeric nucleotides. A nucleic acid includes, but is not limited to, ribonucleic acids (RNA), deoxyribonucleic acids (DNA), single-stranded nucleic acids, double-stranded nucleic acids, small interfering ribonucleic acids (siRNA), and microRNAs (miRNA).

"Nucleobase" means a heterocyclic moiety capable of pairing with a base of another nucleic acid.

"Nucleobase complementarity" refers to a nucleobase that is capable of base pairing with another nucleobase. For example, in DNA, adenine (A) is complementary to thymine (T). For example, in RNA, adenine (A) is complementary to uracil (U). In certain embodiments, complementary nucleobase refers to a nucleobase of an antisense compound that is capable of base pairing with a nucleobase of its target nucleic acid. For example, if a nucleobase at a certain position of an antisense compound is capable of hydrogen bonding with a nucleobase at a certain position of a target nucleic acid, then the position of hydrogen bonding between the oligonucleotide and the target nucleic acid is considered to be complementary at that nucleobase pair.

"Nucleobase sequence" means the order of contiguous nucleobases independent of any sugar, linkage, and/or nucleobase modification.

"Nucleoside" means a nucleobase linked to a sugar.

"Nucleoside mimetic" includes those structures used to replace the sugar or the sugar and the base and not necessarily the linkage at one or more positions of an oligomeric compound such as for example nucleoside mimetics having morpholino, cyclohexenyl, cyclohexyl, tetrahydropyranyl, bicyclo, or tricyclo sugar mimetics, *e.g*., non furanose sugar units. Nucleotide mimetic includes those structures used to replace the nucleoside and the linkage at one or more positions of an oligomeric compound such as for example peptide nucleic acids or morpholinos (morpholinos linked by -N(H)-C(=O)-O- or other non-phosphodiester linkage). Sugar surrogate overlaps with the slightly broader term nucleoside mimetic but is intended to indicate replacement of the sugar unit (furanose ring) only. The tetrahydropyranyl rings provided herein are illustrative of an example of a sugar surrogate wherein the furanose sugar group has been replaced with a tetrahydropyranyl ring system. "Mimetic" refers to groups that are substituted for a sugar, a nucleobase, and/or internucleoside linkage. Generally, a mimetic is used in place of the sugar or sugar-internucleoside linkage combination, and the nucleobase is maintained for hybridization to a selected target.

"Nucleotide" means a nucleoside having a phosphate group covalently linked to the sugar portion of the nucleoside.

"Off-target effect" refers to an unwanted or deleterious biological effect associated with modulation of RNA or protein expression of a gene other than the intended target nucleic acid.

"Oligomeric compound" or "oligomer" means a polymer of linked monomeric subunits which is capable of hybridizing to at least a region of a nucleic acid molecule.

"Oligonucleotide" means a polymer of linked nucleosides each of which can be modified or unmodified, independent one from another.

"Parenteral administration" means administration through injection (e.g., bolus injection) or infusion. Parenteral administration includes subcutaneous administration, intravenous administration, intramuscular administration, intraarterial administration, intraperitoneal administration, or intracranial administration, e.g., intrathecal or intracerebroventricular administration.

"Peptide" means a molecule formed by linking at least two amino acids by amide bonds. Without limitation, as used herein, peptide refers to polypeptides and proteins.

"Pharmaceutical agent" means a substance that provides a therapeutic benefit when administered to an individual. For example, in certain embodiments, an antisense oligonucleotide targeted to MECP2 is a pharmaceutical agent.

"Pharmaceutical composition" means a mixture of substances suitable for administering to an individual. For example, a pharmaceutical composition may comprise an antisense oligonucleotide and a sterile aqueous solution.

"Pharmaceutically acceptable derivative" encompasses pharmaceutically acceptable salts, conjugates, prodrugs or isomers of the compounds described herein.

"Pharmaceutically acceptable salts" means physiologically and pharmaceutically acceptable salts of antisense compounds, *i.e.,* salts that retain the desired biological activity of the parent oligonucleotide and do not impart undesired toxicological effects thereto.

"Phosphorothioate linkage" means a linkage between nucleosides where the phosphodiester bond is modified by replacing one of the non-bridging oxygen atoms with a sulfur atom. A phosphorothioate linkage is a modified internucleoside linkage.

"Portion" means a defined number of contiguous (*i.e.,* linked) nucleobases of a nucleic acid. In certain embodiments, a portion is a defined number of contiguous nucleobases of a target nucleic acid. In certain embodiments, a portion is a defined number of contiguous nucleobases of an antisense compound.

"Prevent" or "preventing" refers to delaying or forestalling the onset or development of a disorder or syndrome for a period of time from minutes to days, weeks to months, or indefinitely.

"Prodrug" means a therapeutic agent that is prepared in an inactive form that is converted to an active form (i.e., drug) within the body or cells thereof by the action of endogenous enzymes or other chemicals and/or conditions.

"Prophylactically effective amount" refers to an amount of a pharmaceutical agent that provides a prophylactic or preventative benefit to an animal.

"Region" is defined as a portion of the target nucleic acid having at least one identifiable structure, function, or characteristic.

"Ribonucleotide" means a nucleotide having a hydroxy at the 2' position of the sugar portion of the nucleotide. Ribonucleotides may be modified with any of a variety of substituents.

"Salt" means a physiologically and pharmaceutically acceptable salt(s) of antisense compounds, i.e., salts that retain the desired biological activity of the parent oligonucleotide and do not impart undesired toxicological effects thereto.

"Segments" are defined as smaller or sub-portions of regions within a target nucleic acid.

"Shortened" or "truncated" versions of antisense oligonucleotides taught herein have one, two or more nucleosides deleted.

"Side effects" means physiological responses attributable to a treatment other than desired effects. In certain embodiments, side effects include, without limitation, injection site reactions, liver function test abnormalities, renal function abnormalities, liver toxicity, renal toxicity, central nervous system abnormalities, and myopathies.

"Single-stranded antisense oligonucleotide" means an oligonucleotide which is not hybridized to a complementary strand. A single-stranded antisense oligonucleotide is not a siRNA.

"Sites" as used herein, are defined as unique nucleobase positions within a target nucleic acid.

"Slows progression" means decrease in the development of the disorder or syndrome.

"Specifically hybridizable" refers to an antisense compound having a sufficient degree of complementarity between an antisense oligonucleotide and a target nucleic acid to induce a desired effect, while exhibiting minimal or no effects on non-target nucleic acids under conditions in which specific binding is desired, *i.e.,* under physiological conditions in the case of *in vivo* assays and therapeutic treatments.

"Standard cell assay" means the assay described in Example 1 and reasonable variations thereof.

"Stringent hybridization conditions" or "stringent conditions" refer to conditions under which an oligomeric compound will hybridize to its target sequence, but to a minimal number of other sequences.

"Targeting" or "targeted" means the process of design and selection of an antisense compound that will specifically hybridize to a target nucleic acid and induce a desired effect.

"Target nucleic acid," "target RNA," and "target RNA transcript" and "nucleic acid target" all mean a nucleic acid capable of being targeted by antisense compounds. In certain embodiments, the target nucleic acid is a MECP2 nucleic acid.

"Target region" means a portion of a target nucleic acid to which one or more antisense compounds is targeted.

"Target segment" means the sequence of nucleotides of a target nucleic acid to which an antisense compound is targeted. "5' target site" refers to the 5'-most nucleotide of a target segment. "3' target site" refers to the 3'-most nucleotide of a target segment.

"Therapeutically effective amount" means an amount of a pharmaceutical agent that provides a therapeutic benefit to an individual.

"Treat" or "treating" or "treatment" refers administering a composition to effect an alteration or improvement of the disorder or syndrome.

"Unmodified nucleobases" mean the purine bases adenine (A) and guanine (G), and the pyrimidine bases thymine (T), cytosine (C) and uracil (U).

"Unmodified nucleotide" means a nucleotide composed of naturally occuring nucleobases, sugar moieties, and internucleoside linkages. In certain embodiments, an unmodified nucleotide is an RNA nucleotide (*i.e.* β-D-ribonucleosides) or a DNA nucleotide (*i.e.* β-D-deoxyribonucleoside).

"Wing segment" means a plurality of nucleosides modified to impart to an oligonucleotide properties such as enhanced inhibitory activity, increased binding affinity for a target nucleic acid, or resistance to degradation by in vivo nucleases.

### Certain Embodiments

Certain embodiments provide methods, compounds, and compositions for inhibiting MECP2 mRNA and protein expression. Certain embodiments provide methods, compounds, and compositions for decreasing MECP2 mRNA and protein levels.

Certain embodiments provide antisense compounds targeted to a MECP2 nucleic acid. In certain embodiments, the MECP2 nucleic acid is the sequence set forth in GENBANK Accession No. NM_004992.3 (incorporated herein as SEQ ID NO: 2) and the complement of GENBANK Accession No. NT_167198.1 truncated from nucleotides 4203000 to 4283000 (incorporated herein as SEQ ID NO: 1).

Certain embodiments provide methods, compounds, and compositions for the treatment, prevention, or amelioration of disorders and syndromes associated with MECP2 in an individual in need thereof. Also contemplated are methods for the preparation of a medicament for the treatment, prevention, or amelioration of a disorder or syndrome associated with MECP2. MECP2 associated disorders and syndromes include neurological disorders. In certain embodiments, MECP2 associated disorders include MECP2 duplication syndrome.

The present disclosure provides the following non-limiting numbered embodiments:
Embodiment 1. A compound, comprising a modified antisense oligonucleotide consisting of 12 to 30 linked nucleosides and having a nucleobase sequence comprising at least 8, at least 9, at least 10, at least 11, at least 12, at least 13, at least 14, at least 15, at least 16, at least 17, at least 18, at least 19, or at least 20 consecutive nucleobases of any of the nucleobase sequences of SEQ ID NOs: 16-327.
Embodiment 2. The compound of embodiment 2, wherein the nucleobase sequence of the modified antisense oligonucleotide is at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% complementary to SEQ ID NO: 1 or SEQ ID NO: 2.
Embodiment 3. The compound of any preceding claim, consisting of a single-stranded modified antisense oligonucleotide.
Embodiment 4. The compound of any preceding embodiment, wherein at least one internucleoside linkage is a modified internucleoside linkage.
Embodiment 5. The compound of embodiment 4, wherein at least one modified internucleoside linkage is a phosphorothioate internucleoside linkage.
Embodiment 6. The compound of embodiment 4, wherein each modified internucleoside linkage is a phosphorothioate internucleoside linkage.
Embodiment 7. The compound of any preceding embodiment, wherein at least one internucleoside linkage is a phosphodiester internucleoside linkage.
Embodiment 8. The compound of any preceding embodiment, wherein at least one internucleoside linkage is a phosphorothioate linkage and at least one internucleoside linkage is a phosphodiester linkage.
Embodiment 9. The compound of any preceding embodiment, wherein at least one nucleoside comprises a modified nucleobase.
Embodiment 10. The compound of embodiment 9, wherein the modified nucleobase is a 5-methylcytosine.
Embodiment 11. The compound of any preceding embodiment, wherein at least one nucleoside of the modified antisense oligonucleotide comprises a modified sugar.
Embodiment 12. The compound of embodiment 11, wherein the at least one modified sugar is a bicyclic sugar.
Embodiment 13. The compound of embodiment 12, wherein the bicyclic sugar comprises a 4'-CH(R)-O-2' bridge wherein R is, independently, H, C₁-C₁₂ alkyl, or a protecting group.
Embodiment 14. The compound of embodiment 13, wherein R is methyl.
Embodiment 15. The compound of embodiment 13, wherein R is H.
Embodiment 16. The compound of embodiment 11, wherein the at least one modified sugar comprises a 2'-O-methoxyethyl group.
Embodiment 17. The compound of any preceding embodiment, wherein the modified antisense oligonucleotide comprises:
   a gap segment consisting of 10 linked deoxynucleosides;
   a 5' wing segment consisting of 5 linked nucleosides; and
   a 3' wing segment consisting of 5 linked nucleosides;
   wherein the gap segment is positioned between the 5' wing segment and the 3' wing segment and wherein each nucleoside of each wing segment comprises a modified sugar.
Embodiment 18. The compound of any preceding embodiment, wherein the modified antisense oligonucleotide consists of 20 linked nucleosides.
Embodiment 19. A composition comprising the compound of any preceding embodiment or salt thereof and at least one of a pharmaceutically acceptable carrier or diluent.
Embodiment 20. A method comprising administering to an animal the compound or composition of any preceding embodiment.
Embodiment 21. The method of embodiment 20, wherein the animal is a human.
Embodiment 22. The method of embodiment 20, wherein administering the compound prevents, treats, ameliorates, or slows progression of a MECP2 associated disorder or syndrome.
Embodiment 23. The method of embodiment 22, wherein the disease, disorder or condition is MECP2 duplication syndrome.
Embodiment 24. Use of the compound or composition of any preceding embodiment for the manufacture of a medicament for treating a neurological disorder.

### Antisense Compounds

Oligomeric compounds include, but are not limited to, oligonucleotides, oligonucleosides, oligonucleotide analogs, oligonucleotide mimetics, antisense compounds, antisense oligonucleotides, and siRNAs. An oligomeric compound may be "antisense" to a target nucleic acid, meaning that is is capable of undergoing hybridization to a target nucleic acid through hydrogen bonding.

In certain embodiments, an antisense compound has a nucleobase sequence that, when written in the 5' to 3' direction, comprises the reverse complement of the target segment of a target nucleic acid to which it is targeted. In certain such embodiments, an antisense oligonucleotide has a nucleobase sequence that, when written in the 5' to 3' direction, comprises the reverse complement of the target segment of a target nucleic acid to which it is targeted.

In certain embodiments, an antisense compound targeted to a target nucleic acid is 12 to 30 subunits in length. In certain embodiments, an antisense compound targeted to a target nucleic acid is 12 to 25 subunits in length. In certain embodiments, an antisense compound targeted to a target nucleic acid is 12 to 22 subunits in length. In certain embodiments, an antisense compound targeted to a target nucleic acid is 14 to 20 subunits in length. In certain embodiments, an antisense compound targeted to a target nucleic acid is 15 to 25 subunits in length. In certain embodiments, an antisense compound targeted to a target nucleic acid is 18 to 22 subunits in length. In certain embodiments, an antisense compound targeted to a target nucleic acid is 19 to 21 subunits in length. In certain embodiments, the antisense compound is 8 to 80, 12 to 50, 13 to 30, 13 to 50, 14 to 30, 14 to 50, 15 to 30, 15 to 50, 16 to 30, 16 to 50, 17 to 30, 17 to 50, 18 to 30, 18 to 50, 19 to 30, 19 to 50, or 20 to 30 linked subunits in length.

In certain embodiments, an antisense compound targeted to a target nucleic acid is 12 subunits in length. In certain embodiments, an antisense compound targeted to a target nucleic acid is 13 subunits in length. In certain embodiments, an antisense compound targeted to a target nucleic acid is 14 subunits in length. In certain embodiments, an antisense compound targeted to a target nucleic acid is 15 subunits in length. In certain embodiments, an antisense compound targeted to a target nucleic acid is 16 subunits in length. In certain embodiments, an antisense compound targeted to a target nucleic acid is 17 subunits in length. In certain embodiments, an antisense compound targeted to a target nucleic acid is 18 subunits in length. In certain embodiments, an antisense compound targeted to a target nucleic acid is 19 subunits in length. In certain embodiments, an antisense compound targeted to a target nucleic acid is 20 subunits in length. In certain embodiments, an antisense compound targeted to a target nucleic acid is 21 subunits in length. In certain embodiments, an antisense compound targeted to a target nucleic acid is 22 subunits in length. In certain embodiments, an antisense compound targeted to a target nucleic acid is 23 subunits in length. In certain embodiments, an antisense compound targeted to a target nucleic acid is 24 subunits in length. In certain embodiments, an antisense compound targeted to a target nucleic acid is 25 subunits in length. In certain embodiments, an antisense compound targeted to a target nucleic acid is 26 subunits in length. In certain embodiments, an antisense compound targeted to a target nucleic acid is 27 subunits in length. In certain embodiments, an antisense compound targeted to a target nucleic acid is 28 subunits in length. In certain embodiments, an antisense compound targeted to a target nucleic acid is 29 subunits in length. In certain embodiments, an antisense compound targeted to a target nucleic acid is 30 subunits in length. In certain embodiments, the antisense compound targeted to a target nucleic acid is 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, or 80 linked subunits in length, or a range defined by any two of the above values. In certain embodiments the antisense compound is an antisense oligonucleotide, and the linked subunits are nucleosides.

In certain embodiments antisense oligonucleotides targeted to a target nucleic acid may be shortened or truncated. For example, a single subunit may be deleted from the 5' end (5' truncation), or alternatively from the 3' end (3' truncation). A shortened or truncated antisense compound targeted to a target nucleic acid may have two subunits deleted from the 5' end, or alternatively may have two subunits deleted from the 3' end, of the antisense compound. Alternatively, the deleted nucleosides may be dispersed throughout the antisense compound, for example, in an antisense compound having one nucleoside deleted from the 5' end and one nucleoside deleted from the 3' end.

When a single additional subunit is present in a lengthened antisense compound, the additional subunit may be located at the 5' or 3' end of the antisense compound. When two or more additional subunits are present, the added subunits may be adjacent to each other, for example, in an antisense compound having two subunits added to the 5' end (5' addition), or alternatively to the 3' end (3' addition), of the antisense compound. Alternatively, the added subunits may be dispersed throughout the antisense compound, for example, in an antisense compound having one subunit added to the 5' end and one subunit added to the 3' end.

It is possible to increase or decrease the length of an antisense compound, such as an antisense oligonucleotide, and/or introduce mismatch bases without eliminating activity. For example, in Woolf et al. (Proc. Natl. Acad. Sci. USA 89:7305-7309, 1992), a series of antisense oligonucleotides 13-25 nucleobases in length were tested for their ability to induce cleavage of a target RNA in an oocyte injection model. Antisense oligonucleotides 25 nucleobases in length with 8 or 11 mismatch bases near the ends of the antisense oligonucleotides were able to direct specific cleavage of the target mRNA, albeit to a lesser extent than the antisense oligonucleotides that contained no mismatches. Similarly, target specific cleavage was achieved using 13 nucleobase antisense oligonucleotides, including those with 1 or 3 mismatches.

Gautschi et al (J. Natl. Cancer Inst. 93:463-471, March 2001) demonstrated the ability of an oligonucleotide having 100% complementarity to the bcl-2 mRNA and having 3 mismatches to the bel-xL mRNA to reduce the expression of both bcl-2 and bcl-xL *in vitro* and *in vivo.* Furthermore, this oligonucleotide demonstrated potent anti-tumor activity *in vivo.*

Maher and Dolnick (Nuc. Acid. Res. 16:3341-3358,1988) tested a series of tandem 14 nucleobase antisense oligonucleotides, and a 28 and 42 nucleobase antisense oligonucleotides comprised of the sequence of two or three of the tandem antisense oligonucleotides, respectively, for their ability to arrest translation of human DHFR in a rabbit reticulocyte assay. Each of the three 14 nucleobase antisense oligonucleotides alone was able to inhibit translation, albeit at a more modest level than the 28 or 42 nucleobase antisense oligonucleotides.

### Antisense Compound Motifs

In certain embodiments, antisense compounds targeted to a target nucleic acid have chemically modified subunits arranged in patterns, or motifs, to confer to the antisense compounds properties such as enhanced inhibitory activity, increased binding affinity for a target nucleic acid, or resistance to degradation by *in vivo* nucleases.

Chimeric antisense compounds typically contain at least one region modified so as to confer increased resistance to nuclease degradation, increased cellular uptake, increased binding affinity for the target nucleic acid, and/or increased inhibitory activity. A second region of a chimeric antisense compound may optionally serve as a substrate for the cellular endonuclease RNase H, which cleaves the RNA strand of an RNA:DNA duplex.

Antisense compounds having a gapmer motif are considered chimeric antisense compounds. In a gapmer an internal region having a plurality of nucleotides that supports RNaseH cleavage is positioned between external regions having a plurality of nucleotides that are chemically distinct from the nucleosides of the internal region. In the case of an antisense oligonucleotide having a gapmer motif, the gap segment generally serves as the substrate for endonuclease cleavage, while the wing segments comprise modified nucleosides. In certain embodiments, the regions of a gapmer are differentiated by the types of sugar moieties comprising each distinct region. The types of sugar moieties that are used to differentiate the regions of a gapmer may in some embodiments include β-D-ribonucleosides, β-D-deoxyribonucleosides, 2'-modified nucleosides (such 2'-modified nucleosides may include 2'-MOE, and 2'-O-CH₃, among others), and bicyclic sugar modified nucleosides (such bicyclic sugar modified nucleosides may include those having a 4'-(CH₂)n-O-2' bridge, where n=1 or n=2 and 4'-CH₂-O-CH₂-2'). In certain embodiments, wings may include several modified sugar moieties, including, for example 2'-MOE. In certain embodiments, wings may include several modified and unmodified sugar moieties. In certain embodiments, wings may include various combinations of 2'-MOE nucleosides and 2'-deoxynucleosides.

Each distinct region may comprise uniform sugar moieties, variant, or alternating sugar moieties. The wing-gap-wing motif is frequently described as "X-Y-Z", where "X" represents the length of the 5' wing, "Y" represents the length of the gap, and "Z" represents the length of the 3' wing. "X" and "Z" may comprise uniform, variant, or alternating sugar moieties. In certain embodiments, "X" and "Y" may include one or more 2'-deoxynucleosides. "Y" may comprise 2'-deoxynucleosides. As used herein, a gapmer described as "X-Y-Z" has a configuration such that the gap is positioned immediately adjacent to each of the 5' wing and the 3' wing. Thus, no intervening nucleotides exist between the 5' wing and gap, or the gap and the 3' wing. Any of the antisense compounds described herein can have a gapmer motif. In certain embodiments, "X" and "Z" are the same; in other embodiments they are different.

In certain embodiments, gapmers provided herein include, for example 20-mers having a motif of 5-10-5. In certain embodiments, gapmers provided herein include, for example 19-mers having a motif of 5-9-5. In certain embodiments, gapmers provided herein include, for example 18-mers having a motif of 5-8-5. In certain embodiments, gapmers provided herein include, for example 18-mers having a motif of 4-8-6. In certain embodiments, gapmers provided herein include, for example 18-mers having a motif of 6-8-4. In certain embodiments, gapmers provided herein include, for example 18-mers having a motif of 5-7-6.

### Target Nucleic Acids, Target Regions and Nucleotide Sequences

Nucleotide sequences that encode MECP2 include, without limitation, the following: GENBANK Accession No. NM_004992.3 (incorporated herein as SEQ ID NO: 2) and the complement of GENBANK Accession No. NT_167198.1 truncated from nucleotides 4203000 to 4283000 (incorporated herein as SEQ ID NO: 1).

It is understood that the sequence set forth in each SEQ ID NO in the Examples contained herein is independent of any modification to a sugar moiety, an internucleoside linkage, or a nucleobase. As such, antisense compounds defined by a SEQ ID NO may comprise, independently, one or more modifications to a sugar moiety, an internucleoside linkage, or a nucleobase. Antisense compounds described by Isis Number (Isis No) indicate a combination of nucleobase sequence and motif.

In certain embodiments, a target region is a structurally defined region of the target nucleic acid. For example, a target region may encompass a 3' UTR, a 5' UTR, an exon, an intron, an exon/intron junction, a coding region, a translation initiation region, translation termination region, or other defined nucleic acid region. The structurally defined regions for MECP2 can be obtained by accession number from sequence databases such as NCBI and such information is incorporated herein by reference. In certain embodiments, a target region may encompass the sequence from a 5' target site of one target segment within the target region to a 3' target site of another target segment within the same target region.

Targeting includes determination of at least one target segment to which an antisense compound hybridizes, such that a desired effect occurs. In certain embodiments, the desired effect is a reduction in mRNA target nucleic acid levels. In certain embodiments, the desired effect is reduction of levels of protein encoded by the target nucleic acid or a phenotypic change associated with the target nucleic acid.

A target region may contain one or more target segments. Multiple target segments within a target region may be overlapping. Alternatively, they may be non-overlapping. In certain embodiments, target segments within a target region are separated by no more than about 300 nucleotides. In certain emodiments, target segments within a target region are separated by a number of nucleotides that is, is about, is no more than, is no more than about, 250, 200, 150, 100, 90, 80, 70, 60, 50, 40, 30, 20, or 10 nucleotides on the target nucleic acid, or is a range defined by any two of the preceeding values. In certain embodiments, target segments within a target region are separated by no more than, or no more than about, 5 nucleotides on the target nucleic acid. In certain embodiments, target segments are contiguous. Contemplated are target regions defined by a range having a starting nucleic acid that is any of the 5' target sites or 3' target sites listed herein.

Suitable target segments may be found within a 5' UTR, a coding region, a 3' UTR, an intron, an exon, or an exon/intron junction. Target segments containing a start codon or a stop codon are also suitable target segments. A suitable target segment may specifcally exclude a certain structurally defined region such as the start codon or stop codon.

The determination of suitable target segments may include a comparison of the sequence of a target nucleic acid to other sequences throughout the genome. For example, the BLAST algorithm may be used to identify regions of similarity amongst different nucleic acids. This comparison can prevent the selection of antisense compound sequences that may hybridize in a non-specific manner to sequences other than a selected target nucleic acid (i.e., non-target or off-target sequences).

There may be variation in activity (e.g., as defined by percent reduction of target nucleic acid levels) of the antisense compounds within an active target region. In certain embodiments, reductions in MECP2 mRNA levels are indicative of inhibition of MECP2 expression. Reductions in levels of an MECP2 protein are also indicative of inhibition of target mRNA expression. Phenotypic changes are indicative of inhibition of MECP2 expression. Improvement in neurological function is indicative of inhibition of MECP2 expression. Improved motor function, activity, social behavior, and memory are indicative of inhibition of MECP2 expression. Reduction of anxiety-like behaviors is indicative of inhibition of MECP2 expression.

### Hybridization

In some embodiments, hybridization occurs between an antisense compound disclosed herein and an MECP2 nucleic acid. The most common mechanism of hybridization involves hydrogen bonding (e.g., Watson-Crick, Hoogsteen or reversed Hoogsteen hydrogen bonding) between complementary nucleobases of the nucleic acid molecules.

Hybridization can occur under varying conditions. Stringent conditions are sequence-dependent and are determined by the nature and composition of the nucleic acid molecules to be hybridized.

Methods of determining whether a sequence is specifically hybridizable to a target nucleic acid are well known in the art. In certain embodiments, the antisense compounds provided herein are specifically hybridizable with a MECP2 nucleic acid.

### Complementarity

An antisense compound and a target nucleic acid are complementary to each other when a sufficient number of nucleobases of the antisense compound can hydrogen bond with the corresponding nucleobases of the target nucleic acid, such that a desired effect will occur (e.g., antisense inhibition of a target nucleic acid, such as a MECP2 nucleic acid).

Non-complementary nucleobases between an antisense compound and a target nucleic acid may be tolerated provided that the antisense compound remains able to specifically hybridize to a target nucleic acid. Moreover, an antisense compound may hybridize over one or more segments of a target nucleic acid such that intervening or adjacent segments are not involved in the hybridization event (e.g., a loop structure, mismatch or hairpin structure).

In certain embodiments, the antisense compounds provided herein, or a specified portion thereof, are, or are at least, 70%, 80%, 85%, 86%, 87%, 88%, 89%, 90%, 91%, 92%, 93%, 94%, 95%, 96%, 97%, 98%, 99%, or 100% complementary to a MECP2 nucleic acid, a target region, target segment, or specified portion thereof. Percent complementarity of an antisense compound with a target nucleic acid can be determined using routine methods.

For example, an antisense compound in which 18 of 20 nucleobases of the antisense compound are complementary to a target region, and would therefore specifically hybridize, would represent 90 percent complementarity. **In** this example, the remaining noncomplementary nucleobases may be clustered or interspersed with complementary nucleobases and need not be contiguous to each other or to complementary nucleobases. As such, an antisense compound which is 18 nucleobases in length having 4 (four) noncomplementary nucleobases which are flanked by two regions of complete complementarity with the target nucleic acid would have 77.8% overall complementarity with the target nucleic acid and would thus fall within the scope of the present invention. Percent complementarity of an antisense compound with a region of a target nucleic acid can be determined routinely using BLAST programs (basic local alignment search tools) and PowerBLAST programs known in the art (Altschul et al., J. Mol. Biol., 1990, 215, 403 410; Zhang and Madden, Genome Res., 1997, 7, 649 656). Percent homology, sequence identity or complementarity, can be determined by, for example, the Gap program (Wisconsin Sequence Analysis Package, Version 8 for Unix, Genetics Computer Group, University Research Park, Madison Wis.), using default settings, which uses the algorithm of Smith and Waterman (Adv. Appl. Math., 1981, 2, 482 489).

In certain embodiments, the antisense compounds provided herein, or specified portions thereof, are fully complementary (*i.e.,* 100% complementary) to a target nucleic acid, or specified portion thereof. For example, an antisense compound may be fully complementary to a MECP2 nucleic acid, or a target region, or a target segment or target sequence thereof. As used herein, "fully complementary" means each nucleobase of an antisense compound is capable of precise base pairing with the corresponding nucleobases of a target nucleic acid. For example, a 20 nucleobase antisense compound is fully complementary to a target sequence that is 400 nucleobases long, so long as there is a corresponding 20 nucleobase portion of the target nucleic acid that is fully complementary to the antisense compound. Fully complementary can also be used in reference to a specified portion of the first and /or the second nucleic acid. For example, a 20 nucleobase portion of a 30 nucleobase antisense compound can be "fully complementary" to a target sequence that is 400 nucleobases long. The 20 nucleobase portion of the 30 nucleobase oligonucleotide is fully complementary to the target sequence if the target sequence has a corresponding 20 nucleobase portion wherein each nucleobase is complementary to the 20 nucleobase portion of the antisense compound. At the same time, the entire 30 nucleobase antisense compound may or may not be fully complementary to the target sequence, depending on whether the remaining 10 nucleobases of the antisense compound are also complementary to the target sequence.

The location of a non-complementary nucleobase may be at the 5' end or 3' end of the antisense compound. Alternatively, the non-complementary nucleobase or nucleobases may be at an internal position of the antisense compound. When two or more non-complementary nucleobases are present, they may be contiguous (i.e., linked) or non-contiguous. In one embodiment, a non-complementary nucleobase is located in the wing segment of a gapmer antisense oligonucleotide.

In certain embodiments, antisense compounds that are, or are up to 11, 12, 13, 14, 15, 16, 17, 18, 19, or 20 nucleobases in length comprise no more than 4, no more than 3, no more than 2, or no more than 1 non-complementary nucleobase(s) relative to a target nucleic acid, or specified portion thereof.

In certain embodiments, antisense compounds that are, or are up to 11, 12, 13, 14, 15, 16, 17, 18, 19, 20, 21, 22, 23, 24, 25, 26, 27, 28, 29, or 30 nucleobases in length comprise no more than 6, no more than 5, no more than 4, no more than 3, no more than 2, or no more than 1 non-complementary nucleobase(s) relative to a target nucleic acid, or specified portion thereof.

The antisense compounds provided herein also include those which are complementary to a portion of a target nucleic acid. As used herein, "portion" refers to a defined number of contiguous (i.e. linked) nucleobases within a region or segment of a target nucleic acid. A "portion" can also refer to a defined number of contiguous nucleobases of an antisense compound. In certain embodiments, the antisense compounds, are complementary to at least an 8 nucleobase portion of a target segment. In certain embodiments, the antisense compounds are complementary to at least a 9 nucleobase portion of a target segment. In certain embodiments, the antisense compounds are complementary to at least a 10 nucleobase portion of a target segment. In certain embodiments, the antisense compounds, are complementary to at least an 11 nucleobase portion of a target segment. In certain embodiments, the antisense compounds, are complementary to at least a 12 nucleobase portion of a target segment. In certain embodiments, the antisense compounds, are complementary to at least a 13 nucleobase portion of a target segment. In certain embodiments, the antisense compounds, are complementary to at least a 14 nucleobase portion of a target segment. In certain embodiments, the antisense compounds, are complementary to at least a 15 nucleobase portion of a target segment. Also contemplated are antisense compounds that are complementary to at least a 9, 10, 11, 12, 13, 14, 15, 16, 17, 18, 19, 20, or more nucleobase portion of a target segment, or a range defined by any two of these values.

### Identity

The antisense compounds provided herein may also have a defined percent identity to a particular nucleotide sequence, SEQ ID NO, or compound represented by a specific Isis number, or portion thereof. As used herein, an antisense compound is identical to the sequence disclosed herein if it has the same nucleobase pairing ability. For example, a RNA which contains uracil in place of thymidine in a disclosed DNA sequence would be considered identical to the DNA sequence since both uracil and thymidine pair with adenine. Shortened and lengthened versions of the antisense compounds described herein as well as compounds having non-identical bases relative to the antisense compounds provided herein also are contemplated. The non-identical bases may be adjacent to each other or dispersed throughout the antisense compound. Percent identity of an antisense compound is calculated according to the number of bases that have identical base pairing relative to the sequence to which it is being compared.

In certain embodiments, the antisense compounds, or portions thereof, are at least 70%, 75%, 80%, 85%, 90%, 95%, 96%, 97%, 98%, 99% or 100% identical to one or more of the antisense compounds or SEQ ID NOs, or a portion thereof, disclosed herein.

In certain embodiments, a portion of the antisense compound is compared to an equal length portion of the target nucleic acid. In certain embodiments, an 8, 9, 10, 11, 12, 13, 14, 15, 16, 17, 18, 19, 20, 21, 22, 23, 24, or 25 nucleobase portion is compared to an equal length portion of the target nucleic acid.

In certain embodiments, a portion of the antisense oligonucleotide is compared to an equal length portion of the target nucleic acid. In certain embodiments, an 8, 9, 10, 11, 12, 13, 14, 15, 16, 17, 18, 19, 20, 21, 22, 23, 24, or 25 nucleobase portion is compared to an equal length portion of the target nucleic acid.

### Modifications

A nucleoside is a base-sugar combination. The nucleobase (also known as base) portion of the nucleoside is normally a heterocyclic base moiety. Nucleotides are nucleosides that further include a phosphate group covalently linked to the sugar portion of the nucleoside. For those nucleosides that include a pentofuranosyl sugar, the phosphate group can be linked to the 2', 3' or 5' hydroxyl moiety of the sugar. Oligonucleotides are formed through the covalent linkage of adjacent nucleosides to one another, to form a linear polymeric oligonucleotide. Within the oligonucleotide structure, the phosphate groups are commonly referred to as forming the internucleoside linkages of the oligonucleotide.

Modifications to antisense compounds encompass substitutions or changes to internucleoside linkages, sugar moieties, or nucleobases. Modified antisense compounds are often preferred over native forms because of desirable properties such as, for example, enhanced cellular uptake, enhanced affinity for nucleic acid target, increased stability in the presence of nucleases, or increased inhibitory activity.

Chemically modified nucleosides may also be employed to increase the binding affinity of a shortened or truncated antisense oligonucleotide for its target nucleic acid. Consequently, comparable results can often be obtained with shorter antisense compounds that have such chemically modified nucleosides.

### Modified Internucleoside Linkages

The naturally occuring internucleoside linkage of RNA and DNA is a 3' to 5' phosphodiester linkage. Antisense compounds having one or more modified, i.e. non-naturally occurring, internucleoside linkages are often selected over antisense compounds having naturally occurring internucleoside linkages because of desirable properties such as, for example, enhanced cellular uptake, enhanced affinity for target nucleic acids, and increased stability in the presence of nucleases.

Oligonucleotides having modified internucleoside linkages include internucleoside linkages that retain a phosphorus atom as well as internucleoside linkages that do not have a phosphorus atom. Representative phosphorus containing internucleoside linkages include, but are not limited to, phosphodiesters, phosphotriesters, methylphosphonates, phosphoramidate, and phosphorothioates. Methods of preparation of phosphorous-containing and non-phosphorous-containing linkages are well known.

In certain embodiments, antisense compounds targeted to a MECP2 nucleic acid comprise one or more modified internucleoside linkages. In certain embodiments, the modified internucleoside linkages are interspersed throughout the antisense compound. In certain embodiments, the modified internucleoside linkages are phosphorothioate linkages. In certain embodiments, each internucleoside linkage of an antisense compound is a phosphorothioate internucleoside linkage.

### Modified Sugar Moieties

Antisense compounds can optionally contain one or more nucleosides wherein the sugar group has been modified. Such sugar modified nucleosides may impart enhanced nuclease stability, increased binding affinity, or some other beneficial biological property to the antisense compounds. In certain embodiments, nucleosides comprise chemically modified ribofuranose ring moieties. Examples of chemically modified ribofuranose rings include without limitation, addition of substitutent groups (including 5' and 2' substituent groups, bridging of non-geminal ring atoms to form bicyclic nucleic acids (BNA), replacement of the ribosyl ring oxygen atom with S, N(R), or C(R₁)(R₂) (R, R₁ and R₂ are each independently H, C₁-C₁₂ alkyl or a protecting group) and combinations thereof. Examples of chemically modified sugars include 2'-F-5'-methyl substituted nucleoside (see PCT International Application WO 2008/101157 Published on 8/21/08 for other disclosed 5',2'-bis substituted nucleosides) or replacement of the ribosyl ring oxygen atom with S with further substitution at the 2'-position (see published U.S. Patent Application US2005-0130923, published on June 16, 2005) or alternatively 5'-substitution of a BNA (see PCT International Application WO 2007/134181 Published on 11/22/07 wherein LNA is substituted with for example a 5'-methyl or a 5'-vinyl group).

Examples of nucleosides having modified sugar moieties include without limitation nucleosides comprising 5'-vinyl, 5'-methyl (R or S), 4'-S, 2'-F, 2'-OCH₃, 2'-OCH₂CH₃, 2'-OCH₂CH₂F and 2'-O(CH₂)₂OCH₃ substituent groups. The substituent at the 2' position can also be selected from allyl, amino, azido, thio, O-allyl, O-C₁-C₁₀ alkyl, OCF₃, OCH₂F, O(CH₂)₂SCH₃, O(CH₂)₂-O-N(Rₘ)(Rₙ), O-CH₂-C(=O)-N(Rₘ)(Rₙ), and O-CH₂-C(=O)-N(R₁)-(CH₂)₂-N(Rₘ)(Rₙ), where each R₁, Rₘ and Rₙ is, independently, H or substituted or unsubstituted C₁-C₁₀ alkyl.

As used herein, "bicyclic nucleosides" refer to modified nucleosides comprising a bicyclic sugar moiety. Examples of bicyclic nucleosides include without limitation nucleosides comprising a bridge between the 4' and the 2' ribosyl ring atoms. In certain embodiments, antisense compounds provided herein include one or more bicyclic nucleosides comprising a 4' to 2' bridge. Examples of such 4' to 2' bridged bicyclic nucleosides, include but are not limited to one of the formulae: 4'-(CH₂)-O-2' (LNA); 4'-(CH₂)-S-2'; 4'-(CH₂)₂-O-2' (ENA); 4'-CH(CH₃)-O-2' and 4'-CH(CH₂OCH₃)-O-2' (and analogs thereof see U.S. Patent 7,399,845, issued on July 15, 2008); 4'-C(CH₃)(CH₃)-O-2' (and analogs thereof see published International Application WO/2009/006478, published January 8, 2009); 4'-CH₂-N(OCH₃)-2' (and analogs thereof see published International Application WO/2008/150729, published December 11, 2008); 4'-CH₂-O-N(CH₃)-2' (see published U.S. Patent Application US2004-0171570, published September 2, 2004 ); 4'-CH₂-N(R)-O-2', wherein R is H, C₁-C₁₂ alkyl, or a protecting group (see U.S. Patent 7,427,672, issued on September 23, 2008); 4'-CH₂-C(H)(CH₃)-2' (see Chattopadhyaya et al., J. Org. Chem., 2009, 74, 118-134); and 4'-CH₂-C-(=CH₂)-2' (and analogs thereof see published International Application WO 2008/154401, published on December 8, 2008).

Further reports related to bicyclic nucleosides can also be found in published literature (see for example: Singh et al., Chem. Commun., 1998, 4, 455-456; Koshkin et al., Tetrahedron, 1998, 54, 3607-3630; Wahlestedt et al., Proc. Natl. Acad. Sci. U. S. A., 2000, 97, 5633-5638; Kumar et al., Bioorg. Med. Chem. Lett., 1998, 8, 2219-2222; Singh et al., J. Org. Chem., 1998, 63, 10035-10039; Srivastava et al., J. Am. Chem. Soc., 2007, 129(26) 8362-8379; Elayadi et al., Curr. Opinion Invest. Drugs, 2001, 2, 558-561; Braasch et al., Chem. Biol., 2001, 8, 1-7; and Orum et al., Curr. Opinion Mol. Ther., 2001, 3, 239-243; U.S. Patent Nos. 6,268,490; 6,525,191; 6,670,461; 6,770,748; 6,794,499; 7,034,133; 7,053,207; 7,399,845; 7,547,684; and 7,696,345; U.S. Patent Publication No. US2008-0039618; US2009-0012281; U.S. Patent Serial Nos. 60/989,574; 61/026,995; 61/026,998; 61/056,564; 61/086,231; 61/097,787; and 61/099,844; Published PCT International applications WO 1994/014226; WO 2004/106356; WO 2005/021570; WO 2007/134181; WO 2008/150729; WO 2008/154401; and WO 2009/006478. Each of the foregoing bicyclic nucleosides can be prepared having one or more stereochemical sugar configurations including for example α-L-ribofuranose and β-D-ribofuranose (see PCT international application PCT/DK98/00393, published on March 25, 1999 as WO 99/14226).

In certain embodiments, bicyclic sugar moieties of BNA nucleosides include, but are not limited to, compounds having at least one bridge between the 4' and the 2' position of the pentofuranosyl sugar moiety wherein such bridges independently comprises 1 or from 2 to 4 linked groups independently selected from - [C(Rₐ)(R_{b})]ₙ-, -C(Rₐ)=C(R_{b})-, -C(Rₐ)=N-, -C(=O)-, -C(=NRₐ)-, -C(=S)-, -O-, -Si(Rₐ)₂-, -S(=O)ₓ-, and -N(Rₐ)-; wherein:
x is 0, 1, or 2;
n is 1, 2, 3, or 4;
each Rₐ and R_{b} is, independently, H, a protecting group, hydroxyl, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, heterocycle radical, substituted heterocycle radical, heteroaryl, substituted heteroaryl, C₅-C₇ alicyclic radical, substituted C₅-C₇ alicyclic radical, halogen, OJ₁, NJ₁J₂, SJ₁, N₃, COOJ₁, acyl (C(=O)-H), substituted acyl, CN, sulfonyl (S(=O)₂-J₁), or sulfoxyl (S(=O)-J₁); and
each J₁ and J₂ is, independently, H, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, acyl (C(=O)-H), substituted acyl, a heterocycle radical, a substituted heterocycle radical, C₁-C₁₂ aminoalkyl, substituted C₁-C₁₂ aminoalkyl or a protecting group.

In certain embodiments, the bridge of a bicyclic sugar moiety is -[C(Rₐ)(R_{b})]ₙ-, -[C(Rₐ)(R_{b})]ₙ-O-, -C(RₐR_{b})-N(R)-O- or -C(RₐR_{b})-O-N(R)-. In certain embodiments, the bridge is 4'-CH₂-2', 4'-(CH₂)₂-2', 4'-(CH₂)₃-2', 4'-CH₂-O-2', 4'-(CH₂)₂-O-2', 4'-CH₂-O-N(R)-2' and 4'-CH₂-N(R)-O-2'- wherein each Ris, independently, H, a protecting group or C₁-C₁₂ alkyl.

In certain embodiments, bicyclic nucleosides are further defined by isomeric configuration. For example, a nucleoside comprising a 4'-2' methylene-oxy bridge, may be in the α-L configuration or in the β-D configuration. Previously, α-L-methyleneoxy (4'-CH₂-O-2') BNA's have been incorporated into antisense oligonucleotides that showed antisense activity (Frieden et al., Nucleic Acids Research, 2003, 21, 6365-6372).

In certain embodiments, bicyclic nucleosides include, but are not limited to, (A) α-L-methyleneoxy (4'-CH₂-O-2') BNA, (B) β-D-methyleneoxy (4'-CH₂-O-2') BNA, (C) ethyleneoxy (4'-(CH₂)₂-O-2') BNA , (D) aminooxy (4'-CH₂-O-N(R)-2') BNA, (E) oxyamino (4'-CH₂-N(R)-O-2') BNA, and (F) methyl(methyleneoxy) (4'-CH(CH₃)-O-2') BNA, (G) methylene-thio (4'-CH₂-S-2') BNA, (H) methylene-amino (4'-CH₂-N(R)-2') BNA, (I) methyl carbocyclic (4'-CH₂-CH(CH₃)-2') BNA, and (J) propylene carbocyclic (4'-(CH₂)₃-2') BNA as depicted below. 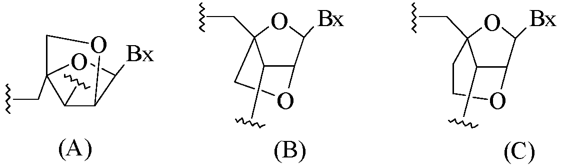 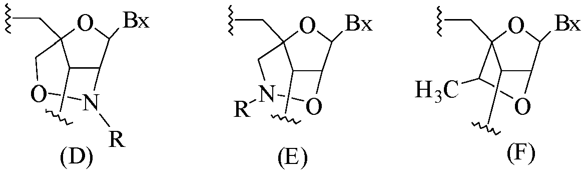 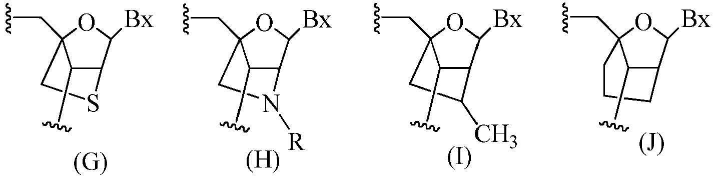 wherein Bx is the base moiety and R is independently H, a protecting group or C₁-C₁₂ alkyl.

In certain embodiments, bicyclic nucleosides are provided having Formula I: 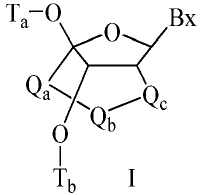 wherein:
Bx is a heterocyclic base moiety;
-Qₐ-Q_{b}-Q_{c}- is -CH₂-N(R_{c})-CH₂-, -C(=O)-N(R_{c})-CH₂-, -CH₂-O-N(R_{c})-, -CH₂-N(R_{c})-O- or -N(R_{c})-O-CH₂;
R_{c} is C₁-C₁₂ alkyl or an amino protecting group; and
Tₐ and T_{b} are each, independently H, a hydroxyl protecting group, a conjugate group, a reactive phosphorus group, a phosphorus moiety or a covalent attachment to a support medium.

In certain embodiments, bicyclic nucleosides are provided having Formula II: 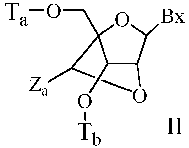 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
Zₐ is C₁-C₆ alkyl, C₂-C₆ alkenyl, C₂-C₆ alkynyl, substituted C₁-C₆ alkyl, substituted C₂-C₆ alkenyl, substituted C₂-C₆ alkynyl, acyl, substituted acyl, substituted amide, thiol or substituted thio.

In one embodiment, each of the substituted groups is, independently, mono or poly substituted with substituent groups independently selected from halogen, oxo, hydroxyl, OJ_{c}, NJ_{c}J_{d}, SJ_{c}, N₃, OC(=X)J_{c}, and NJₑC(=X)NJ_{c}J_{d}, wherein each J_{c}, J_{d} and Jₑ is, independently, H, C₁-C₆ alkyl, or substituted C₁-C₆ alkyl and X is O or NJ_{c}.

In certain embodiments, bicyclic nucleosides are provided having Formula III: 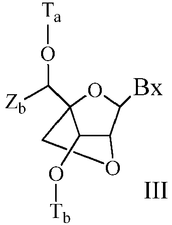 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
Z_{b} is C₁-C₆ alkyl, C₂-C₆ alkenyl, C₂-C₆ alkynyl, substituted C₁-C₆ alkyl, substituted C₂-C₆ alkenyl, substituted C₂-C₆ alkynyl or substituted acyl (C(=O)-).

In certain embodiments, bicyclic nucleosides are provided having Formula IV: 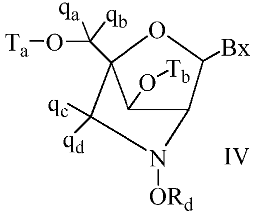 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
R_{d} is C₁-C₆ alkyl, substituted C₁-C₆ alkyl, C₂-C₆ alkenyl, substituted C₂-C₆ alkenyl, C₂-C₆ alkynyl or substituted C₂-C₆ alkynyl;
each qₐ, q_{b}, q_{c} and q_{d} is, independently, H, halogen, C₁-C₆ alkyl, substituted C₁-C₆ alkyl, C₂-C₆ alkenyl, substituted C₂-C₆ alkenyl, C₂-C₆ alkynyl or substituted C₂-C₆ alkynyl, C₁-C₆ alkoxyl, substituted C₁-C₆ alkoxyl, acyl, substituted acyl, C₁-C₆ aminoalkyl or substituted C₁-C₆ aminoalkyl;

In certain embodiments, bicyclic nucleosides are provided having Formula V: 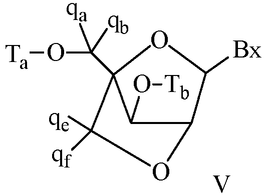 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
qₐ, q_{b}, qₑ and q_{f} are each, independently, hydrogen, halogen, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₁-C₁₂ alkoxy, substituted C₁-C₁₂ alkoxy, OJⱼ, SJⱼ, SOJⱼ, SO₂Jⱼ, NJⱼJₖ, N₃, CN, C(=O)OJⱼ, C(=O)NJⱼJₖ, C(=O)Jⱼ, O-C(=O)NJⱼJₖ, N(H)C(=NH)NJⱼJₖ, N(H)C(=O)NJⱼJₖ or N(H)C(=S)NJⱼJₖ;
or qₑ and q_{f} together are =C(q_{g})(qₕ);
q_{g} and qₕ are each, independently, H, halogen, C₁-C₁₂ alkyl or substituted C₁-C₁₂ alkyl.

The synthesis and preparation of the methyleneoxy (4'-CH₂-O-2') BNA monomers adenine, cytosine, guanine, 5-methyl-cytosine, thymine and uracil, along with their oligomerization, and nucleic acid recognition properties have been described (Koshkin et al., Tetrahedron, 1998, 54, 3607-3630). BNAs and preparation thereof are also described in WO 98/39352 and WO 99/14226.

Analogs of methyleneoxy (4'-CH₂-O-2') BNA and 2'-thio-BNAs, have also been prepared (Kumar et al., Bioorg. Med. Chem. Lett., 1998, 8, 2219-2222). Preparation of locked nucleoside analogs comprising oligodeoxyribonucleotide duplexes as substrates for nucleic acid polymerases has also been described (Wengel et al., WO 99/14226 ). Furthermore, synthesis of 2'-amino-BNA, a novel comformationally restricted high-affinity oligonucleotide analog has been described in the art (Singh et al., J. Org. Chem., 1998, 63, 10035-10039). In addition, 2'-amino- and 2'-methylamino-BNA's have been prepared and the thermal stability of their duplexes with complementary RNA and DNA strands has been previously reported.

In certain embodiments, bicyclic nucleosides are provided having Formula VI: 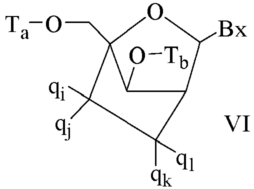 wherein:
Bx is a heterocyclic base moiety;
Tₐ and T_{b} are each, independently H, a hydroxyl protecting group, a conjugate group, a reactive phosphorus group, a phosphorus moiety or a covalent attachment to a support medium;
each qᵢ, qⱼ, qₖ and qₗ is, independently, H, halogen, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₁-C₁₂ alkoxyl, substituted C₁-C₁₂ alkoxyl, OJⱼ, SJⱼ, SOJⱼ, SO₂Jⱼ, NJⱼJₖ, N₃, CN, C(=O)OJⱼ, C(=O)NJⱼJₖ, C(=O)Jⱼ, O-C(=O)NJⱼJₖ, N(H)C(=NH)NJⱼJₖ, N(H)C(=O)NJⱼJₖ or N(H)C(=S)NJⱼJₖ; and
qᵢ and qⱼ or qₗ and qₖ together are =C(q_{g})(qₕ), wherein q_{g} and qₕ are each, independently, H, halogen, C₁-C₁₂ alkyl or substituted C₁-C₁₂ alkyl.

One carbocyclic bicyclic nucleoside having a 4'-(CH₂)₃-2' bridge and the alkenyl analog bridge 4'-CH=CH-CH₂-2' have been described (Freier et al., Nucleic Acids Research, 1997, 25(22), 4429-4443 and Albaek et al., J. Org. Chem., 2006, 71, 7731-7740). The synthesis and preparation of carbocyclic bicyclic nucleosides along with their oligomerization and biochemical studies have also been described (Srivastava et al., J. Am. Chem. Soc., 2007, 129(26), 8362-8379).

As used herein, "4'-2' bicyclic nucleoside" or "4' to 2' bicyclic nucleoside" refers to a bicyclic nucleoside comprising a furanose ring comprising a bridge connecting two carbon atoms of the furanose ring connects the 2' carbon atom and the 4' carbon atom of the sugar ring.

As used herein, "monocylic nucleosides" refer to nucleosides comprising modified sugar moieties that are not bicyclic sugar moieties. In certain embodiments, the sugar moiety, or sugar moiety analogue, of a nucleoside may be modified or substituted at any position.

As used herein, "2'-modified sugar" means a furanosyl sugar modified at the 2' position. In certain embodiments, such modifications include substituents selected from: a halide, including, but not limited to substituted and unsubstituted alkoxy, substituted and unsubstituted thioalkyl, substituted and unsubstituted amino alkyl, substituted and unsubstituted alkyl, substituted and unsubstituted allyl, and substituted and unsubstituted alkynyl. In certain embodiments, 2' modifications are selected from substituents including, but not limited to: O[(CH₂)ₙO]ₘCH₃, O(CH₂)ₙNH₂, O(CH₂)ₙCH₃, O(CH₂)ₙF, O(CH₂)ₙONH₂, OCH₂C(=O)N(H)CH₃, and O(CH₂)ₙON[(CH₂)ₙCH₃]₂, where n and m are from 1 to about 10. Other 2'-substituent groups can also be selected from: C₁-C₁₂ alkyl, substituted alkyl, alkenyl, alkynyl, alkaryl, aralkyl, O-alkaryl or O-aralkyl, SH, SCH₃, OCN, Cl, Br, CN, F, CF₃, OCF₃, SOCH₃, SO₂CH₃, ONO₂, NO₂, N₃, NH₂, heterocycloalkyl, heterocycloalkaryl, aminoalkylamino, polyalkylamino, substituted silyl, an RNA cleaving group, a reporter group, an intercalator, a group for improving pharmacokinetic properties, or a group for improving the pharmacodynamic properties of an antisense compound, and other substituents having similar properties. In certain embodiments, modifed nucleosides comprise a 2'-MOE side chain (Baker et al., J. Biol. Chem., 1997, 272, 11944-12000). Such 2'-MOE substitution have been described as having improved binding affinity compared to unmodified nucleosides and to other modified nucleosides, such as 2'- *O-*methyl, *O*-propyl, and *O*-aminopropyl. Oligonucleotides having the 2'-MOE substituent also have been shown to be antisense inhibitors of gene expression with promising features for *in vivo* use (Martin, Helv. Chim. Acta, 1995, 78, 486-504; Altmann et al., Chimia, 1996, 50, 168-176; Altmann et al., Biochem. Soc. Trans., 1996, 24, 630-637; and Altmann et al., Nucleosides Nucleotides, 1997, 16, 917-926).

As used herein, a "modified tetrahydropyran nucleoside" or "modified THP nucleoside" means a nucleoside having a six-membered tetrahydropyran "sugar" substituted in for the pentofuranosyl residue in normal nucleosides (a sugar surrogate). Modified THP nucleosides include, but are not limited to, what is referred to in the art as hexitol nucleic acid (HNA), anitol nucleic acid (ANA), manitol nucleic acid (MNA) (see Leumann, Bioorg. Med. Chem., 2002, 10, 841-854), fluoro HNA (F-HNA) or those compounds having Formula VII: 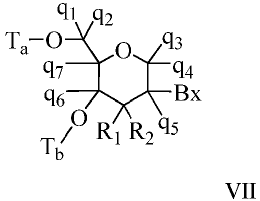 wherein independently for each of said at least one tetrahydropyran nucleoside analog of Formula VII:
Bx is a heterocyclic base moiety;
Tₐ and T_{b} are each, independently, an internucleoside linking group linking the tetrahydropyran nucleoside analog to the antisense compound or one of Tₐ and T_{b} is an internucleoside linking group linking the tetrahydropyran nucleoside analog to the antisense compound and the other of Tₐ and T_{b} is H, a hydroxyl protecting group, a linked conjugate group or a 5' or 3'-terminal group;
q₁, q₂, q₃, q₄, q₅, q₆ and q₇ are each independently, H, C₁-C₆ alkyl, substituted C₁-C₆ alkyl, C₂-C₆ alkenyl, substituted C₂-C₆ alkenyl, C₂-C₆ alkynyl or substituted C₂-C₆ alkynyl; and each of R₁ and R₂ is selected from hydrogen, hydroxyl, halogen, substituted or unsubstituted alkoxy, NJ₁J₂, SJ₁, N₃, OC(=X)J₁, OC(=X)NJ₁J₂, NJ₃C(=X)NJ₁J₂ and CN, wherein X is O, S or NJ₁ and each J₁, J₂ and J₃ is, independently, H or C₁-C₆ alkyl.

In certain embodiments, the modified THP nucleosides of Formula VII are provided wherein q₁, q₂, q₃, q₄, q₅, q₆ and q₇ are each H. In certain embodiments, at least one of q₁, q₂, q₃, q₄, q₅, q₆ and q₇ is other than H. In certain embodiments, at least one of q₁, q₂, q₃, q₄, q₅, q₆ and q₇ is methyl. In certain embodiments, THP nucleosides of Formula VII are provided wherein one of R₁ and R₂ is fluoro. In certain embodiments, R₁ is fluoro and R₂ is H; R₁ is methoxy and R₂ is H, and R₁ is H and R₂ is methoxyethoxy.

As used herein, "2'-modified" or "2'-substituted" refers to a nucleoside comprising a sugar comprising a substituent at the 2' position other than H or OH. 2'-modified nucleosides, include, but are not limited to, bicyclic nucleosides wherein the bridge connecting two carbon atoms of the sugar ring connects the 2' carbon and another carbon of the sugar ring; and nucleosides with non-bridging 2'substituents, such as allyl, amino, azido, thio, O-allyl, O-C₁-C₁₀ alkyl, -OCF₃, O-(CH₂)₂-O-CH₃, 2'-O(CH₂)₂SCH₃, O-(CH₂)₂-ON(Rₘ)(Rₙ), or O-CH₂-C(=O)-N(Rₘ)(Rₙ), where each Rₘ and Rₙ is, independently, H or substituted or unsubstituted C₁-C₁₀ alkyl. 2'-modifed nucleosides may further comprise other modifications, for example at other positions of the sugar and/or at the nucleobase.

As used herein, "2'-F" refers to a nucleoside comprising a sugar comprising a fluoro group at the 2' position.

As used herein, "2'-OMe" or "2'-OCH₃" or "2'-O-methyl" each refers to a nucleoside comprising a sugar comprising an -OCH₃ group at the 2' position of the sugar ring.

As used herein, "MOE" or "2'-MOE" or "2'-OCH₂CH₂OCH₃" or "2'-O-methoxyethyl" each refers to a nucleoside comprising a sugar comprising a -OCH₂CH₂OCH₃ group at the 2' position of the sugar ring.

As used herein, "oligonucleotide" refers to a compound comprising a plurality of linked nucleosides. In certain embodiments, one or more of the plurality of nucleosides is modified. In certain embodiments, an oligonucleotide comprises one or more ribonucleosides (RNA) and/or deoxyribonucleosides (DNA).

Many other bicyclo and tricyclo sugar surrogate ring systems are also known in the art that can be used to modify nucleosides for incorporation into antisense compounds (see for example review article: Leumann, Bioorg. Med. Chem., 2002, 10, 841-854).

Such ring systems can undergo various additional substitutions to enhance activity.

Methods for the preparations of modified sugars are well known to those skilled in the art.

In nucleotides having modified sugar moieties, the nucleobase moieties (natural, modified or a combination thereof) are maintained for hybridization with an appropriate nucleic acid target.

In certain embodiments, antisense compounds comprise one or more nucleosides having modified sugar moieties. In certain embodiments, the modified sugar moiety is 2'-MOE. In certain embodiments, the 2'-MOE modified nucleosides are arranged in a gapmer motif. In certain embodiments, the modified sugar moiety is a bicyclic nucleoside having a (4'-CH(CH₃)-O-2') bridging group. In certain embodiments, the (4'-CH(CH₃)-O-2') modified nucleosides are arranged throughout the wings of a gapmer motif.

### Compositions and Methods for Formulating Pharmaceutical Compositions

Antisense oligonucleotides may be admixed with pharmaceutically acceptable active or inert substances for the preparation of pharmaceutical compositions or formulations. Compositions and methods for the formulation of pharmaceutical compositions are dependent upon a number of criteria, including, but not limited to, route of administration, extent of disorder, or dose to be administered.

An antisense compound targeted to a MECP2 nucleic acid can be utilized in pharmaceutical compositions by combining the antisense compound with a suitable pharmaceutically acceptable diluent or carrier. A pharmaceutically acceptable diluent includes phosphate-buffered saline (PBS). PBS is a diluent suitable for use in compositions to be delivered parenterally. Accordingly, in one embodiment, employed in the methods described herein is a pharmaceutical composition comprising an antisense compound targeted to a MECP2 nucleic acid and a pharmaceutically acceptable diluent. In certain embodiments, the pharmaceutically acceptable diluent is PBS. In certain embodiments, the antisense compound is an antisense oligonucleotide.

Pharmaceutical compositions comprising antisense compounds encompass any pharmaceutically acceptable salts, esters, or salts of such esters, or any other oligonucleotide which, upon administration to an animal, including a human, is capable of providing (directly or indirectly) the biologically active metabolite or residue thereof. Accordingly, for example, the disclosure is also drawn to pharmaceutically acceptable salts of antisense compounds, prodrugs, pharmaceutically acceptable salts of such prodrugs, and other bioequivalents. Suitable pharmaceutically acceptable salts include, but are not limited to, sodium and potassium salts.

A prodrug can include the incorporation of additional nucleosides at one or both ends of an antisense compound which are cleaved by endogenous nucleases within the body, to form the active antisense compound.

### Conjugated Antisense Compounds

Antisense compounds may be covalently linked to one or more moieties or conjugates which enhance the activity, cellular distribution or cellular uptake of the resulting antisense oligonucleotides. Typical conjugate groups include cholesterol moieties and lipid moieties. Additional conjugate groups include carbohydrates, phospholipids, biotin, phenazine, folate, phenanthridine, anthraquinone, acridine, fluoresceins, rhodamines, coumarins, and dyes.

Antisense compounds can also be modified to have one or more stabilizing groups that are generally attached to one or both termini of antisense compounds to enhance properties such as, for example, nuclease stability. Included in stabilizing groups are cap structures. These terminal modifications protect the antisense compound having terminal nucleic acid from exonuclease degradation, and can help in delivery and/or localization within a cell. The cap can be present at the 5'-terminus (5'-cap), or at the 3'-terminus (3'-cap), or can be present on both termini. Cap structures are well known in the art and include, for example, inverted deoxy abasic caps. Further 3' and 5'-stabilizing groups that can be used to cap one or both ends of an antisense compound to impart nuclease stability include those disclosed in WO 03/004602 published on January 16, 2003.

### Cell culture and antisense compounds treatment

The effects of antisense compounds on the level, activity or expression of MECP2 nucleic acids can be tested *in vitro* in a variety of cell types. Cell types used for such analyses are available from commerical vendors (e.g. American Type Culture Collection, Manassus, VA; Zen-Bio, Inc., Research Triangle Park, NC; Clonetics Corporation, Walkersville, MD) and are cultured according to the vendor's instructions using commercially available reagents (e.g. Invitrogen Life Technologies, Carlsbad, CA). Illustrative cell types include, but are not limited to, HepG2 cells, Hep3B cells, and primary hepatocytes. In certain embodiments, cells are patient cells, such as B-lymphoblast cells.

### In vitro testing of antisense oligonucleotides

Described herein are methods for treatment of cells with antisense oligonucleotides, which can be modified appropriately for treatment with other antisense compounds.

Cells may be treated with antisense oligonucleotides when the cells reach approximately 60-80% confluency in culture.

One reagent commonly used to introduce antisense oligonucleotides into cultured cells includes the cationic lipid transfection reagent LIPOFECTIN (Invitrogen, Carlsbad, CA). Antisense oligonucleotides may be mixed with LIPOFECTIN in OPTI-MEM 1 (Invitrogen, Carlsbad, CA) to achieve the desired final concentration of antisense oligonucleotide and a LIPOFECTIN concentration that may range from 2 to 12 ug/mL per 100 nM antisense oligonucleotide.

Another reagent used to introduce antisense oligonucleotides into cultured cells includes LIPOFECTAMINE (Invitrogen, Carlsbad, CA). Antisense oligonucleotide is mixed with LIPOFECTAMINE in OPTI-MEM 1 reduced serum medium (Invitrogen, Carlsbad, CA) to achieve the desired concentration of antisense oligonucleotide and a LIPOFECTAMINE concentration that may range from 2 to 12 ug/mL per 100 nM antisense oligonucleotide.

Another reagent used to introduce antisense oligonucleotides into cultured cells includes TURBOFECT (Thermo Scientific, Carlsbad, CA).

Another technique used to introduce antisense oligonucleotides into cultured cells includes electroporation.

Cells are treated with antisense oligonucleotides by routine methods. Cells may be harvested 16-24 hours after antisense oligonucleotide treatment, at which time RNA or protein levels of target nucleic acids are measured by methods known in the art and described herein. In general, when treatments are performed in multiple replicates, the data are presented as the average of the replicate treatments.

The concentration of antisense oligonucleotide used varies from cell line to cell line. Methods to determine the optimal antisense oligonucleotide concentration for a particular cell line are well known in the art. Antisense oligonucleotides are typically used at concentrations ranging from 1 nM to 300 nM when transfected with LIPOFECTAMINE. Antisense oligonucleotides are used at higher concentrations ranging from 625 to 20,000 nM when transfected using electroporation.

### RNA Isolation

RNA analysis can be performed on total cellular RNA or poly(A)+ mRNA. Methods of RNA isolation are well known in the art. RNA is prepared using methods well known in the art, for example, using the TRIZOL Reagent (Invitrogen, Carlsbad, CA) according to the manufacturer's recommended protocols.

### Analysis of inhibition of target levels or expression

Inhibition of levels or expression of a MECP2 nucleic acid can be assayed in a variety of ways known in the art. For example, target nucleic acid levels can be quantitated by, e.g., Northern blot analysis, competitive polymerase chain reaction (PCR), or quantitaive real-time PCR. RNA analysis can be performed on total cellular RNA or poly(A)+ mRNA. Methods of RNA isolation are well known in the art. Northern blot analysis is also routine in the art. Quantitative real-time PCR can be conveniently accomplished using the commercially available ABI PRISM 7600, 7700, or 7900 Sequence Detection System, available from PE-Applied Biosystems, Foster City, CA and used according to manufacturer's instructions.

### Quantitative Real-Time PCR Analysis of Target RNA Levels

Quantitation of target RNA levels may be accomplished by quantitative real-time PCR using the ABI PRISM 7600, 7700, or 7900 Sequence Detection System (PE-Applied Biosystems, Foster City, CA) according to manufacturer's instructions. Methods of quantitative real-time PCR are well known in the art.

Prior to real-time PCR, the isolated RNA is subjected to a reverse transcriptase (RT) reaction, which produces complementary DNA (cDNA) that is then used as the substrate for the real-time PCR amplification. The RT and real-time PCR reactions are performed sequentially in the same sample well. RT and real-time PCR reagents may be obtained from Invitrogen (Carlsbad, CA). RT real-time-PCR reactions are carried out by methods well known to those skilled in the art.

Gene (or RNA) target quantities obtained by real time PCR are normalized using either the expression level of a gene whose expression is constant, such as cyclophilin A, or by quantifying total RNA using RIBOGREEN (Invitrogen, Inc. Carlsbad, CA). Cyclophilin A expression is quantified by real time PCR, by being run simultaneously with the target, multiplexing, or separately. Total RNA is quantified using RIBOGREEN RNA quantification reagent (Invetrogen, Inc. Eugene, OR). Methods of RNA quantification by RIBOGREEN are taught in Jones, L.J., et al, (Analytical Biochemistry, 1998, 265, 368-374). A CYTOFLUOR 4000 instrument (PE Applied Biosystems) is used to measure RIBOGREEN fluorescence.

Probes and primers are designed to hybridize to a MECP2 nucleic acid. Methods for designing real-time PCR probes and primers are well known in the art, and may include the use of software such as PRIMER EXPRESS Software (Applied Biosystems, Foster City, CA).

### Analysis of Protein Levels

Antisense inhibition of MECP2 nucleic acids can be assessed by measuring MECP2 protein levels. Protein levels of MECP2 can be evaluated or quantitated in a variety of ways well known in the art, such as immunoprecipitation, Western blot analysis (immunoblotting), enzyme-linked immunosorbent assay (ELISA), quantitative protein assays, protein activity assays (for example, caspase activity assays), immunohistochemistry, immunocytochemistry or fluorescence-activated cell sorting (FACS). Antibodies directed to a target can be identified and obtained from a variety of sources, such as the MSRS catalog of antibodies (Aerie Corporation, Birmingham, MI), or can be prepared via conventional monoclonal or polyclonal antibody generation methods well known in the art.

### In vivo testing of antisense compounds

Antisense compounds, for example, antisense oligonucleotides, are tested in animals to assess their ability to inhibit expression of MECP2 and produce phenotypic changes, such as, improved behavior, motor function, and cognition. In certain embodiments, motor function is measured by walking initiation analysis, rotarod, grip strength, pole climb, open field performance, balance beam, hindpaw footprint testing in the animal. In certain embodiments, behavior is measured by elevated plus maze and three-chamber social interaction. Testing may be performed in normal animals, or in experimental models. For administration to animals, antisense oligonucleotides are formulated in a pharmaceutically acceptable diluent, such as phosphate-buffered saline. Administration includes parenteral routes of administration, such as intraperitoneal, intravenous, and subcutaneous. Calculation of antisense oligonucleotide dosage and dosing frequency is within the abilities of those skilled in the art, and depends upon factors such as route of administration and animal body weight. Following a period of treatment with antisense oligonucleotides, RNA is isolated from CNS tissue or CSF and changes in MECP2 nucleic acid expression are measured.

### Certain Indications

In certain embodiments, provided herein are methods of treating an individual comprising administering one or more pharmaceutical compositions described herein. In certain embodiments, the individual has a neurological disorder. In certain embodiments, the individual is at risk for developing a neurological disorder, including, but not limited to, MECP2 duplication syndrome. In certain embodiments, the individual has been identified as having a MECP2 associated disorder. In certain embodiments, provided herein are methods for prophylactically reducing MECP2 expression in an individual. Certain embodiments include treating an individual in need thereof by administering to an individual a therapeutically effective amount of an antisense compound targeted to a MECP2 nucleic acid.

In one embodiment, administration of a therapeutically effective amount of an antisense compound targeted to a MECP2 nucleic acid is accompanied by monitoring of MECP2 levels in an individual, to determine an individual's response to administration of the antisense compound. An individual's response to administration of the antisense compound may be used by a physician to determine the amount and duration of therapeutic intervention.

In certain embodiments, administration of an antisense compound targeted to a MECP2 nucleic acid results in reduction of MECP2 mRNA and or protein expression by at least 15, 20, 25, 30, 35, 40, 45, 50, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100%, or a range defined by any two of these values.

In certain embodiments, administration of an antisense compound targeted to a MECP2 nucleic acid results in improved motor function in an animal. In certain embodiments, administration of a MECP2 antisense compound improves motor function by at least 15, 20, 25, 30, 35, 40, 45, 50, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100%, or a range defined by any two of these values.

In certain embodiments, administration of an antisense compound targeted to a MECP2 nucleic acid results in improved anxiety in an animal. In certain embodiments, administration of a MECP2 antisense compound improves anxiety by at least 15, 20, 25, 30, 35, 40, 45, 50, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100%, or a range defined by any two of these values.

In certain embodiments, administration of an antisense compound targeted to a MECP2 nucleic acid results in improved social interaction in an animal. In certain embodiments, administration of a MECP2 antisense compound improves social interaction by at least 15, 20, 25, 30, 35, 40, 45, 50, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100%, or a range defined by any two of these values.

In certain embodiments, administration of an antisense compound targeted to a MECP2 nucleic acid results in improved activity in an animal. In certain embodiments, administration of a MECP2 antisense compound improves activity by at least 15, 20, 25, 30, 35, 40, 45, 50, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100%, or a range defined by any two of these values.

In certain embodiments, administration of an antisense compound targeted to a MECP2 nucleic acid results in reduction of seizures. In certain embodiments, administration of a MECP2 antisense compound reduces seizures by at least 15, 20, 25, 30, 35, 40, 45, 50, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100%, or a range defined by any two of these values.

In certain embodiments, administration of an antisense compound targeted to a MECP2 nucleic acid results in normalized EEG discharges.

In certain embodiments, pharmaceutical compositions comprising an antisense compound targeted to MECP2 are used for the preparation of a medicament for treating a patient suffering or susceptible to a neurological disorder including MECP2 duplication syndrome.

### Certain Amplicon Regions

Certain antisense oligonucleotides described herein may target the amplicon region of the primer probe set. Additional assays may be used to measure the potency and efficacy of these compounds.

### Certain Hotspot Regions

### 1. Nucleobases 28-382, 386-437, 439-464, 478-513, 519-602, 606-716, 720-789, 797-973, 977-1126, 1130-1189, 1192-1275, 1310-1337, 1440-1509, and 1514-1793

In certain embodiments, modified antisense oligonucleotides are complementary to nucleobases 28-382, 386-437, 439-464, 478-513, 519-602, 606-716, 720-789, 797-973, 977-1126, 1130-1189, 1192-1275, 1310-1337, 1440-1509, and 1514-1793 of SEQ ID NO: 2. In certain embodiments, nucleobases 28-382, 386-437, 439-464, 478-513, 519-602, 606-716, 720-789, 797-973, 977-1126, 1130-1189, 1192-1275, 1310-1337, 1440-1509, and 1514-1793 of SEQ ID NO: 2 are hotspot regions. In certain embodiments, such modified antisense oligonucleotides are 20 nucleobases in length. In certain embodiments, such modified antisense oligonucleotides are gapmers. In certain such embodiments, the gapmers are 5-10-5 MOE gapmers. In certain embodiments, the nucleosides of the modified antisense oligonucleotides are linked by phosphorothioate and phosphodiester internucleoside linkages.

The nucleobase sequences of SEQ ID Nos: 17, 18, 22-24, 50-60, 62-84, 86-93, 95-96, 99-102, 129-131, 133, 135-158, 161-171, 173-174, 177-180, 207-213, 215-237, 239-244, 246-252, 256-258, 284-288, 290, 292, 293, 296-305, 307-315, and 317-327 are complementary to nucleobases 28-382, 386-437, 439-464, 478-513, 519-602, 606-716, 720-789, 797-973, 977-1126, 1130-1189, 1192-1275, 1310-1337, 1440-1509, and 1514-1793 of SEQ ID NO: 2.

In certain embodiments, modified antisense oligonucleotides complementary to nucleobases 28-382, 386-437, 439-464, 478-513, 519-602, 606-716, 720-789, 797-973, 977-1126, 1130-1189, 1192-1275, 1310-1337, 1440-1509, and 1514-1793 of SEQ ID NO: 2 achieve at least 25% reduction of MECP2 RNA *in vitro* in the standard cell assay.

### 2. Nucleobases 44-79, 87-126, 131-273, 321-376, 478-513, 535-570, 630-716, 834-928, 930-973, 977-1004, 1081-1126, 1130-1189, 1224-1275, 1440-1509, 1514-1745, and 1750-1785

In certain embodiments, modified antisense oligonucleotides are complementary to nucleobases 44-79, 87-126, 131-273, 321-376, 478-513, 535-570, 630-716, 834-928, 930-973, 977-1004, 1081-1126, 1130-1189, 1224-1275, 1440-1509, 1514-1745, and 1750-1785 of SEQ ID NO: 2. In certain embodiments, nucleobases 44-79, 87-126, 131-273, 321-376, 478-513, 535-570, 630-716, 834-928, 930-973, 977-1004, 1081-1126, 1130-1189, 1224-1275, 1440-1509, 1514-1745, and 1750-1785 of SEQ ID NO: 2 are hotspot regions. In certain embodiments, such modified antisense oligonucleotides are 20 nucleobases in length. In certain embodiments, such modified antisense oligonucleotides are gapmers. In certain such embodiments, the gapmers are 5-10-5 MOE gapmers. In certain embodiments, the nucleosides of the modified antisense oligonucleotides are linked by phosphorothioate and phosphodiester internucleoside linkages.

The nucleobase sequences of SEQ ID Nos: 17, 18, 22-24, 50, 52, 54, 58, 63-65, 68-73, 77-79, 81, 83, 88, 90, 91, 93, 100, 102, 133, 137, 141-143, 146, 147, 154-156, 158, 161-163, 165-169, 171, 173, 174, 177-179, 210, 216, 218-220, 223, 224, 226-228, 232-234, 236, 239-242, 244, 246, 247, 251, 257, 258, 284, 287, 288, 292, 293, 298, 303, 307, 310, 311, 314, 315, 317-319, and 321-327 are complementary to nucleobases 44-79, 87-126, 131-273, 321-376, 478-513, 535-570, 630-716, 834-928, 930-973, 977-1004, 1081-1126, 1130-1189, 1224-1275, 1440-1509, 1514-1745, and 1750-1785 of SEQ ID NO: 2.

In certain embodiments, modified antisense oligonucleotides complementary to nucleobases 44-79, 87-126, 131-273, 321-376, 478-513, 535-570, 630-716, 834-928, 930-973, 977-1004, 1081-1126, 1130-1189, 1224-1275, 1440-1509, 1514-1745, and 1750-1785 of SEQ ID NO: 2 achieve at least 50% reduction of MECP2 RNA *in vitro* in the standard cell assay.

### 3. Nucleobases 1902-2000, 7300-7418, 67188-67239, 67241-67266, 67280-67315, 67321-67404, 68164-68274, 68278-68347, 68355-68531, 68535-68684, 68688-68747, 68750-68833, 68868-68895, 68998-69067, and 69072-69351

In certain embodiments, modified antisense oligonucleotides are complementary to nucleobases 1902-2000, 7300-7418, 67188-67239, 67241-67266, 67280-67315, 67321-67404, 68164-68274, 68278-68347, 68355-68531, 68535-68684, 68688-68747, 68750-68833, 68868-68895, 68998-69067, and 69072-69351 of SEQ ID NO: 1. In certain embodiments, nucleobases 1902-2000, 7300-7418, 67188-67239, 67241-67266, 67280-67315, 67321-67404, 68164-68274, 68278-68347, 68355-68531, 68535-68684, 68688-68747, 68750-68833, 68868-68895, 68998-69067, and 69072-69351 of SEQ ID NO: 1 are hotspot regions. In certain embodiments, such modified antisense oligonucleotides are 20 nucleobases in length. In certain embodiments, such modified oligonucleotides are gapmers. In certain such embodiments, the gapmers are 5-10-5 MOE gapmers. In certain embodiments, the nucleosides of the modified oligonucleotides are linked by phosphorothioate and phosphodiester internucleoside linkages.

The nucleobase sequences of SEQ ID Nos: 17, 18, 22-24, 56-60, 62-84, 86-93, 95-96, 100-102, 135-156, 158, 161-171, 173-174, 177-179, 212-213, 215-237, 239-244, 246-251, 256-258, 290-293, 296-305, 307-315, and 317-327 are complementary to nucleobases 1902-2000, 7300-7418, 67188-67239, 67241-67266, 67280-67315, 67321-67404, 68164-68274, 68278-68347, 68355-68531, 68535-68684, 68688-68747, 68750-68833, 68868-68895, 68998-69067, and 69072-69351 of

SEQ ID NO: 1.

In certain embodiments, modified oligonucleotides complementary to nucleobases 1902-2000, 7300-7418, 67188-67239, 67241-67266, 67280-67315, 67321-67404, 68164-68274, 68278-68347, 68355-68531, 68535-68684, 68688-68747, 68750-68833, 68868-68895, 68998-69067, and 69072-69351 of SEQ ID NO: 1 achieve at least 25% reduction of MECP2 RNA *in vitro* in the standard cell assay.

### 4. Nucleobases 1918-1953, 1961-2000, 7300-7418, 67123-67178, 67280-67315, 67337-67372, 68188-68274, 68392-68486, 68488-68531, 68535-68562, 68639-68684, 68688-68747, 68782-68833, 68998-69067, 69072-69303, and 69308-69343

In certain embodiments, modified antisense oligonucleotides are complementary to nucleobases 1918-1953, 1961-2000, 7300-7418, 67123-67178, 67280-67315, 67337-67372, 68188-68274, 68392-68486, 68488-68531, 68535-68562, 68639-68684, 68688-68747, 68782-68833, 68998-69067, 69072-69303, and 69308-69343 of SEQ ID NO: 1. In certain embodiments, nucleobases 1918-1953, 1961-2000, 7300-7418, 67123-67178, 67280-67315, 67337-67372, 68188-68274, 68392-68486, 68488-68531, 68535-68562, 68639-68684, 68688-68747, 68782-68833, 68998-69067, 69072-69303, and 69308-69343 of SEQ ID NO: 1 are hotspot regions. In certain embodiments, such modified oligonucleotides are 20 nucleobases in length. In certain embodiments, such modified oligonucleotides are gapmers. In certain such embodiments, the gapmers are 5-10-5 MOE gapmers. In certain embodiments, the nucleosides of the modified oligonucleotides are linked by phosphorothioate and phosphodiester internucleoside linkages.

The nucleobase sequences of SEQ ID Nos: 17, 18, 22-24, 52, 54, 58, 63-65, 68-73, 77-79, 81, 83, 88, 90, 91, 93, 100, 102, 133, 137, 141-143, 146, 147, 154-156, 158, 161-163, 165-169, 171, 173, 174, 177-179, 210, 216, 218-220, 223, 224, 226-228, 232-234, 236, 239, 240-242, 244, 246, 247, 251, 257, 258, 287, 288, 292, 293, 298, 303, 307, 310, 311, 314, 315, 317-319, and 321-327 are complementary to nucleobases 1918-1953, 1961-2000, 7300-7418, 67123-67178, 67280-67315, 67337-67372, 68188-68274, 68392-68486, 68488-68531, 68535-68562, 68639-68684, 68688-68747, 68782-68833, 68998-69067, 69072-69303, and 69308-69343 of SEQ ID NO: 1.

In certain embodiments, modified antisense oligonucleotides complementary to nucleobases 1918-1953, 1961-2000, 7300-7418, 67123-67178, 67280-67315, 67337-67372, 68188-68274, 68392-68486, 68488-68531, 68535-68562, 68639-68684, 68688-68747, 68782-68833, 68998-69067, 69072-69303, and 69308-69343 of SEQ ID NO: 1 achieve at least 50% reduction of MECP2 RNA *in vitro* in the standard cell assay.

### EXAMPLES

### Non-limiting disclosure and incorporation by reference

While certain methods described herein have been described with specificity in accordance with certain embodiments, the following examples serve only to illustrate the compounds described herein and are not intended to limit the same. Each of the references recited in the present application is incorporated herein by reference in its entirety.

### Example 1: Screening of antisense oligonucleotides targeting MECP2

Antisense oligonucleotides (ASOs) that target human Methyl CpG Binding Protein 2 (MECP2), the complement of GENBANK accession number NT_167198.1 truncated from 4203000 to 4283000, SEQ ID NO: 1, were synthesized using standard solid phase oligonucleotide synthetic methods. They are chimeric oligonucleotides ("gapmers"), composed of a central "gap" region consisting of 2'-deoxynucleotides, which is flanked on both sides (5' and 3') by "wings" that are composed of modified nucleotides. The internucleoside (backbone) linkages are phosphorothioate or phosphodiester throughout the oligonucleotides. The sequences and structures of the antisense oligonucleotides and their start and stop sites along SEQ ID NO: 1 are shown in the tables below. ASOs were designed to target exons and introns along the MECP2 pre-mRNA and some ASOs also target the mRNA. Isis Numbers 628567 (Table 1), 628553 (Table 2), 628566 (Table 3), and 628552 (Table 4) have mismatches to SEQ ID NO: 1 but are 100% complementary to human MECP2 mRNA, GENBANK accession number NM_004992.3 (SEQ ID NO: 2), with start sites of 246, 123, 238, and 115, respectively, on SEQ ID NO: 2. Isis Number 18078 does not target MECP2 and was used as a negative control.

The antisense oligonucleotides were analyzed for their effects on target mRNA levels. HepG2 cells were plated at a density of 20,000 cells per well in 96-well plates and were electroporated with 4.00 µM oligonucleotide or with no oligonucleotide for untreated controls. After approximately 24 hours, RNA was isolated from the cells, and MECP2 mRNA levels were measured by quantitative real-time PCR using primer probe set RTS4253 (forward: 5'- TGAAGGAGTCTTCTATCCGATCTGT-3', SEQ ID NO: 12; reverse: 5'-CACTTCCTTGACCTCGATGCT-3', SEQ ID NO: 13; probe: 5'-AGACCGTACTCCCCATCAAGAAGCGC-3', SEQ ID NO: 14). MECP2 mRNA levels were adjusted according to total RNA content, as measured by RIBOGREEN^{®}. Results are presented as average percent inhibition of MECP2 mRNA expression level, relative to untreated control cells, in the tables below. The levels of MECP2 mRNA in untreated control cells (UTC) represents 0% inhibition, and an undetectable level of MECP2 mRNA represents 100% inhibition. A negative inhibition value means that the level of MECP2 mRNA detected was greater than that detected in untreated control cells. The results show that many of the antisense oligonucleotides inhibited MECP2 mRNA levels. The antisense oligonucleotides marked with an asterisk (*) target the region of the primer probe set. Additional assays may be used to measure the potency and efficacy of these antisense oligonucleotides.

**Table 1: Inhibition of human MECP2 by antisense oligonucleotides in vitro**

| Isis No. | Sequence (5' to 3') | Start site | Stop site | % Inhibition | SEQ ID NO: |
|---|---|---|---|---|---|
| 18078 | Gₑₛ Tₑₛ Gₑₛ ^{m}Cₑₛ Gₑₛ C_{ds} G_{ds} C_{ds} G_{ds} A_{ds} G_{ds} C_{ds} C_{ds} C_{ds} Gₑₛ Aₑₛ Aₑₛ Aₑₛ Tₑₛ ^{m}Cₑ | n/a | n/a | 0.5 | 15 |
| 628543 | mCes Teo mCeo Teo mCeo mCds Gds Ads Gds Ads Gds Gds Ads Gds Gds Geo Aeo Ges mCes Ge | 1894 | 1913 | -1.7 | 16 |
| 628547 | Ges mCeo mCeo Aeo Teo Tds Tds Tds mCds mCds Gds Gds Ads mCds Gds Geo mCeo Tes Tes Te | 1926 | 1945 | 61.8 | 17 |
| 628551 | Tₑₛ ^{m}Cₑₒ Tₑₒ ^{m}Cₑₒ Tₑₒ ^{m}C_{ds} ^{m}C_{ds} T_{ds} ^{m}C_{ds} ^{m}C_{ds} T_{ds} ^{m}C_{ds} G_{ds} ^{m}C_{ds} ^{m}C_{ds} Tₑₒ ^{m}Cₑₒ ^{m}Cₑₛ Tₑₛ ^{m}Cₑ | 1981 | 2000 | 54.2 | 18 |
| 628739 | Aes mCeo mCeo mCeo mCeo mCds Gds mCds mCds mCds mCds mCds mCds Gds Gds mCeo Aeo Aes Ges Ge | 2036 | 2055 | 33.6 | 19 |
| 628743 | Aes Geo Aeo Geo Aeo mCds mCds Tds mCds Ads Ads mCds Tds Tds Gds Teo mCeo Aes mCes Ge | 4053 | 4072 | 36.6 | 20 |
| 628747 | ^mC_es A_eo T_eo T_eo A_eo A_ds G_ds A_ds T_ds A_ds A_ds ^mC_ds ^mC_ds A_ds T_ds ^mC_eo A_eo T_es T_es T_e | 6590 | 6609 | 43.4 | 21 |
| 628555 | Ges Geo Aeo Aeo mCeo Tds Gds Gds Tds Gds Ads Gds Tds mCds Tds Geo Teo Aes Tes Te | 7308 | 7327 | 60.7 | 22 |
| 628559 | G_es A_eo A_eo G_eo ^mC_eo A_ds A_ds G_ds G_ds T_ds G_ds T_ds A_ds T_ds T_ds ^mC_eo T_eo G_es G_es G_e | 7351 | 7370 | 62.7 | 23 |
| 628563 | ^mC_es T_eo A_eo ^mC_eo ^mC_eo A_ds T_ds G_ds G_ds A_ds A_ds T_ds ^mC_ds ^mC_ds T_ds G_eo T_eo T_es G_es G_e | 7383 | 7402 | 63.1 | 24 |
| 628751 | Tₑₛ Tₑₒ Tₑₒ Tₑₒ ^{m}Cₑₒ T_{ds} A_{ds} T_{ds} A_{ds} A_{ds} A_{ds} T_{ds} ^{m}C_{ds} ^{m}C_{ds} A_{ds} Tₑₒ Gₑₒ Tₑₛ Aₑₛ Tₑ | 9115 | 9134 | 78.3 | 25 |
| 628755 | T_es A_eo G_eo ^mC_eo ^mC_eo ^mC_ds ^mC_ds A_ds ^mC_ds T_ds ^mC_ds ^mC_ds ^mC_ds G_ds G_ds A_eo T_eo A_es A_es G_e | 11509 | 11528 | 62.1 | 26 |
| 628759 | A_es ^mC_eo T_eo ^mC_eo A_eo A_ds G_ds ^mC_ds ^mC_ds ^mC_ds A_ds A_ds G_ds G_ds A_ds G_eo T_eo T_es ^mC_es A_e | 14390 | 14409 | 39.1 | 27 |
| 628763 | Gₑₛ ^{m}Cₑₒ Tₑₒ Tₑₒ Tₑₒ A_{ds} A_{ds} T_{ds} G_{ds} ^{m}C_{ds} T_{ds} T_{ds} T_{ds} A_{ds} T_{ds} Tₑₒ Tₑₒ Tₑₛ Tₑₛ Aₑ | 17349 | 17368 | 89.3 | 28 |
| 628767 | Tes Geo mCeo mCeo Aeo Ads mCds Ads Gds mCds Ads Gds Gds mCds mCds mCeo Aeo Ges mCes Ge | 19691 | 19710 | 82.2 | 29 |
| 628771 | G_es A_eo T_eo A_eo T_eo ^mC_ds A_ds G_ds T_ds G_ds A_ds G_ds G_ds A_ds A_ds G_eo T_eo T_es G_es T_e | 22318 | 22337 | 69.0 | 30 |
| 628775 | mCes Geo Teo Geo mCeo mCds Ads Tds Gds Gds Ads Ads Gds Tds mCds mCeo Teo Tes mCes mCe | 24936 | 24955 | 82.8 | 31 |
| 628779 | Ges Geo Teo Geo Aeo Gds mCds Tds Gds Ads Tds Gds mCds Tds Ads Teo Aeo Tes Ges Ae | 27172 | 27191 | 75.8 | 32 |
| 628783 | A_es G_eo G_eo ^mC_eo G_eo G_ds ^mC_ds A_ds G_ds T_ds G_ds G_ds ^mC_ds T_ds T_ds A_eo ^mC_eo G_es ^mC_es ^mC_e | 29717 | 29736 | 35.2 | 33 |
| 628787 | Aes Geo mCeo mCeo mCeo mCds Tds Tds Ads Ads Tds Tds Tds Tds Gds Teo Teo mCes Tes mCe | 31758 | 31777 | 81.2 | 34 |
| 628791 | T_es G_eo G_eo ^mC_eo G_eo G_ds ^mC_ds T_ds ^mC_ds A_ds A_ds G_ds A_ds A_ds ^mC_ds ^mC_eo A_eo G_es ^mC_es ^mC_e | 34273 | 34292 | 39.2 | 35 |
| 628795 | ᵐCₑₛ Aₑₒ Aₑₒ Aₑₒ Tₑₒ A_ds T_ds T_ds A_ds G_ds A_ds A_ds T_ds A_ds G_ds Aₑₒ ᵐCₑₒ Tₑₛ ᵐCₑₛ Aₑ | 36288 | 36307 | 62.8 | 36 |
| 628799 | Tₑₛ Gₑₒ Gₑₒ Gₑₒ Aₑₒ ^{m}C_{ds} T_{ds} ^{m}C_{ds} A_{ds} G_{ds} A_{ds} T_{ds} T_{ds} ^{m}C_{ds} T_{ds} Aₑₒ Tₑₒ Aₑₛ Gₑₛ Gₑ | 39071 | 39090 | 67.2 | 37 |
| 628803 | Gₑₛ Tₑₒ ^{m}Cₑₒ ^{m}Cₑₒ Tₑₒ G_{ds} G_{ds} A_{ds} A_{ds} ^{m}C_{ds} G_{ds} A_{ds} ^{m}C_{ds} A_{ds} G_{ds} Gₑₒ ^{m}Cₑₒ Tₑₛ Tₑₛ Gₑ | 41073 | 41092 | 66.0 | 38 |
| 628807 | ᵐCₑₛ ᵐCₑₒ Aₑₒ Aₑₒ Aₑₒ Tₔₛ Tₔₛ Tₔₛ Aₔₛ Tₔₛ Aₔₛ Aₔₛ ᵐCₔₛ Tₔₛ Tₔₛ Aₑₒ Aₑₒ Gₑₛ Aₑₛ Aₑ | 43580 | 43599 | 18.3 | 39 |
| 628811 | Gₑₛ Gₑₒ Tₑₒ Gₑₒ Aₑₒ T_{ds} G_{ds} T_{ds} G_{ds} T_{ds} A_{ds} T_{ds} T_{ds} T_{ds} T_{ds} Aₑₒ ^{m}Cₑₒ Tₑₛ Aₑₛ ^{m}Cₑ | 45768 | 45787 | 86.2 | 40 |
| 628815 | T_es G_eo G_eo T_eo G_eo G_ds G_ds A_ds ^mC_ds A_ds A_ds A_ds A_ds A_ds T_ds T_eo G_eo T_es G_es G_e | 47850 | 47869 | 64.6 | 41 |
| 628819 | A_es A_eo A_eo T_eo A_eo A_ds G_ds ^mC_ds A_ds T_ds ^mC_ds T_ds G_ds G_ds ^mC_ds A_eo T_eo T_es T_es G_e | 49865 | 49884 | 52.8 | 42 |
| 628823 | Tₑₛ Aₑₒ ^{m}Cₑₒ Aₑₒ Tₑₒ T_{ds} G_{ds} A_{ds} A_{ds} A_{ds} A_{ds} ^{m}C_{ds} A_{ds} G_{ds} ^{m}Cₑₒ ^{m}Cₑₒ Aₑₛ Gₑₛ Aₑ | 52552 | 52571 | 17.2 | 43 |
| 628827 | Ges Geo Aeo Teo mCeo mCds Ads Tds Gds mCds Gds Ads Gds Ads Gds Aeo Aeo Ges mCes Ae | 54569 | 54588 | 29.1 | 44 |
| 628831 | T_es A_eo T_eo A_eo A_eo T_ds A_ds T_ds ^mC_ds A_ds T_ds T_ds ^mC_ds A_ds G_ds ^mC_eo ^mC_eo T_es ^mC_es A_e | 56608 | 56627 | 61.7 | 45 |
| 628835 | ^mC_es A_eo G_eo ^mC_eo A_eo G_ds G_ds A_ds A_ds G_ds A_ds G_ds T_ds ^mC_ds ^mC_ds A_eo G_eo A_es G_es A_e | 59223 | 59242 | 15.9 | 46 |
| 628839 | Aes Geo Aeo Aeo Aeo mCds mCds Tds Gds mCds mCds Ads Gds Gds Tds Geo Teo Ges Ges Te | 61278 | 61297 | 64.4 | 47 |
| 628843 | ^mC_es ^mC_eo A_eo G_eo G_eo T_ds G_ds T_ds G_ds G_ds ^mC_ds ^mC_ds ^mC_ds A_ds G_ds G_eo G_eo T_es G_es G_e | 63401 | 63420 | 20.5 | 48 |
| 628847 | Ges Geo mCeo Aeo Teo mCds mCds Tds Ads mCds Ads Ads mCds mCds mCds Aeo mCeo Aes Ges Ae | 65432 | 65451 | 58.1 | 49 |
| 628567 | Tes Geo Aeo mCeo Teo Tds Tds Tds mCds Tds Tds mCds mCds mCds Tds Geo Aeo Ges mCes mCe | 67048 | 67067 | 51.8 | 50 |
| 628571 | Ges Geo Geo Teo Teo Tds Gds Tds mCds mCds Tds Tds Gds Ads Gds Geo mCeo mCes mCes Te | 67084 | 67103 | 40.4 | 51 |
| 628575 | ᵐCₑₛ Tₑₒ ᵐCₑₒ Tₑₒ Tₑₒ ᵐCds Tds Tds Tds ᵐCds Tds Tds Ads Tds ᵐCds Tₑₒ Tₑₒ Tₑₛ ᵐCₑₛ Tₑ | 67123 | 67142 | 55.4 | 52 |
| 628579 | mCes Teo Teo Geo mCeo mCds mCds Tds mCds Tds Tds Tds mCds Tds mCds Teo Teo mCes Tes Te | 67135 | 67154 | 49.6 | 53 |
| 628583 | Tes Geo Geo mCeo Teo Gds mCds Ads mCds Gds Gds Gds mCds Tds mCds Aeo Teo Ges mCes Te | 67153 | 67172 | 57.4 | 54 |
| 628587 | G_es T_eo G_eo G_eo T_eo G_ds G_ds G_ds ^mC_ds T_ds G_ds A_ds T_ds G_ds G_ds ^mC_eo T_eo G_es ^mC_es A_e | 67165 | 67184 | 30.9 | 55 |
| 628591 | ^mC_es T_eo G_eo ^mC_eo T_eo T_ds T_ds G_ds ^mC_ds ^mC_ds T_ds G_ds ^mC_ds ^mC_ds T_ds ^mC_eo T_eo G_es ^mC_es G_e | 67196 | 67215 | 50.0 | 56 |
| 628595 | A_es A_eo G_eo ^mC_eo T_eo T_ds ^mC_ds ^mC_ds G_ds G_ds ^mC_ds A_ds ^mC_ds A_ds G_ds ^mC_eo ^mC_eo G_es G_es G_e | 67241 | 67260 | 47.3 | 57 |
| 628599 | Ges Geo Teo mCeo Aeo mCds Gds Gds Ads Tds Gds Ads Tds Gds Gds Aeo Geo mCes Ges mCe | 67280 | 67299 | 50.6 | 58 |
| 628603 | Tes Teo mCeo mCeo Geo Tds Gds Tds mCds mCds Ads Gds mCds mCds Tds Teo mCeo Aes Ges Ge | 67329 | 67348 | 28.8 | 59 |
| *628607 | mCes Aeo Geo mCeo Aeo Gds Ads Gds mCds Gds Gds mCds mCds Ads Gds Aeo Teo Tes Tes mCe | 67361 | 67380 | 33.0 | 60 |
| 628851 | Tₑₛ Gₑₒ Tₑₒ ^{m}Cₑₒ ^{m}Cₑₒ ^{m}C_{ds} T_{ds} G_{ds} ^{m}C_{ds} ^{m}C_{ds} ^{m}C_{ds} T_{ds} ^{m}C_{ds} ^{m}C_{ds} ^{m}C_{ds} Tₑₒ Gₑₒ ^{m}Cₑₛ ^{m}Cₑₛ ^{m}Cₑ | 67434 | 67453 | 20.0 | 61 |
| 628611 | Gₑₛ ^{m}Cₑₒ Gₑₒ Aₑₒ Aₑₒ A_{ds} G_{ds} G_{ds} ^{m}C_{ds} T_{ds} T_{ds} T_{ds} T_{ds} ^{m}C_{ds} ^{m}C_{ds} ^{m}Cₑₒ Tₑₒ Gₑₛ Gₑₛ Gₑ | 68164 | 68183 | 71.0 | 62 |
| 628615 | Gₑₛ Aₑₒ Aₑₒ Gₑₒ Tₑₒ A_{ds} ^{m}C_{ds} G_{ds} ^{m}C_{ds} A_{ds} A_{ds} T_{ds} ^{m}C_{ds} A_{ds} A_{ds} ^{m}Cₑₒ Tₑₒ ^{m}Cₑₛ ^{m}Cₑₛ Aₑ | 68191 | 68210 | 65.9 | 63 |
| 628619 | Gₑₛ Gₑₒ Gₑₒ Aₑₒ Tₑₒ G_{ds} T_{ds} G_{ds} T_{ds} ^{m}C_{ds} G_{ds} ^{m}C_{ds} ^{m}C_{ds} T_{ds} A_{ds} ^{m}Cₑₒ ^{m}Cₑₒ Tₑₛ Tₑₛ Tₑ | 68213 | 68232 | 62.3 | 64 |
| 628623 | ᵐCₑₛ Gₑₒ Tₑₒ Gₑₒ Aₑₒ A_ds G_ds T_ds ᵐC_ds A_ds A_ds A_ds A_ds T_ds ᵐC_ds Aₑₒ Tₑₒ Tₑₛ Aₑₛ Gₑ | 68239 | 68258 | 68.7 | 65 |
| 628627 | Tₑₛ Tₑₒ Aₑₒ Gₑₒ Gₑₒ T_{ds} G_{ds} G_{ds} T_{ds} T_{ds} T_{ds} ^{m}C_{ds} T_{ds} G_{ds} ^{m}C_{ds} Tₑₒ ^{m}Cₑₒ Tₑₛ ^{m}Cₑₛ Gₑ | 68286 | 68305 | 43.0 | 66 |
| 628631 | ᵐCₑₛ Gₑₒ Gₑₒ ᵐCₑₒ ᵐCₑₒ T_ds ᵐC_ds T_ds G_ds ᵐC_ds ᵐC_ds A_ds G_ds T_ds T_ds ᵐCₑₒ ᵐCₑₒ Tₑₛ Gₑₛ Gₑ | 68328 | 68347 | 36.5 | 67 |
| 628635 | Aes mCeo mCeo mCeo Teo Tds Tds Tds mCds Ads mCds mCds Tds Gds mCds Aeo mCeo Aes mCes mCe | 68400 | 68419 | 53.2 | 68 |
| 628639 | Aes Aeo Geo Geo Aeo Gds mCds Tds Tds mCds mCds mCds Ads Gds Gds Aeo mCeo Tes Tes Te | 68428 | 68447 | 60.0 | 69 |
| 628643 | T_es G_eo G_eo ^mC_eo G_eo A_ds A_ds G_ds T_ds T_ds T_ds G_ds A_ds A_ds A_ds A_eo G_eo G_es ^mC_es A_e | 68455 | 68474 | 57.8 | 70 |
| 628647 | A_es G_eo ^mC_eo ^mC_eo T_eo T_ds G_ds ^mC_ds ^mC_ds ^mC_ds ^mC_ds T_ds G_ds G_ds ^mC_eo G_eo A_es A_es G_e | 68467 | 68486 | 59.3 | 71 |
| 628651 | Tes Geo Geo Aeo Teo Gds Tds Gds Gds Tds Gds Gds mCds mCds mCds mCeo Aeo mCes mCes mCe | 68492 | 68511 | 50.3 | 72 |
| 628655 | Ges mCeo Teo Teo Teo Tds mCds Gds mCds Tds Tds mCds mCds Tds Gds mCeo mCeo Ges Ges Ge | 68535 | 68554 | 67.2 | 73 |
| 628659 | Gₑₛ Tₑₒ Tₑₒ Tₑₒ ^{m}Cₑₒ T_{ds} T_{ds} G_{ds} G_{ds} G_{ds} A_{ds} A_{ds} T_{ds} G_{ds} G_{ds} ^{m}Cₑₒ ^{m}Cₑₒ Tₑₛ Gₑₛ Aₑ | 68567 | 68586 | 41.1 | 74 |
| 628663 | Gₑₛ Gₑₒ ^{m}Cₑₒ Tₑₒ Gₑₒ ^{m}C_{ds} ^{m}C_{ds} A_{ds} ^{m}C_{ds} ^{m}C_{ds} A_{ds} ^{m}C_{ds} A_{ds} ^{m}C_{ds} T_{ds} ^{m}Cₑₒ ^{m}Cₑₒ ^{m}Cₑₛ ^{m}Cₑₛ Gₑ | 68599 | 68618 | 40.4 | 75 |
| *628667 | Tes Teo mCeo Aeo mCeo Gds Gds mCds Tds Tds Tds mCds Tds Tds Tds Teo Teo Ges Ges mCe | 68631 | 68650 | 38.2 | 76 |
| *628671 | mCes Teo mCeo mCeo Teo Gds mCds Ads mCds Ads Gds Ads Tds mCds Gds Geo Aeo Tes Aes Ge | 68659 | 68678 | 76.9 | 77 |
| *628675 | Tes mCeo Teo mCeo mCeo mCds Gds Gds Gds Tds mCds Tds Tds Gds mCds Geo mCeo Tes Tes mCe | 68696 | 68715 | 85.3 | 78 |
| *628679 | mCes Teo Teo mCeo Aeo mCds mCds Ads mCds Tds Tds mCds mCds Tds Tds Geo Aeo mCes mCes Te | 68728 | 68747 | 74.5 | 79 |
| 628683 | Tₑₛ ^{m}Cₑₒ Aₑₒ Gₑₒ Tₑₒ ^{m}C_{ds} ^{m}C_{ds} T_{ds} T_{ds} T_{ds} ^{m}C_{ds} ^{m}C_{ds} ^{m}C_{ds} G_{ds} ^{m}C_{ds} Tₑₒ ^{m}Cₑₒ Tₑₛ Tₑₛ ^{m}Cₑ | 68774 | 68793 | 43.0 | 80 |
| 628687 | ᵐCₑₛ Tₑₒ Tₑₒ Gₑₒ ᵐCₑₒ T_ds T_ds T_ds T_ds ᵐC_ds ᵐC_ds G_ds ᵐC_ds ᵐC_ds ᵐC_ds Aₑₒ Gₑₒ Gₑₛ Gₑₛ ᵐCₑ | 68806 | 68825 | 69.2 | 81 |
| 628691 | Ges Geo Teo Geo Aeo Tds Gds Gds Tds Gds Gds Tds Gds Gds Tds Geo mCeo Tes mCes mCe | 68876 | 68895 | 49.4 | 82 |
| 628695 | G_es ^mC_eo T_eo G_eo ^mC_eo T_ds G_ds ^mC_ds T_ds ^mC_ds A_ds A_ds G_ds T_ds ^mC_ds ^mC_eo T_eo G_es G_es G_e | 68998 | 69017 | 62.3 | 83 |
| 628699 | ^mC_es T_eo ^mC_eo ^mC_eo A_eo G_ds T_ds G_ds A_ds G_ds ^mC_ds ^mC_ds T_ds ^mC_ds ^mC_ds T_eo ^mC_eo T_es G_es G_e | 69040 | 69059 | 45.7 | 84 |
| 628703 | A_es A_eo ^mC_eo ^mC_eo G_eo ^mC_ds G_ds G_ds G_ds ^mC_ds T_ds G_ds A_ds G_ds T_ds ^mC_eo T_eo T_es A_es G_e | 69085 | 69104 | 23.7 | 85 |
| 628707 | Tₑₛ Gₑₒ Gₑₒ ^{m}Cₑₒ Gₑₒ G_{ds} ^{m}C_{ds} G_{ds} G_{ds} T_{ds} G_{ds} G_{ds} ^{m}C_{ds} A_{ds} A_{ds} ^{m}Cₑₒ ^{m}Cₑₒ Gₑₛ ^{m}Cₑₛ Gₑ | 69098 | 69117 | 43.7 | 86 |
| 628711 | Tes Teo Teo Teo mCeo Tds Gds mCds Gds Gds mCds mCds Gds Tds Gds Geo mCeo Ges Ges mCe | 69111 | 69130 | 44.4 | 87 |
| 628715 | ᵐCes Teo ᵐCeo Teo ᵐCeo ᵐCds ᵐCds Tds ᵐCds ᵐCds ᵐCds ᵐCds Tds ᵐCds Gds Geo Teo Ges Tes Te | 69136 | 69155 | 54.7 | 88 |
| 628719 | ᵐCₑₛ Tₑₒ Tₑₒ Gₑₒ Gₑₒ ᵐCdₛ Adₛ Tdₛ Gdₛ Gdₛ Adₛ Gdₛ Gdₛ Adₛ Tdₛ Gₑₒ Aₑₒ Aₑₛ Aₑₛ ᵐCₑ | 69168 | 69187 | 41.5 | 89 |
| 628723 | T_es ^mC_eo ^mC_eo G_eo G_eo ^mC_ds T_ds G_ds T_ds ^mC_ds ^mC_ds A_ds ^mC_ds A_ds G_ds G_eo ^mC_eo T_es ^mC_es ^mC_e | 69200 | 69219 | 59.6 | 90 |
| 628727 | Aes Aeo Teo mCeo mCeo Gds mCds Tds mCds mCds Gds Tds Gds Tds Ads Aeo Aeo Ges Tes mCe | 69244 | 69263 | 50.4 | 91 |
| 628731 | ᵐCₑₛ Aₑₒ Gₑₒ ᵐCₑₒ Tₑₒ Gₔₛ ᵐCₔₛ ᵐCₔₛ Tₔₛ Tₔₛ Tₔₛ Aₔₛ Tₔₛ Tₔₛ ᵐCₔₛ Tₑₒ Tₑₒ Gₑₛ Tₑₛ Tₑ | 69276 | 69295 | 43.3 | 92 |
| 628735 | Ges Teo mCeo Aeo Geo Ads Gds mCds mCds mCds Tds Ads mCds mCds mCds Aeo Teo Aes Aes Ge | 69308 | 69327 | 70.7 | 93 |

Superscript "m" indicates 5-methylcytosine. Subscripts: "o" indicates a phosphodiester internucleoside linkage, "s" indicates a phosphorothioate internucleoside linkage, "e" indicates a 2'-methoxyethyl modified nucleoside, and "d" indicates a 2'-deoxynucleoside.

**Table 2: Inhibition of human MECP2 by antisense oligonucleotides in vitro**

| Isis No. | Sequence (5' to 3') | Start site | Stop site | % Inhibition | SEQ ID NO: |
|---|---|---|---|---|---|
| 18078 | Ges Tes Ges mCes Ges Cds Gds Cds Gds Ads Gds Cds Cds Cds Ges Aes Aes Aes Tes mCe | n/a | n/a | 3.8 | 15 |
| 628541 | Aes Geo mCeo Geo mCeo Gds mCds Gds mCds Gds mCds mCds Gds mCds mCds Geo Aeo mCes Ges mCe | 1878 | 1897 | 15.3 | 94 |
| 628545 | ᵐC_es T_eo T_eo T_eo T_eo A_ds ᵐC_ds ᵐC_ds A_ds ᵐC_ds A_ds G_ds ᵐC_ds ᵐC_ds ᵐC_ds T_eo ᵐC_eo T_es ᵐC_es T_e | 1910 | 1929 | 48.4 | 95 |
| 628549 | mCes mCeo Geo mCeo Teo mCds Gds Gds mCds Gds mCds Gds Gds mCds Gds Geo mCeo Ges Ges mCe | 1953 | 1972 | 32.9 | 96 |
| 628741 | T_es ^mC_eo A_eo G_eo T_eo T_ds T_ds G_ds G_ds G_ds T_ds G_ds A_ds T_ds T_ds ^mC_eo G_eo G_es T_es ^mC_e | 3047 | 3066 | 45.7 | 97 |
| 628745 | ^mC_es A_eo G_eo ^mC_eo A_eo ^mC_ds A_ds G_ds ^mC_ds G_ds G_ds G_ds A_ds A_ds ^mC_ds A_eo ^mC_eo A_es T_es T_e | 5561 | 5580 | 42.2 | 98 |
| 628553 | Tₑₛ Aₑₒ Tₑₒ Tₑₒ Tₑₒ T_{ds} T_{ds} A_{ds} T_{ds} G_{ds} G_{ds} A_{ds} G_{ds} ^{m}C_{ds} A_{ds} Gₑₒ Tₑₒ ^{m}Cₑₛ Tₑₛ ^{m}Cₑ | 7292 | 7311 | 36.6 | 99 |
| 628557 | Aes Teo Geo Teo mCeo Ads mCds Ads Tds mCds Ads Ads Ads Gds mCds Aeo Geo Ges Aes Ae | 7324 | 7343 | 70.2 | 100 |
| 628561 | Tₑₛ Tₑₒ Gₑₒ Gₑₒ Aₑₒ G_{ds} ^{m}C_{ds} T_{ds} G_{ds} G_{ds} T_{ds} ^{m}C_{ds} T_{ds} A_{ds} ^{m}C_{ds} Aₑₒ Gₑₒ Aₑₛ Aₑₛ Gₑ | 7367 | 7386 | 43.4 | 101 |
| 628565 | A_es G_eo ^mC_eo ^mC_eo ^mC_eo T_ds A_ds A_ds ^mC_ds A_ds T_ds ^mC_ds ^mC_ds ^mC_ds A_ds G_eo ^mC_eo T_es A_es ^mC_e | 7399 | 7418 | 72.5 | 102 |
| 628749 | mCes Aeo mCeo Aeo mCeo Tds Gds Ads mCds mCds Tds Tds Tds mCds Ads Geo Geo Ges mCes Te | 7615 | 7634 | 96.4 | 103 |
| 628753 | T_es A_eo A_eo A_eo A_eo A_ds A_ds G_ds G_ds A_ds T_ds T_ds T_ds ^mC_ds ^mC_ds T_eo A_eo A_es G_es T_e | 10408 | 10427 | 12.3 | 104 |
| 628757 | Gₑₛ Tₑₒ Aₑₒ ^{m}Cₑₒ Aₑₒ ^{m}C_{ds} A_{ds} ^{m}C_{ds} A_{ds} ^{m}C_{ds} G_{ds} ^{m}C_{ds} T_{ds} T_{ds} T_{ds} Tₑₒ Tₑₒ Tₑₛ Tₑₛ Tₑ | 13332 | 13351 | 85.5 | 105 |
| 628761 | G_es A_eo A_eo A_eo G_eo ^mC_ds ^mC_ds G_ds A_ds G_ds ^mC_ds ^mC_ds T_ds G_ds G_ds ^mC_eo ^mC_eo G_es G_es G_e | 15686 | 15705 | 51.4 | 106 |
| 628765 | G_es A_eo A_eo G_eo A_eo A_ds A_ds A_ds T_ds G_ds T_ds G_ds G_ds A_ds T_ds T_eo T_eo T_es T_es T_e | 18630 | 18649 | 78.3 | 107 |
| 628769 | ^mC_es G_eo A_eo G_eo A_eo A_ds T_ds G_ds A_ds G_ds A_ds ^mC_ds T_ds ^mC_ds ^mC_ds G_eo T_eo A_es T_es ^mC_e | 21317 | 21336 | 63.6 | 108 |
| 628773 | Aes Aeo Aeo mCeo mCeo mCds Ads Ads Ads mCds mCds Ads mCds mCds Tds Teo Aeo mCes mCes mCe | 23339 | 23358 | 48.9 | 109 |
| 628777 | A_es A_eo A_eo A_eo T_eo A_ds A_ds A_ds G_ds T_ds ^mC_ds A_ds G_ds G_ds A_ds G_eo G_eo ^mC_es T_es G_e | 26037 | 26056 | 65.5 | 110 |
| 628781 | A_es A_eo A_eo A_eo A_eo T_ds G_ds G_ds A_ds G_ds G_ds G_ds ^mC_ds A_ds ^mC_ds A_eo G_eo T_es G_es G_e | 28177 | 28196 | 12.2 | 111 |
| 628785 | Ges Geo Teo Teo Teo Tds Tds mCds Tds mCds mCds Tds Tds Tds Ads Teo Teo Aes Tes mCe | 30744 | 30763 | 92.2 | 112 |
| 628789 | Tₑₛ Aₑₒ Tₑₒ Gₑₒ Tₑₒ T_{ds} G_{ds} G_{ds} ^{m}C_{ds} ^{m}C_{ds} T_{ds} A_{ds} G_{ds} A_{ds} A_{ds} ^{m}Cₑₒ Tₑₒ ^{m}Cₑₛ ^{m}Cₑₛ Tₑ | 33273 | 33292 | 52.4 | 113 |
| 628793 | T_es G_eo ^mC_eo T_eo ^mC_eo T_ds ^mC_ds A_ds T_ds A_ds T_ds T_ds ^mC_ds A_ds ^mC_ds ^mC_eo ^mC_eo A_es ^mC_es G_e | 35287 | 35306 | 79.3 | 114 |
| 628797 | Gₑₛ Tₑₒ Gₑₒ ^{m}Cₑₒ Aₑₒ G_{ds} A_{ds} G_{ds} A_{ds} ^{m}C_{ds} T_{ds} ^{m}C_{ds} A_{ds} A_{ds} G_{ds} Gₑₒ Gₑₒ Aₑₛ Gₑₛ Gₑ | 38049 | 38068 | 21.7 | 115 |
| 628801 | Ges mCeo Teo Aeo Aeo Gds mCds mCds Tds mCds mCds Tds Gds Gds Tds Geo Aeo Aes mCes mCe | 40072 | 40091 | 65.0 | 116 |
| 628805 | Ges Teo Aeo Teo Geo Ads Ads mCds Ads Tds mCds Ads Gds mCds Tds Geo Aeo mCes Ges mCe | 42580 | 42599 | 69.6 | 117 |
| 628809 | Aₑₛ Gₑₒ Gₑₒ ^{m}Cₑₒ Gₑₒ ^{m}C_{ds} G_{ds} ^{m}C_{ds} T_{ds} G_{ds} G_{ds} T_{ds} G_{ds} ^{m}C_{ds} A_{ds} Aₑₒ Gₑₒ ^{m}Cₑₛ ^{m}Cₑₛ Tₑ | 44735 | 44754 | 16.5 | 118 |
| 628813 | mCes Aeo Geo mCeo mCeo Ads mCds Tds mCds Tds Tds Tds Tds Tds Tds Teo Teo Tes Ges Ae | 46834 | 46853 | 59.1 | 119 |
| 628817 | Gₑₛ Tₑₒ Aₑₒ ^{m}Cₑₒ ^{m}Cₑₒ T_{ds} G_{ds} G_{ds} G_{ds} A_{ds} G_{ds} G_{ds} A_{ds} A_{ds} ^{m}C_{ds} Tₑₒ Aₑₒ ^{m}Cₑₛ Aₑₛ Aₑ | 48863 | 48882 | 68.0 | 120 |
| 628821 | Aes Geo Geo Geo mCeo Gds Ads Gds Ads Gds Ads Tds mCds mCds Ads Geo Geo Aes mCes Te | 50865 | 50884 | 80.7 | 121 |
| 628825 | G_es G_eo A_eo T_eo T_eo A_ds G_ds G_ds A_ds A_ds T_ds T_ds A_ds G_ds A_eo T_eo G_es ^mC_es A_e | 53552 | 53571 | 59.3 | 122 |
| 628829 | G_es G_eo A_eo A_eo A_eo G_ds ^mC_ds ^mC_ds T_ds G_ds T_ds ^mC_ds T_ds T_ds T_ds T_eo A_eo A_es A_es A_e | 55596 | 55615 | 54.0 | 123 |
| 628833 | ^mC_es ^mC_eo A_eo G_eo A_eo T_ds G_ds G_ds T_ds G_ds T_ds T_ds T_ds ^mC_ds ^mC_ds A_eo A_eo T_es T_es ^mC_e | 57622 | 57641 | 85.3 | 124 |
| 628837 | A_es ^mC_eo T_eo T_eo ^mC_eo T_ds A_ds G_ds A_ds ^mC_ds ^mC_ds G_ds G_ds G_ds ^mC_ds G_eo ^mC_eo A_es G_es T_e | 60266 | 60285 | 48.3 | 125 |
| 628841 | Ges Teo Aeo mCeo Aeo Ads Tds Gds Ads Ads Tds Gds Ads Ads mCds Teo Teo Tes Tes Te | 62361 | 62380 | 69.7 | 126 |
| 628845 | mCes Aeo Aeo Aeo mCeo Ads Tds Ads Tds mCds Tds Ads mCds Tds Gds mCeo Aeo Tes Tes mCe | 64407 | 64426 | 27.4 | 127 |
| 628849 | Aes mCeo Aeo Geo Geo Tds Ads Ads mCds mCds mCds mCds Ads Tds mCds Teo Aeo Ges Ges mCe | 66432 | 66451 | 68.5 | 128 |
| 628569 | G_es G_eo A_eo G_eo G_eo T_ds ^mC_ds ^mC_ds T_ds G_ds G_ds T_ds ^mC_ds T_ds T_ds ^mC_eo T_eo G_es A_es ^mC_e | 67064 | 67083 | 41.4 | 129 |
| 628573 | Tₑₛ Tₑₒ Aₑₒ Tₑₒ ^{m}Cₑₒ T_{ds} T_{ds} T_{ds} ^{m}C_{ds} T_{ds} T_{ds} ^{m}C_{ds} A_{ds} ^{m}C_{ds} ^{m}C_{ds} Tₑₒ Tₑₒ Tₑₛ Tₑₛ Tₑ | 67113 | 67132 | 27.1 | 130 |
| 628577 | mCes Teo mCeo Teo Teo Tds mCds Tds mCds Tds Tds mCds Tds Tds Tds mCeo Teo Tes Aes Te | 67129 | 67148 | 43.9 | 131 |
| 628581 | mCes Aeo mCeo Geo Geo Gds mCds Tds mCds Ads Tds Gds mCds Tds Tds Geo mCeo mCes mCes Te | 67147 | 67166 | 15.0 | 132 |
| 628585 | Gₑₛ Gₑₒ ᵐCₑₒ Tₑₒ Gₑₒ Aₔₛ Tₔₛ Gₔₛ Gₔₛ ᵐCₔₛ Tₔₛ Gₔₛ ᵐCₔₛ Aₔₛ ᵐCₔₛ Gₑₒ Gₑₒ Gₑₛ ᵐCₑₛ Tₑ | 67159 | 67178 | 73.7 | 133 |
| 628589 | Tes Geo mCeo Geo Geo Gds mCds Tds mCds Ads Gds mCds Ads Gds Ads Geo Teo Ges Ges Te | 67180 | 67199 | 22.6 | 134 |
| 628593 | Gₑₛ Aₑₒ ^{m}Cₑₒ ^{m}Cₑₒ ^{m}Cₑₒ T_{ds} T_{ds} ^{m}C_{ds} T_{ds} G_{ds} A_{ds} T_{ds} G_{ds} T_{ds} ^{m}C_{ds} Tₑₒ ^{m}Cₑₒ Tₑₛ Gₑₛ ^{m}Cₑ | 67212 | 67231 | 47.4 | 135 |
| 628597 | A_es G_eo G_eo ^mC_eo A_eo G_ds A_ds A_ds G_ds ^mC_ds T_ds T_ds ^mC_ds ^mC_ds G_ds G_eo ^mC_eo A_es ^mC_es A_e | 67247 | 67266 | 48.6 | 136 |
| 628601 | T_es ^mC_eo A_eo T_eo A_eo ^mC_ds A_ds T_ds G_ds G_ds G_ds T_ds ^mC_ds ^mC_ds ^mC_ds ^mC_ds ^mC_eo G_eo G_es T_es ^mC_e | 67296 | 67315 | 53.7 | 137 |
| 628605 | T_es T_eo T_eo ^mC_eo ^mC_eo T_ds T_ds T_ds G_ds ^mC_ds T_ds T_ds A_ds A_ds G_ds ^mC_eo T_eo T_es ^mC_es ^mC_e | 67345 | 67364 | 46.5 | 138 |
| 628609 | Tes Aeo mCeo Aeo mCeo Ads Tds mCds Ads Tds Ads mCds Tds Tds mCds mCeo mCeo Aes Ges mCe | 67377 | 67396 | 40.1 | 139 |
| 628613 | T_es ^mC_eo A_eo A_eo ^mC_eo T_ds ^mC_ds ^mC_ds A_ds ^mC_ds T_ds T_ds T_ds A_ds G_ds A_eo G_eo ^mC_es G_es A_e | 68180 | 68199 | 42.9 | 140 |
| 628617 | G_es ^mC_eo ^mC_eo T_eo A_eo ^mC_ds ^mC_ds T_ds T_ds T_ds T_ds ^mC_ds G_ds A_ds A_ds G_eo T_eo A_es ^mC_es G_e | 68203 | 68222 | 65.5 | 141 |
| 628621 | G_es G_eo T_eo ^mC_eo ^mC_eo A_ds G_ds G_ds G_ds A_ds T_ds G_ds T_ds G_ds T_ds ^mC_eo G_eo ^mC_es ^mC_es T_e | 68219 | 68238 | 62.9 | 142 |
| 628625 | Tes mCeo mCeo mCeo Teo mCds Tds mCds mCds mCds Ads Gds Tds Tds Ads mCeo mCeo Ges Tes Ge | 68255 | 68274 | 65.6 | 143 |
| 628629 | T_es G_eo G_eo A_eo G_eo ^mC_ds T_ds T_ds T_ds G_ds G_ds G_ds A_ds G_ds A_ds T_eo T_eo T_es G_es G_e | 68311 | 68330 | 28.3 | 144 |
| 628633 | mCes Geo Teo Geo Geo mCds mCds Gds mCds mCds Tds Tds Gds Gds Gds Teo mCeo Tes mCes Ge | 68374 | 68393 | 25.5 | 145 |
| 628637 | Aes Geo Geo Aeo mCeo Tds Tds Tds Tds mCds Tds mCds mCds Ads Gds Geo Aeo mCes mCes mCe | 68416 | 68435 | 86.1 | 146 |
| 628641 | Aes Geo Geo mCeo Aeo Tds mCds Tds Tds Gds Ads mCds Ads Ads Gds Geo Aeo Ges mCes Te | 68440 | 68459 | 81.0 | 147 |
| 628645 | Ges mCeo mCeo mCeo mCeo mCds Tds Gds Gds mCds Gds Ads Ads Gds Tds Teo Teo Ges Aes Ae | 68461 | 68480 | 29.6 | 148 |
| 628649 | ^mC_es ^mC_eo ^mC_eo ^mC_eo A_eo ^mC_ds ^mC_ds ^mC_ds ^mC_ds ^mC_ds ^mC_ds T_ds ^mC_ds A_ds G_ds ^mC_eo ^mC_eo T_es T_es G_e | 68480 | 68499 | 39.9 | 149 |
| 628653 | mCes mCeo Aeo Teo Geo Ads mCds mCds Tds Gds Gds Gds Tds Gds Gds Aeo Teo Ges Tes Ge | 68504 | 68523 | 39.7 | 150 |
| 628657 | ^mC_es T_eo G_eo A_eo G_eo G_ds G_ds T_ds ^mC_ds G_ds G_ds ^mC_ds ^mC_ds T_ds ^mC_ds A_eo G_eo ^mC_es T_es T_e | 68551 | 68570 | 28.6 | 151 |
| 628661 | ^mC_es ^mC_eo ^mC_eo G_eo G_eo ^mC_ds T_ds T_ds T_ds ^mC_ds G_ds G_ds ^mC_ds ^mC_ds ^mC_ds ^mC_eo G_eo T_es T_es T_e | 68583 | 68602 | 37.3 | 152 |
| 628665 | Tₑₛ Gₑₒ Gₑₒ ^{m}Cₑₒ ^{m}Cₑₒ T_{ds} ^{m}C_{ds} G_{ds} G_{ds} ^{m}C_{ds} G_{ds} ^{m}C_{ds} A_{ds} G_{ds} ^{m}Cₑₒ Gₑₒ Gₑₛ ^{m}Cₑₛ Tₑ | 68615 | 68634 | 38.2 | 153 |
| *628669 | Tes mCeo Geo Geo Aeo Tds Ads Gds Ads Ads Gds Ads mCds Tds mCds mCeo Teo Tes mCes Ae | 68647 | 68666 | 66.3 | 154 |
| *628673 | Tes Aeo mCeo Geo Geo Tds mCds Tds mCds mCds Tds Gds mCds Ads mCds Aeo Geo Aes Tes mCe | 68665 | 68684 | 83.3 | 155 |
| *628677 | A_es ^mC_eo ^mC_eo T_eo ^mC_eo G_ds A_ds T_ds G_ds ^mC_ds T_ds G_ds A_ds ^mC_ds ^mC_ds G_eo T_eo ^mC_es T_es ^mC_e | 68712 | 68731 | 70.8 | 156 |
| 628681 | ^mC_es T_eo T_eo ^mC_eo T_eo ^mC_ds A_ds ^mC_ds ^mC_ds G_ds A_ds G_ds G_ds G_ds T_ds G_eo G_eo A_es ^mC_es A_e | 68758 | 68777 | 24.2 | 157 |
| 628685 | Ges Geo Geo mCeo Teo mCds Tds Tds Ads mCds Ads Gds Gds Tds mCds Teo Teo mCes Aes Ge | 68790 | 68809 | 62.3 | 158 |
| 628689 | ᵐCₑₛ Tₑₒ Gₑₒ ᵐCₑₒ Tₑₒ G_ds ᵐC_ds T_ds G_ds ᵐC_ds G_ds ᵐC_ds ᵐC_ds ᵐC_ds ᵐC_ds Tₑₒ Tₑₒ Gₑₛ Gₑₛ Gₑ | 68835 | 68854 | 73.7 | 159 |
| 628693 | T_es ^mC_eo G_eo G_eo G_eo ^mC_ds T_ds ^mC_ds A_ds G_ds G_ds T_ds G_ds G_ds A_ds G_eo G_eo T_es G_es G_e | 68946 | 68965 | 13.0 | 160 |
| 628697 | mCes Teo Geo Geo Geo mCds Ads Tds mCds Tds Tds mCds Tds mCds mCds Teo mCeo Tes Tes Te | 69024 | 69043 | 59.0 | 161 |
| 628701 | Gₑₛ Tₑₒ ^{m}Cₑₒ Tₑₒ Tₑₒ A_{ds} G_{ds} ^{m}C_{ds} T_{ds} G_{ds} G_{ds} ^{m}C_{ds} T_{ds} ^{m}C_{ds} ^{m}C_{ds} Tₑₒ Tₑₒ Gₑₛ Gₑₛ Gₑ | 69072 | 69091 | 63.7 | 162 |
| 628705 | Gₑₛ Gₑₒ Tₑₒ Gₑₒ Gₑₒ ^{m}C_{ds} A_{ds} A_{ds} ^{m}C_{ds} ^{m}C_{ds} G_{ds} ^{m}C_{ds} G_{ds} G_{ds} G_{ds} ^{m}Cₑₒ Tₑₒ Gₑₛ Aₑₛ Gₑ | 69091 | 69110 | 59.5 | 163 |
| 628709 | ᵐCₑₛ Gₑₒ Gₑₒ ᵐCₑₒ ᵐCₑₒ G_ds T_ds G_ds G_ds ᵐC_ds G_ds G_ds ᵐC_ds G_ds G_ds Tₑₒ Gₑₒ Gₑₛ ᵐCₑₛ Aₑ | 69104 | 69123 | 39.5 | 164 |
| 628713 | Tₑₛ Gₑₒ Tₑₒ Tₑₒ Tₑₒ G_{ds} T_{ds} A_{ds} ^{m}C_{ds} T_{ds} T_{ds} T_{ds} T_{ds} ^{m}C_{ds} T_{ds} Gₑₒ ^{m}Cₑₒ Gₑₛ Gₑₛ ^{m}Cₑ | 69120 | 69139 | 58.1 | 165 |
| 628717 | Aes Aeo Aeo mCeo Aeo Ads Tds Gds Tds mCds Tds Tds Tds Gds mCds Geo mCeo Tes mCes Te | 69152 | 69171 | 59.3 | 166 |
| 628721 | mCes Teo mCeo mCeo Teo mCds Tds mCds Tds Gds Tds Tds Tds Gds Gds mCeo mCeo Tes Tes Ge | 69184 | 69203 | 61.6 | 167 |
| 628725 | Aes Geo Teo mCeo Aeo Gds mCds Tds Ads Ads mCds Tds mCds Tds mCds Teo mCeo Ges Ges Te | 69228 | 69247 | 66.3 | 168 |
| 628729 | Tes Geo Teo Teo Geo Gds Tds Tds Tds Gds mCds Tds Tds Tds Gds mCeo Aeo Aes Tes mCe | 69260 | 69279 | 79.7 | 169 |
| 628733 | Tes Aeo Aeo Geo Geo Ads Gds Ads Ads Gds Ads Gds Ads mCds Ads Aeo mCeo Aes Ges mCe | 69292 | 69311 | 35.1 | 170 |
| 628737 | Tes Teo Aeo Aeo Teo mCds Gds Gds Gds Ads Ads Gds mCds Tds Tds Teo Geo Tes mCes Ae | 69324 | 69343 | 78.1 | 171 |

Superscript "m" indicates 5-methylcytosine. Subscripts: "o" indicates a phosphodiester internucleoside linkage, "s" indicates a phosphorothioate internucleoside linkage, "e" indicates a 2'-methoxyethyl modified nucleoside, and "d" indicates a 2'-deoxynucleoside.

**Table 3: Inhibition of human MECP2 by antisense oligonucleotides in vitro**

| Isis No. | Sequence (5' to 3') | Start site | Stop site | % Inhibition | SEQ ID NO: |
|---|---|---|---|---|---|
| 18078 | Ges Tes Ges mCes Ges Cds Gds Cds Gds Ads Gds Cds Cds Cds Ges Aes Aes Aes Tes mCe | n/a | n/a | 0.6 | 15 |
| 628542 | Ges Aeo Geo Geo Aeo Gds Gds Gds Ads Gds mCds Gds mCds Gds mCds Geo mCeo Ges mCes mCe | 1886 | 1905 | 23.9 | 172 |
| 628546 | mCes mCeo Geo Geo Aeo mCds Gds Gds mCds Tds Tds Tds Tds Ads mCds mCeo Aeo mCes Aes Ge | 1918 | 1937 | 60.4 | 173 |
| 628550 | mCes Teo mCeo mCeo Teo mCds mCds Tds mCds mCds Gds mCds Tds mCds Gds Geo mCeo Ges mCes Ge | 1961 | 1980 | 67.0 | 174 |
| 628742 | A_es T_eo G_eo ^mC_eo T_eo T_ds ^mC_ds A_ds T_ds T_ds T_ds T_ds T_ds A_ds ^mC_ds A_eo G_eo T_es A_es T_e | 3547 | 3566 | 82.9 | 175 |
| 628746 | Ges Aeo Geo mCeo mCeo Ads Gds Ads Gds Gds mCds Tds Gds Gds Gds Teo Geo mCes Ges Ge | 6078 | 6097 | 38.7 | 176 |
| 628554 | T_es G_eo A_eo G_eo T_eo ^mC_ds T_ds G_ds T_ds A_ds T_ds T_ds T_ds T_ds T_ds A_eo T_eo G_es G_es A_e | 7300 | 7319 | 57.7 | 177 |
| 628558 | Gₑₛ Gₑₒ Aₑₒ Gₑₒ Tₑₒ ^{m}C_{ds} A_{ds} ^{m}C_{ds} A_{ds} T_{ds} G_{ds} T_{ds} ^{m}C_{ds} A_{ds} ^{m}C_{ds} Aₑₒ Tₑₒ ^{m}Cₑₛ Aₑₛ Aₑ | 7332 | 7351 | 82.8 | 178 |
| 628562 | Gₑₛ Aₑₒ Aₑₒ Tₑₒ ^{m}Cₑₒ ^{m}C_{ds} T_{ds} G_{ds} T_{ds} T_{ds} G_{ds} G_{ds} A_{ds} G_{ds} ^{m}C_{ds} Tₑₒ Gₑₒ Gₑₛ Tₑₛ ^{m}Cₑ | 7375 | 7394 | 67.3 | 179 |
| 628566 | ^mC_es T_eo T_eo ^mC_eo ^mC_eo ^mC_ds T_ds G_ds A_ds G_ds ^mC_ds ^mC_ds ^mC_ds T_ds A_ds A_eo ^mC_eo A_es T_es ^mC_e | 7407 | 7426 | 32.1 | 180 |
| 628750 | ^mC_es ^mC_eo ^mC_eo A_eo ^mC_eo A_ds G_ds ^mC_ds A_ds G_ds T_ds A_ds A_ds A_ds A_ds G_eo A_eo G_es A_es A_e | 8615 | 8634 | 54.0 | 181 |
| 628754 | A_es ^mC_eo ^mC_eo ^mC_eo ^mC_eo A_ds G_ds T_ds A_ds G_ds T_ds T_ds G_ds A_ds G_ds A_eo T_eo T_es A_es ^mC_e | 11009 | 11028 | 40.8 | 182 |
| 628758 | A_es T_eo A_eo G_eo T_eo A_ds G_ds T_ds T_ds G_ds ^mC_ds ^mC_ds A_ds G_ds A_ds G_eo G_eo G_es T_es G_e | 13862 | 13881 | 38.7 | 183 |
| 628762 | Ges Geo mCeo Teo Teo mCds Tds Ads Tds Tds Gds Tds Ads Ads Ads Aeo mCeo Tes Aes Te | 16687 | 16706 | 95.8 | 184 |
| 628766 | A_es ^mC_eo T_eo G_eo G_eo T_ds T_ds T_ds T_ds A_ds A_ds G_ds A_ds G_ds A_eo T_eo G_es G_es G_e | 19134 | 19153 | 83.1 | 185 |
| 628770 | T_es A_eo A_eo A_eo A_eo T_ds ^mC_ds T_ds A_ds T_ds G_ds G_ds G_ds A_ds A_ds T_eo A_eo A_es A_es A_e | 21818 | 21837 | -7.4 | 186 |
| 628774 | Gₑₛ Aₑₒ Aₑₒ Aₑₒ Tₑₒ G_{ds} T_{ds} G_{ds} G_{ds} G_{ds} ^{m}C_{ds} T_{ds} T_{ds} G_{ds} G_{ds} ^{m}Cₑₒ Aₑₒ Tₑₛ Gₑₛ Gₑ | 23936 | 23955 | 62.6 | 187 |
| 628778 | A_es A_eo ^mC_eo A_eo T_eo G_ds G_ds T_ds T_ds T_ds A_ds G_ds T_ds A_ds G_ds A_eo A_eo A_es ^mC_es ^mC_e | 26672 | 26691 | 72.8 | 188 |
| 628782 | Ges Geo Teo Aeo Teo Tds Ads Tds Ads Tds Tds Tds Tds Gds Teo Aeo Aes Tes Te | 28682 | 28701 | 49.9 | 189 |
| 628786 | ^mC_es A_eo A_eo ^mC_eo A_eo T_ds T_ds ^mC_ds ^mC_ds A_ds T_ds T_ds T_ds A_ds T_ds T_eo T_eo A_es G_es G_e | 31258 | 31277 | 90.5 | 190 |
| 628790 | A_es T_eo T_eo T_eo T_eo ^mC_ds A_ds ^mC_ds ^mC_ds ^mC_ds T_ds T_ds T_ds A_ds A_ds A_eo A_eo A_es T_es ^mC_e | 33773 | 33792 | 63.0 | 191 |
| 628794 | Tₑₛ Aₑₒ Aₑₒ Tₑₒ Aₑₒ ^{m}C_{ds} A_{ds} G_{ds} T_{ds} G_{ds} A_{ds} ^{m}C_{ds} A_{ds} A_{ds} G_{ds} ^{m}Cₑₒ Aₑₒ Tₑₛ ^{m}Cₑₛ ^{m}Cₑ | 35787 | 35806 | 68.0 | 192 |
| 628798 | Tes mCeo mCeo Aeo Teo mCds Tds Tds Gds mCds Ads Gds Gds Tds Gds Geo Aeo Ges Tes Ae | 38549 | 38568 | 77.6 | 193 |
| 628802 | Gₑₛ Aₑₒ Aₑₒ Gₑₒ ^{m}Cₑₒ ^{m}C_{ds} A_{ds} A_{ds} A_{ds} A_{ds} A_{ds} A_{ds} G_{ds} ^{m}C_{ds} A_{ds} Aₑₒ ^{m}Cₑₒ Aₑₛ Aₑₛ Aₑ | 40573 | 40592 | 60.5 | 194 |
| 628806 | ^mC_es ^mC_eo A_eo A_eo G_eo A_ds ^mC_ds A_ds A_ds G_ds G_ds A_ds A_ds A_ds A_ds A_eo ^mC_eo G_es G_es G_e | 43080 | 43099 | 57.5 | 195 |
| 628810 | ᵐCₑₛ Tₑₒ Aₑₒ Gₑₒ ᵐCₑₒ T_ds A_ds T_ds ᵐC_ds A_ds G_ds ᵐC_ds T_ds G_ds G_ds Gₑₒ ᵐCₑₒ Aₑₛ Tₑₛ Gₑ | 45258 | 45277 | 66.7 | 196 |
| 628814 | Tₑₛ Gₑₒ ^{m}Cₑₒ ^{m}Cₑₒ Tₑₒ T_{ds} G_{ds} T_{ds} T_{ds} G_{ds} G_{ds} G_{ds} T_{ds} A_{ds} G_{ds} Tₑₒ Aₑₒ ^{m}Cₑₛ Aₑₛ Gₑ | 47334 | 47353 | 72.0 | 197 |
| 628818 | Ges mCeo Teo Aeo Aeo Gds Tds Tds Ads Gds Ads Ads mCds Tds mCds mCeo Geo Tes Ges Ge | 49363 | 49382 | 49.4 | 198 |
| 628822 | A_es ^mC_eo A_eo ^mC_eo G_eo ^mC_ds ^mC_ds T_ds G_ds T_ds A_ds A_ds T_ds ^mC_ds ^mC_ds T_eo G_eo ^mC_es A_es T_e | 51552 | 51571 | 81.5 | 199 |
| 628826 | mCes Aeo Aeo mCeo Teo Gds Gds Ads Gds Gds mCds mCds Gds Gds Gds mCeo Geo mCes Ges Ae | 54069 | 54088 | 9.7 | 200 |
| 628830 | Aes Geo mCeo mCeo mCeo Ads mCds Ads mCds Ads Gds mCds Tds Gds Tds mCeo Teo mCes Aes Ge | 56096 | 56115 | 41.4 | 201 |
| 628834 | Tes Teo mCeo mCeo Teo mCds Ads Tds Gds Ads Ads Tds Gds Tds Gds Aeo mCeo mCes Tes Ge | 58122 | 58141 | 47.6 | 202 |
| 628838 | Gₑₛ Aₑₒ Gₑₒ Gₑₒ Aₑₒ A_{ds} ^{m}C_{ds} T_{ds} T_{ds} G_{ds} T_{ds} ^{m}C_{ds} T_{ds} G_{ds} A_{ds} Gₑₒ Aₑₒ Tₑₛ ^{m}Cₑₛ Aₑ | 60766 | 60785 | 68.3 | 203 |
| 628842 | ᵐCes Aeo Geo ᵐCeo Teo Ads ᵐCds Tds ᵐCds Gds ᵐCds Tds Ads Gds Ads Aeo Aeo Ges Ges Ge | 62880 | 62899 | 70.8 | 204 |
| 628846 | mCes Teo mCeo mCeo mCeo mCds Ads Tds Ads Ads Ads Gds Gds Ads Gds Geo Geo Aes Ges Ge | 64930 | 64949 | 1.6 | 205 |
| 628850 | ᵐCes ᵐCeo Aeo Teo ᵐCeo Ads Tds Ads ᵐCds Ads ᵐCds Tds ᵐCds Ads Gds Aeo Teo ᵐCes Tes Te | 66932 | 66951 | 62.6 | 206 |
| 628570 | Tₑₛ Tₑₒ Gₑₒ Aₑₒ Gₑₒ G_{ds} ^{m}C_{ds} ^{m}C_{ds} ^{m}C_{ds} T_{ds} G_{ds} G_{ds} A_{ds} G_{ds} G_{ds} Tₑₒ ^{m}Cₑₒ ^{m}Cₑₛ Tₑₛ Gₑ | 67074 | 67093 | 31.1 | 207 |
| 628574 | Tₑₛ Tₑₒ ^{m}Cₑₒ Tₑₒ Tₑₒ T_{ds} ^{m}C_{ds} T_{ds} T_{ds} A_{ds} T_{ds} ^{m}C_{ds} T_{ds} T_{ds} T_{ds} ^{m}Cₑₒ Tₑₒ Tₑₛ ^{m}Cₑₛ Aₑ | 67120 | 67139 | 39.8 | 208 |
| 628578 | Ges mCeo mCeo mCeo Teo mCds Tds Tds Tds mCds Tds mCds Tds Tds mCds Teo Teo Tes mCes Te | 67132 | 67151 | 46.7 | 209 |
| 628582 | mCes Teo Geo mCeo Aeo mCds Gds Gds Gds mCds Tds mCds Ads Tds Gds mCeo Teo Tes Ges mCe | 67150 | 67169 | 69.0 | 210 |
| 628586 | Ges Teo Geo Geo Geo mCds Tds Gds Ads Tds Gds Gds mCds Tds Gds mCeo Aeo mCes Ges Ge | 67162 | 67181 | 31.7 | 211 |
| 628590 | ᵐCₑₛ ᵐCₑₒ Tₑₒ Gₑₒ ᵐCₑₒ ᵐCds Tds ᵐCds Tds Gds ᵐCds Gds Gds Gds ᵐCds Tₑₒ ᵐCₑₒ Aₑₛ Gₑₛ ᵐCₑ | 67188 | 67207 | 39.4 | 212 |
| 628594 | mCes Geo Geo Aeo Geo mCds mCds Tds Gds Ads mCds mCds mCds Tds Tds mCeo Teo Ges Aes Te | 67220 | 67239 | 50.9 | 213 |
| 628598 | Gₑₛ Gₑₒ Gₑₒ Aₑₒ Gₑₒ G_{ds} ^{m}C_{ds} A_{ds} G_{ds} A_{ds} A_{ds} G_{ds} ^{m}C_{ds} T_{ds} T_{ds} ^{m}Cₑₒ ^{m}Cₑₒ Gₑₛ Gₑₛ ^{m}Cₑ | 67250 | 67269 | 15.8 | 214 |
| 628602 | ᵐCₑₛ ᵐCₑₒ Aₑₒ Gₑₒ ᵐCₑₒ ᵐCds Tds Tds ᵐCds Ads Gds Gds ᵐCds Ads Gds Gₑₒ Gₑₒ Tₑₛ Gₑₛ Gₑ | 67321 | 67340 | 37.1 | 215 |
| 628606 | mCes Geo Geo mCeo mCeo Ads Gds Ads Tds Tds Tds mCds mCds Tds Tds Teo Geo mCes Tes Te | 67353 | 67372 | 55.6 | 216 |
| 628610 | Tes Geo Aeo Teo mCeo Ads Ads Ads Tds Ads mCds Ads mCds Ads Tds mCeo Aeo Tes Aes mCe | 67385 | 67404 | 53.0 | 217 |
| 628614 | Ges Teo Aeo mCeo Geo mCds Ads Ads Tds mCds Ads Ads mCds Tds mCds mCeo Aeo mCes Tes Te | 68188 | 68207 | 83.9 | 218 |
| 628618 | Tes Geo Teo Geo Teo mCds Gds mCds mCds Tds Ads mCds mCds Tds Tds Teo Teo mCes Ges Ae | 68209 | 68228 | 50.1 | 219 |
| 628622 | mCes Aeo Aeo Aeo Aeo Tds mCds Ads Tds Tds Ads Gds Gds Gds Tds mCeo mCeo Aes Ges Ge | 68231 | 68250 | 58.6 | 220 |
| 628626 | Tes Teo Teo mCeo Teo Gds mCds Tds mCds Tds mCds Gds mCds mCds Gds Geo Geo Aes Ges Ge | 68278 | 68297 | 35.9 | 221 |
| 628630 | mCes mCeo Aeo Geo Teo Tds mCds mCds Tds Gds Gds Ads Gds mCds Tds Teo Teo Ges Ges Ge | 68319 | 68338 | 51.3 | 222 |
| 628634 | mCes Aeo mCeo mCeo Teo Gds mCds Ads mCds Ads mCds mCds mCds Tds mCds Teo Geo Aes mCes Ge | 68392 | 68411 | 53.6 | 223 |
| 628638 | Ges Aeo Geo mCeo Teo Tds mCds mCds mCds Ads Gds Gds Ads mCds Tds Teo Teo Tes mCes Te | 68425 | 68444 | 57.1 | 224 |
| 628642 | G_es T_eo T_eo T_eo G_eo A_ds A_ds A_ds A_ds G_ds G_ds ^mC_ds A_ds T_ds ^mC_ds T_eo T_eo G_es A_es ^mC_e | 68448 | 68467 | 40.8 | 225 |
| 628646 | mCes Teo Teo Geo mCeo mCds mCds mCds Tds Gds Gds mCds Gds Ads Aeo Geo Tes Tes Te | 68464 | 68483 | 58.5 | 226 |
| 628650 | T_es G_eo T_eo G_eo G_eo T_ds G_ds G_ds ^mC_ds ^mC_ds ^mC_ds ^mC_ds A_ds ^mC_ds ^mC_ds ^mC_eo ^mC_eo ^mC_es ^mC_es T_e | 68488 | 68507 | 57.7 | 227 |
| 628654 | Tₑₛ Tₑₒ Tₑₒ Gₑₒ Aₑₒ T_{ds} ^{m}C_{ds} A_{ds} ^{m}C_{ds} ^{m}C_{ds} A_{ds} T_{ds} G_{ds} A_{ds} ^{m}C_{ds} ^{m}Cₑₒ Tₑₒ Gₑₛ Gₑₛ Gₑ | 68512 | 68531 | 69.1 | 228 |
| 628658 | G_es G_eo A_eo A_eo T_eo G_ds G_ds ^mC_ds ^mC_ds T_ds G_ds A_ds G_ds G_ds G_ds T_eo ^mC_eo G_es G_es ^mC_e | 68559 | 68578 | 35.4 | 229 |
| 628662 | ᵐCes ᵐCeo Aeo ᵐCeo Aeo ᵐCds Tds ᵐCds ᵐCds ᵐCds ᵐCds Gds Gds ᵐCds Tds Teo Teo ᵐCes Ges Ge | 68591 | 68610 | 58.1 | 230 |
| 628666 | Tes Teo Teo mCeo Teo Tds Tds Tds Tds Gds Gds mCds mCds Tds mCds Geo Geo mCes Ges Ge | 68623 | 68642 | 47.3 | 231 |
| 628670 | mCes Teo Geo mCeo Aeo mCds Ads Gds Ads Tds mCds Gds Gds Ads Tds Aeo Geo Aes Aes Ge | 68656 | 68675 | 81.0 | 232 |
| *628674 | Ges Teo mCeo Teo Teo Gds mCds Gds mCds Tds Tds mCds Tds Tds Gds Aeo Teo Ges Ges Ge | 68688 | 68707 | 69.4 | 233 |
| *628678 | mC_es T_eo T_eo mC_eo mC_eo T_ds T_ds G_ds A_ds mC_ds mC_ds T_ds mC_ds G_ds A_ds T_eo G_eo mC_es T_es G_e | 68720 | 68739 | 95.8 | 234 |
| 628682 | T_es T_eo ^mC_eo ^mC_eo ^mC_eo G_ds ^mC_ds T_ds ^mC_ds T_ds T_ds ^mC_ds T_ds ^mC_ds A_ds ^mC_eo ^mC_eo G_es A_es G_e | 68766 | 68785 | 45.1 | 235 |
| 628686 | Tₑₛ ^{m}Cₑₒ ^{m}Cₑₒ Gₑₒ ^{m}Cₑₒ ^{m}C_{ds} ^{m}C_{ds} A_{ds} G_{ds} G_{ds} G_{ds} ^{m}C_{ds} T_{ds} ^{m}C_{ds} T_{ds} Tₑₒ Aₑₒ ^{m}Cₑₛ Aₑₛ Gₑ | 68798 | 68817 | 53.0 | 236 |
| 628690 | Tₑₛ Gₑₒ Gₑₒ Tₑₒ Gₑₒ G_{ds} T_{ds} G_{ds} ^{m}C_{ds} T_{ds} ^{m}C_{ds} ^{m}C_{ds} T_{ds} T_{ds} ^{m}C_{ds} Tₑₒ Tₑₒ Gₑₛ Gₑₛ Gₑ | 68868 | 68887 | 87.2 | 237 |
| 628694 | ᵐCₑₛ Gₑₒ Gₑₒ Aₑₒ Gₑₒ ᵐC_ds T_ds ᵐC_ds T_ds ᵐC_ds G_ds G_ds G_ds ᵐC_ds T_ds ᵐCₑₒ Aₑₒ Gₑₛ Gₑₛ Tₑ | 68954 | 68973 | 79.7 | 238 |
| 628698 | A_es G_eo ^mC_eo ^mC_eo T_eo ^mC_ds ^mC_ds T_ds ^mC_ds T_ds G_ds G_ds G_ds ^mC_ds A_ds T_eo ^mC_eo T_es T_es ^mC_e | 69032 | 69051 | 51.1 | 239 |
| 628702 | ^mC_es G_eo G_eo G_eo ^mC_eo T_ds G_ds A_ds G_ds T_ds ^mC_ds T_ds T_ds A_ds G_ds ^mC_eo T_eo G_es G_es ^mC_e | 69080 | 69099 | 69.6 | 240 |
| 628706 | Gₑₛ Gₑₒ ^{m}Cₑₒ Gₑₒ Gₑₒ T_{ds} G_{ds} G_{ds} ^{m}C_{ds} A_{ds} A_{ds} ^{m}C_{ds} ^{m}C_{ds} G_{ds} ^{m}C_{ds} Gₑₒ Gₑₒ Gₑₛ ^{m}Cₑₛ Tₑ | 69094 | 69113 | 62.7 | 241 |
| 628710 | T_es ^mC_eo T_eo G_eo ^mC_eo G_ds G_ds ^mC_ds ^mC_ds G_ds T_ds G_ds G_ds ^mC_ds G_ds G_eo ^mC_eo G_es G_es T_e | 69108 | 69127 | 54.2 | 242 |
| 628714 | mCes mCeo mCeo mCeo Teo mCds Gds Gds Tds Gds Tds Tds Tds Gds Tds Aeo mCeo Tes Tes Te | 69128 | 69147 | 47.5 | 243 |
| 628718 | Ges Geo Aeo Geo Geo Ads Tds Gds Ads Ads Ads mCds Ads Ads Tds Geo Teo mCes Tes Te | 69160 | 69179 | 53.6 | 244 |
| 628722 | Tes mCeo mCeo Aeo mCeo Ads Gds Gds mCds Tds mCds mCds Tds mCds Tds mCeo Teo Ges Tes Te | 69192 | 69211 | 24.4 | 245 |
| 628726 | ^mC_es ^mC_eo G_eo T_eo G_eo T_ds A_ds A_ds A_ds G_ds T_ds ^mC_ds A_ds G_ds ^mC_ds T_eo A_eo A_es ^mC_es T_e | 69236 | 69255 | 65.9 | 246 |
| 628730 | Tes Teo Teo Aeo Teo Tds mCds Tds Tds Gds Tds Tds Gds Gds Tds Teo Teo Ges mCes Te | 69268 | 69287 | 65.8 | 247 |
| 628734 | ᵐCₑₛ ᵐCₑₒ Tₑₒ Aₑₒ ᵐCₑₒ ᵐCₔₛ ᵐCₔₛ Aₔₛ Tₔₛ Aₔₛ Aₔₛ Gₔₛ Gₔₛ Aₔₛ Gₔₛ Aₑₒ Aₑₒ Gₑₛ Aₑₛ Gₑ | 69300 | 69319 | 48.9 | 248 |
| 628738 | Tes Aeo Teo Teo Teo mCds Ads Gds Tds Tds Ads Ads Tds mCds Gds Geo Geo Aes Aes Ge | 69332 | 69351 | 47.8 | 249 |

Superscript "m" indicates 5-methylcytosine. Subscripts: "o" indicates a phosphodiester internucleoside linkage, "s" indicates a phosphorothioate internucleoside linkage, "e" indicates a 2'-methoxyethyl modified nucleoside, and "d" indicates a 2'-deoxynucleoside.

**Table 4: Inhibition of human MECP2 by antisense oligonucleotides in vitro**

| Isis No. | Sequence (5' to 3') | Start site | Stop site | % Inhibition | SEQ ID NO: |
|---|---|---|---|---|---|
| 18078 | Gₑₛ Tₑₛ Gₑₛ ^{m}Cₑₛ Gₑₛ C_{ds} G_{ds} C_{ds} G_{ds} A_{ds} G_{ds} C_{ds} C_{ds} C_{ds} Gₑₛ Aₑₛ Aₑₛ Aₑₛ Tₑₛ ^{m}Cₑ | n/a | n/a | -9.9 | 15 |
| 628544 | Aes mCeo Aeo Geo mCeo mCds mCds Tds mCds Tds mCds Tds mCds mCds Gds Aeo Geo Aes Ges Ge | 1902 | 1921 | 45.6 | 250 |
| 628548 | ^mC_es G_eo G_eo ^mC_eo G_eo G_ds ^mC_ds G_ds G_ds ^mC_ds ^mC_ds A_ds T_ds T_ds T_ds T_eo ^mC_eo ^mC_es G_es G_e | 1934 | 1953 | 69.6 | 251 |
| 628552 | T_es G_eo G_eo A_eo G_eo ^mC_ds A_ds G_ds T_ds ^mC_ds T_ds ^mC_ds T_ds ^mC_ds ^mC_ds T_eo ^mC_eo ^mC_es T_es ^mC_e | 1989 | 2008 | 40.3 | 252 |
| 628740 | Tₑₛ Tₑₒ ^{m}Cₑₒ Aₑₒ Tₑₒ G_{ds} G_{ds} A_{ds} A_{ds} T_{ds} G_{ds} G_{ds} G_{ds} ^{m}C_{ds} G_{ds} Aₑₒ Gₑₒ Aₑₛ Aₑₛ Gₑ | 2547 | 2566 | 23.4 | 253 |
| 628744 | Aes mCeo Aeo Geo Aeo Gds Gds mCds Ads Gds Gds Gds mCds Ads Gds Geo mCeo Aes mCes Ge | 4561 | 4580 | 62.6 | 254 |
| 628748 | A_es A_eo G_eo A_eo T_eo T_ds ^mC_ds A_ds T_ds G_ds ^mC_ds T_ds T_ds G_ds T_ds T_eo A_eo G_es A_es A_e | 7090 | 7109 | 39.7 | 255 |
| 628556 | T_es ^mC_eo A_eo A_eo A_eo G_ds ^mC_ds A_ds G_ds G_ds A_ds A_ds ^mC_ds T_ds G_ds G_eo T_eo G_es A_es G_e | 7316 | 7335 | 40.7 | 256 |
| 628560 | Gₑₛ Gₑₒ Tₑₒ ^{m}Cₑₒ Tₑₒ A_{ds} ^{m}C_{ds} A_{ds} G_{ds} A_{ds} A_{ds} G_{ds} ^{m}C_{ds} A_{ds} A_{ds} Gₑₒ Gₑₒ Tₑₛ Gₑₛ Tₑ | 7359 | 7378 | 76.3 | 257 |
| 628564 | mCes Aeo Teo mCeo mCeo mCds Ads Gds mCds Tds Ads mCds mCds Ads Tds Geo Geo Aes Aes Te | 7391 | 7410 | 63.3 | 258 |
| 628752 | ^mC_es A_eo ^mC_eo ^mC_eo A_eo T_ds ^mC_ds ^mC_ds T_ds G_ds A_ds G_ds G_ds ^mC_ds ^mC_ds A_eo G_eo G_es ^mC_es A_e | 9908 | 9927 | 86.2 | 259 |
| 628756 | Tₑₛ Aₑₒ Aₑₒ ^{m}Cₑₒ Tₑₒ T_{ds} T_{ds} T_{ds} T_{ds} T_{ds} ^{m}C_{ds} T_{ds} A_{ds} T_{ds} T_{ds} Aₑₒ Tₑₒ Tₑₛ Aₑₛ Tₑ | 12623 | 12642 | 14.8 | 260 |
| 628760 | A_es ^mC_eo A_eo G_eo T_eo ^mC_ds A_ds ^mC_ds A_ds G_ds A_ds A_ds ^mC_ds A_ds A_ds ^mC_eo A_eo A_es A_es G_e | 14890 | 14909 | 80.6 | 261 |
| 628764 | Ges Geo mCeo mCeo Teo Ads Ads Tds Tds Tds Tds Tds Ads Tds mCeo Teo Tes Tes Ge | 17865 | 17884 | 80.6 | 262 |
| 628768 | Aes mCeo Aeo Geo Geo Gds Tds Tds Gds Tds Ads Gds mCds mCds Ads Teo mCeo Aes Ges mCe | 20758 | 20777 | 91.0 | 263 |
| 628772 | Gₑₛ Aₑₒ Tₑₒ ^{m}Cₑₒ Aₑₒ ^{m}C_{ds} T_{ds} G_{ds} G_{ds} A_{ds} A_{ds} ^{m}C_{ds} A_{ds} ^{m}C_{ds} A_{ds} Aₑₒ Tₑₒ Gₑₛ Gₑₛ Tₑ | 22839 | 22858 | 91.6 | 264 |
| 628776 | Ges Geo Aeo Aeo Geo Ads Gds Ads Ads Ads Ads Gds Ads Ads Gds Geo Geo mCes Aes mCe | 25437 | 25456 | 43.3 | 265 |
| 628780 | ^mC_es A_eo T_eo T_eo T_eo A_ds A_ds T_ds A_ds A_ds A_ds T_ds A_ds A_ds A_ds T_eo ^mC_eo ^mC_es ^mC_es T_e | 27672 | 27691 | 21.9 | 266 |
| 628784 | Tes Teo Teo Aeo mCeo mCds Ads Gds Tds Gds mCds mCds Ads Tds Tds Teo Teo Tes mCes mCe | 30227 | 30246 | 58.2 | 267 |
| 628788 | ^mC_es A_eo G_eo ^mC_eo A_eo A_ds A_ds T_ds T_ds T_ds ^mC_ds T_ds G_ds T_ds G_ds G_eo T_eo T_es T_es T_e | 32258 | 32277 | 94.0 | 268 |
| 628792 | Ges mCeo Teo mCeo Teo mCds Ads Gds Ads mCds mCds Ads Gds Ads mCds mCeo Aeo Ges Aes mCe | 34773 | 34792 | 78.3 | 269 |
| 628796 | Aes mCeo Aeo Geo mCeo Tds Gds Ads Tds Gds Ads Gds Gds Ads Gds Geo Geo Tes Ges Ge | 37542 | 37561 | 11.6 | 270 |
| 628800 | Tes Aeo mCeo Aeo mCeo Ads Ads Ads Tds Ads mCds Tds Ads Ads Gds mCeo mCeo Aes mCes Ae | 39572 | 39591 | 76.1 | 271 |
| 628804 | Aes mCeo Teo Geo mCeo mCds Ads mCds mCds Ads mCds mCds Ads Tds Gds Aeo mCeo Tes Aes Ae | 41573 | 41592 | 63.7 | 272 |
| 628808 | G_es T_eo T_eo A_eo G_eo A_ds A_ds G_ds T_ds T_ds G_ds A_ds T_ds T_ds T_ds T_eo T_eo T_es ^mC_es T_e | 44142 | 44161 | 80.7 | 273 |
| 628812 | A_es T_eo A_eo ^mC_eo T_eo ^mC_ds A_ds ^mC_ds A_ds T_ds G_ds G_ds T_ds G_ds G_ds A_eo G_eo A_es A_es A_e | 46268 | 46287 | 52.5 | 274 |
| 628816 | G_es A_eo G_eo A_eo A_eo G_ds A_ds A_ds T_ds G_ds G_ds A_ds A_ds G_ds G_ds G_eo A_eo G_es A_es A_e | 48363 | 48382 | 21.0 | 275 |
| 628820 | Tₑₛ Aₑₒ Gₑₒ Aₑₒ Gₑₒ G_{ds} G_{ds} T_{ds} T_{ds} G_{ds} G_{ds} A_{ds} G_{ds} G_{ds} A_{ds} Aₑₒ ^{m}Cₑₒ Aₑₛ Gₑₛ Gₑ | 50365 | 50384 | 25.7 | 276 |
| 628824 | ᵐCes Teo Teo Aeo Geo Ads Ads ᵐCds Ads Ads Ads Gds Ads Gds Ads Aeo Geo Aes Aes Te | 53052 | 53071 | -5.2 | 277 |
| 628828 | Gₑₛ Aₑₒ ^{m}Cₑₒ Aₑₒ ^{m}Cₑₒ T_{ds} G_{ds} A_{ds} ^{m}C_{ds} A_{ds} ^{m}C_{ds} T_{ds} G_{ds} T_{ds} G_{ds} ^{m}Cₑₒ Aₑₒ Tₑₛ Gₑₛ Aₑ | 55069 | 55088 | 67.5 | 278 |
| 628832 | Ges Geo Aeo Geo Teo Tds Ads mCds mCds Ads Tds Ads Tds Gds Ads mCeo mCeo Tes Ges Ge | 57122 | 57141 | 68.6 | 279 |
| 628836 | ᵐCes Geo Teo Aeo Aeo Gds ᵐCds Tds Tds ᵐCds Tds Ads Gds ᵐCds Ads Aeo Geo Ges Aes Ge | 59723 | 59742 | 70.0 | 280 |
| 628840 | Ges Geo Teo Aeo Aeo Ads Ads Ads Tds Gds Ads Tds Ads Ads Ads Aeo Aeo Aes mCes Ge | 61802 | 61821 | -9.7 | 281 |
| 628844 | A_es G_eo ^mC_eo ^mC_eo T_eo T_ds ^mC_ds T_ds ^mC_ds ^mC_ds T_ds G_ds ^mC_ds ^mC_ds T_ds ^mC_eo A_eo G_es ^mC_es T_e | 63907 | 63926 | 82.8 | 282 |
| 628848 | A_es G_eo A_eo A_eo G_eo ^mC_ds A_ds G_ds ^mC_ds A_ds G_ds ^mC_ds ^mC_ds A_ds ^mC_ds ^mC_eo T_eo G_es ^mC_es G_e | 65932 | 65951 | 14.3 | 283 |
| 628568 | Tes Geo Geo Teo mCeo Tds Tds mCds Tds Gds Ads mCds Tds Tds Tds Teo mCeo Tes Tes mCe | 67056 | 67075 | 56.8 | 284 |
| 628572 | mCes Aeo mCeo mCeo Teo Tds Tds Tds Tds Ads Ads Ads mCds Tds Tds Geo Aeo Ges Ges Ge | 67102 | 67121 | 74.9 | 285 |
| 628576 | Tes Teo Teo mCeo Teo mCds Tds Tds mCds Tds Tds Tds mCds Tds Tds Aeo Teo mCes Tes Te | 67126 | 67145 | 47.3 | 286 |
| 628580 | T_es ^mC_eo A_eo T_eo G_eo ^mC_ds T_ds T_ds G_ds ^mC_ds ^mC_ds ^mC_ds T_ds ^mC_ds T_ds T_eo T_eo ^mC_es T_es ^mC_e | 67140 | 67159 | 57.4 | 287 |
| 628584 | Tes Geo Aeo Teo Geo Gds mCds Tds Gds mCds Ads mCds Gds Gds Gds mCeo Teo mCes Aes Te | 67156 | 67175 | 58.1 | 288 |
| 628588 | mCes Aeo Geo mCeo Aeo Gds Ads Gds Tds Gds Gds Tds Gds Gds Gds mCeo Teo Ges Aes Te | 67172 | 67191 | 10.9 | 289 |
| 628592 | Tes Geo Aeo Teo Geo Tds mCds Tds mCds Tds Gds mCds Tds Tds Tds Geo mCeo mCes Tes Ge | 67204 | 67223 | 37.9 | 290 |
| 628596 | mCes Aeo Geo Aeo Aeo Gds mCds Tds Tds mCds mCds Gds Gds mCds Ads mCeo Aeo Ges mCes mCe | 67244 | 67263 | 16.0 | 291 |
| 628600 | G_es G_eo G_eo T_eo ^mC_eo ^mC_ds ^mC_ds ^mC_ds G_ds G_ds T_ds ^mC_ds A_ds ^mC_ds G_ds G_eo A_eo T_es G_es A_e | 67288 | 67307 | 50.5 | 292 |
| 628604 | Gₑₛ ^{m}Cₑₒ Tₑₒ Tₑₒ Aₑₒ A_{ds} G_{ds} ^{m}C_{ds} T_{ds} T_{ds} ^{m}C_{ds} ^{m}C_{ds} G_{ds} T_{ds} G_{ds} Tₑₒ ^{m}Cₑₒ ^{m}Cₑₛ Aₑₛ Gₑ | 67337 | 67356 | 59.6 | 293 |
| 628608 | A_es T_eo A_eo ^mC_eo T_eo T_ds ^mC_ds ^mC_ds ^mC_ds A_ds G_ds ^mC_ds A_ds G_ds A_ds G_eo ^mC_eo G_es G_es ^mC_e | 67369 | 67388 | 19.9 | 294 |
| 628852 | A_es G_eo ^mC_eo A_eo A_eo ^mC_ds ^mC_ds A_ds A_ds A_ds G_ds A_ds G_ds T_ds ^mC_ds A_eo G_eo G_es ^mC_es ^mC_e | 67934 | 67953 | 76.1 | 295 |
| 628612 | A_es ^mC_eo T_eo T_eo T_eo A_ds G_ds A_ds G_ds ^mC_ds G_ds A_ds A_ds A_ds G_ds G_eo ^mC_eo T_es T_es T_e | 68172 | 68191 | 36.2 | 296 |
| 628616 | T_es T_eo T_eo T_eo ^mC_eo G_ds A_ds A_ds G_ds T_ds A_ds ^mC_ds G_ds ^mC_ds A_ds A_eo T_eo ^mC_es A_es A_e | 68196 | 68215 | 41.9 | 297 |
| 628620 | mCes mCeo Aeo Geo Geo Gds Ads Tds Gds Tds Gds Tds mCds Gds mCds mCeo Teo Aes mCes mCe | 68216 | 68235 | 62.3 | 298 |
| 628624 | ᵐCₑₛ ᵐCₑₒ Aₑₒ Gₑₒ Tₑₒ Tₔₛ Aₔₛ ᵐCₔₛ ᵐCₔₛ Gₔₛ Tₔₛ Gₔₛ Aₔₛ Aₔₛ Gₔₛ Tₑₒ ᵐCₑₒ Aₑₛ Aₑₛ Aₑ | 68247 | 68266 | 40.4 | 299 |
| 628628 | Tₑₛ Gₑₒ Gₑₒ Gₑₒ ^{m}Cₑₒ T_{ds} T_{ds} ^{m}C_{ds} T_{ds} T_{ds} A_{ds} G_{ds} G_{ds} T_{ds} G_{ds} Gₑₒ Tₑₒ Tₑₛ Tₑₛ ^{m}Cₑ | 68294 | 68313 | 53.5 | 300 |
| 628632 | Ges Teo Geo Geo Teo Gds mCds mCds Gds mCds Tds mCds mCds mCds Tds Teo Teo Ges Ges Ge | 68355 | 68374 | 65.8 | 301 |
| 628636 | T_es ^mC_eo T_eo ^mC_eo ^mC_eo A_ds G_ds G_ds A_ds ^mC_ds ^mC_ds ^mC_ds T_ds T_ds T_ds T_eo ^mC_eo A_es ^mC_es ^mC_e | 68408 | 68427 | 38.7 | 302 |
| 628640 | Ges Aeo mCeo Aeo Aeo Gds Gds Ads Gds mCds Tds Tds mCds mCds mCds Aeo Geo Ges Aes mCe | 68431 | 68450 | 57.4 | 303 |
| 628644 | mCes mCeo mCeo Teo Geo Gds mCds Gds Ads Ads Gds Tds Tds Tds Gds Aeo Aeo Aes Aes Ge | 68458 | 68477 | 37.8 | 304 |
| 628648 | ᵐCₑₛ ᵐCₑₒ ᵐCₑₒ ᵐCₑₒ Tₑₒ ᵐCₔₛ Aₔₛ Gₔₛ ᵐCₔₛ ᵐCₔₛ Tₔₛ Tₔₛ Gₔₛ ᵐCₔₛ ᵐCₔₛ ᵐCₑₒ ᵐCₑₒ ᵐCₑₛ Tₑₛ Gₑ | 68473 | 68492 | 25.0 | 305 |
| 628652 | Tₑₛ Gₑₒ Gₑₒ Gₑₒ Tₑₒ G_{ds} G_{ds} A_{ds} T_{ds} G_{ds} T_{ds} G_{ds} G_{ds} T_{ds} G_{ds} Gₑₒ ^{m}Cₑₒ ^{m}Cₑₛ ^{m}Cₑₛ ^{m}Cₑ | 68496 | 68515 | 13.6 | 306 |
| 628656 | mCes Geo Geo mCeo mCeo Tds mCds Ads Gds mCds Tds Tds Tds Tds mCds Geo mCeo Tes Tes mCe | 68543 | 68562 | 59.4 | 307 |
| 628660 | T_es ^mC_eo G_eo G_eo ^mC_eo ^mC_ds ^mC_ds G_ds T_ds T_ds T_ds ^mC_ds T_ds T_ds G_eo G_eo G_es A_es A_e | 68575 | 68594 | 33.8 | 308 |
| 628664 | Ges mCeo Geo Geo mCeo Ads Gds mCds Gds Gds mCds Tds Gds mCds mCds Aeo mCeo mCes Aes mCe | 68607 | 68626 | 31.0 | 309 |
| *628668 | A_es A_eo G_eo A_eo ^mC_eo T_ds ^mC_ds ^mC_ds T_ds T_ds ^mC_ds A_ds ^mC_ds G_ds G_ds ^mC_eo T_eo T_es T_es ^mC_e | 68639 | 68658 | 69.2 | 310 |
| *628672 | G_es G_eo T_eo ^mC_eo T_eo ^mC_ds ^mC_ds T_ds G_ds ^mC_ds A_ds ^mC_ds A_ds G_ds A_ds T_eo ^mC_eo G_es G_es A_e | 68662 | 68681 | 95.2 | 311 |
| *628676 | Gₑₛ ^{m}Cₑₒ Tₑₒ Gₑₒ Aₑₒ ^{m}C_{ds} ^{m}C_{ds} G_{ds} T_{ds} ^{m}C_{ds} T_{ds} ^{m}C_{ds} ^{m}C_{ds} ^{m}C_{ds} G_{ds} Gₑₒ Gₑₒ Tₑₛ ^{m}Cₑₛ Tₑ | 68704 | 68723 | 40.4 | 312 |
| 628680 | mCes Geo Aeo Geo Geo Gds Tds Gds Gds Ads mCds Ads mCds mCds Ads Geo mCeo Aes Ges Ge | 68750 | 68769 | 37.2 | 313 |
| 628684 | A_es ^mC_eo A_eo G_eo G_eo T_ds ^mC_ds T_ds T_ds ^mC_ds A_ds G_ds T_ds ^mC_ds ^mC_ds T_eo T_eo T_es ^mC_es ^mC_e | 68782 | 68801 | 63.5 | 314 |
| 628688 | mCes Teo Geo mCeo Teo mCds Tds mCds mCds Tds Tds Gds mCds Tds Tds Teo Teo mCes mCes Ge | 68814 | 68833 | 84.1 | 315 |
| 628692 | ^mC_es T_eo G_eo A_eo G_eo T_ds G_ds G_ds T_ds G_ds G_ds T_ds G_ds A_ds T_ds G_eo G_eo T_es G_es G_e | 68885 | 68904 | 11.9 | 316 |
| 628696 | mCes Teo Teo mCeo Teo mCds mCds Tds mCds Tds Tds Tds Gds mCds Ads Geo Aeo mCes Ges mCe | 69016 | 69035 | 70.9 | 317 |
| 628700 | ᵐCₑₛ ᵐCₑₒ Gₑₒ Tₑₒ ᵐCₑₒ Gₔₛ ᵐCₔₛ Tₔₛ ᵐCₔₛ Tₔₛ ᵐCₔₛ ᵐCₔₛ Aₔₛ Gₔₛ Tₔₛ Gₑₒ Aₑₒ Gₑₛ ᵐCₑₛ ᵐCₑ | 69048 | 69067 | 71.6 | 318 |
| 628704 | Ges Geo mCeo Aeo Aeo mCds mCds Gds mCds Gds Gds Gds mCds Tds Gds Aeo Geo Tes mCes Te | 69088 | 69107 | 72.3 | 319 |
| 628708 | ^mC_es ^mC_eo G_eo T_eo G_eo G_ds ^mC_ds G_ds G_ds ^mC_ds G_ds G_ds T_ds G_ds G_ds ^mC_eo A_eo A_es ^mC_es ^mC_e | 69101 | 69120 | 32.3 | 320 |
| 628712 | Tₑₛ Aₑₒ ^{m}Cₑₒ Tₑₒ Tₑₒ T_{ds} T_{ds} ^{m}C_{ds} T_{ds} G_{ds} ^{m}C_{ds} G_{ds} G_{ds} ^{m}C_{ds} ^{m}C_{ds} Gₑₒ Tₑₒ Gₑₛ Gₑₛ ^{m}Cₑ | 69114 | 69133 | 55.9 | 321 |
| 628716 | T_es ^mC_eo T_eo T_eo T_eo G_ds ^mC_ds G_ds ^mC_ds T_ds ^mC_ds T_ds ^mC_ds ^mC_ds ^mC_ds T_eo ^mC_eo ^mC_es ^mC_es ^mC_e | 69144 | 69163 | 52.4 | 322 |
| 628720 | Tₑₛ Gₑₒ Tₑₒ Tₑₒ Tₑₒ G_{ds} G_{ds} ^{m}C_{ds} ^{m}C_{ds} T_{ds} T_{ds} G_{ds} G_{ds} ^{m}C_{ds} A_{ds} Tₑₒ Gₑₒ Gₑₛ Aₑₛ Gₑ | 69176 | 69195 | 55.1 | 323 |
| 628724 | Aes Aeo mCeo Teo mCeo Tds mCds Tds mCds Gds Gds Tds mCds Ads mCds Geo Geo Ges mCes Ge | 69220 | 69239 | 82.2 | 324 |
| 628728 | Tes Geo mCeo Teo Teo Tds Gds mCds Ads Ads Tds mCds mCds Gds mCds Teo mCeo mCes Ges Te | 69252 | 69271 | 77.9 | 325 |
| 628732 | Aes Geo Aeo Geo Aeo mCds Ads Ads mCds Ads Gds mCds Tds Gds mCds mCeo Teo Tes Tes Ae | 69284 | 69303 | 57.7 | 326 |
| 628736 | Ges Aeo Aeo Geo mCeo Tds Tds Tds Gds Tds mCds Ads Gds Ads Gds mCeo mCeo mCes Tes Ae | 69316 | 69335 | 90.1 | 327 |

Superscript "m" indicates 5-methylcytosine. Subscripts: "o" indicates a phosphodiester internucleoside linkage, "s" indicates a phosphorothioate internucleoside linkage, "e" indicates a 2'-methoxyethyl modified nucleoside, and "d" indicates a 2'-deoxynucleoside.

### Example 2: Dose response of antisense oligonucleotides targeting MECP2 in vitro

MECP2 targeting antisense oligonucleotides selected from Tables 1-4 were tested for dose response analysis in HepG2 cells. Isis Number 141923 does not target MECP2 and was used as a negative control. Cells were electroporated with 0, 0.111, 0.333, 1.00, 3.00, or 9.00 µM antisense oligonucleotide, and MECP2 mRNA was analyzed as described in Example 1. Results are presented in Tables 5 and 6 below. Isis Numbers 141923 and 628749 were included in both data sets as references for comparison. The results show that the antisense oligonucleotides targeting MECP2 inhibited MECP2 mRNA expression in a dose dependent manner.

**Table 5: Dose repsonse in vitro**

| Isis No. | % Inhibition | | | | | SEQ ID NO: |
|---|---|---|---|---|---|---|
| | 0.111 µM | 0.333 µM | 1.00 µM | 3.00 µM | 9.00 µM | |
| 141923 | 101.7 | 124.0 | 97.3 | 105.3 | 70.6 | 328 |
| 628688 | 89.1 | 73.1 | 47.8 | 24.3 | 17.0 | 315 |
| 628724 | 83.6 | 76.8 | 41.2 | 17.4 | 14.1 | 324 |
| 628736 | 84.6 | 68.9 | 36.3 | 21.4 | 9.1 | 327 |
| 628749 | 63.8 | 36.4 | 19.3 | 7.7 | 3.7 | 103 |
| 628751 | 102.3 | 77.4 | 39.2 | 19.5 | 8.3 | 25 |
| 628752 | 76.0 | 77.0 | 47.4 | 28.0 | 18.4 | 259 |
| 628763 | 63.5 | 37.1 | 11.5 | 8.1 | 6.7 | 28 |
| 628767 | 82.2 | 56.7 | 33.0 | 16.0 | 12.0 | 29 |
| 628768 | 98.4 | 68.4 | 43.7 | 21.7 | 11.0 | 263 |
| 628772 | 84.3 | 60.6 | 34.4 | 13.7 | 5.4 | 264 |
| 628775 | 84.3 | 62.4 | 37.0 | 15.9 | 6.3 | 31 |
| 628787 | 81.8 | 60.5 | 38.6 | 26.6 | 10.0 | 34 |
| 628788 | 79.8 | 65.1 | 35.9 | 10.5 | 4.9 | 268 |
| 628811 | 69.1 | 46.8 | 20.6 | 22.1 | 4.2 | 40 |
| 628844 | 82.6 | 76.4 | 49.6 | 38.1 | 16.2 | 282 |

**Table 6: Dose repsonse in vitro**

| Isis No. | % Inhibition | | | | | SEQ ID NO: |
|---|---|---|---|---|---|---|
| | 0.111 µM | 0.333 µM | 1.00 µM | 3.00 µM | 9.00 µM | |
| 141923 | 116.6 | 123.1 | 120.7 | 119.6 | 119.0 | 328 |
| 628558 | 94.5 | 65.4 | 49.2 | 19.9 | 9.2 | 178 |
| 628614 | 85.8 | 84.4 | 60.5 | 28.9 | 15.3 | 218 |
| 628637 | 93.1 | 80.1 | 63.4 | 25.3 | 8.3 | 146 |
| 628641 | 100.4 | 81.8 | 55.3 | 24.3 | 11.2 | 147 |
| 628690 | 101.7 | 77.9 | 51.5 | 28.0 | 16.4 | 237 |
| 628694 | 101.6 | 86.5 | 50.6 | 25.1 | 13.9 | 238 |
| 628742 | 103.3 | 73.6 | 48.8 | 20.1 | 14.7 | 175 |
| 628749 | 78.3 | 45.6 | 15.8 | 9.1 | 9.9 | 103 |
| 628757 | 88.6 | 70.5 | 39.0 | 21.7 | 13.5 | 105 |
| 628762 | 67.5 | 47.3 | 22.8 | 8.1 | 18.2 | 184 |
| 628766 | 119.8 | 77.5 | 65.6 | 31.5 | 18.0 | 185 |
| 628785 | 72.5 | 45.8 | 25.8 | 15.1 | 18.9 | 112 |
| 628786 | 85.6 | 55.5 | 36.0 | 17.3 | 10.6 | 190 |
| 628822 | 88.4 | 84.3 | 45.8 | 36.6 | 11.5 | 199 |
| 628833 | 90.6 | 70.1 | 55.2 | 32.1 | 10.8 | 124 |

### Example 3: Effect of antisense oligonucleotides targeting MECP2 in vivo

Antisense oligonucleotides described in Example 1 (hereinabove) were analyzed for their effects on MECP2 mRNA and protein levels in transgenic MECP2 duplication mice that overexpress wild type human MECP2 (F1 hybrid MECP2-TG1 mice(FVB/N x 129)(Samaco et al., Nat Genet, 2012). At 8 weeks of age, FVB/N x 129 mice display hypoactivity in the open field test, increased anxiety in the open field and elevated plus maze tests, abnormal social behavior in the 3-chamber test, and increased motor coordination in the rotarod test. Seven week old *MECP2*-TG mice were given stereotactic intracerebral injection of 500 µg of an antisense oligonucleotide listed in Table 7 or saline into the right ventricle of the brain. Wild type mice were given stereotactic intracerebral injection of saline into the right ventricle of the brain as a control. Each group consisted of two or three mice. Two weeks following the injection, the mice were sacrificed, and cortical brain samples were collected for analysis of MECP2 mRNA and protein levels. MECP2 and GAPDH protein levels were analyzed by western blot performed on the cortical sample lysates. Rabbit antiserum raised against the N-terminus of MECP2 and mouse anti-GAPDH 6C5 (Advanced Immunochemicals, Long Beach, CA) were used as the primary antibodies. Western blot images were quanitifed using Image J software, and the MECP2 protein levels normalized to GAPDH levels are shown in Table 7 below.

Total MECP2 mRNA, human MECP2 mRNA (both the e1 and e2 isoforms), and mouse MECP2 mRNA (both the e1 and e2 isoforms) were separeately analyzed by RT-qPCR. The primers common to human and mouse used for total MECP2 mRNA were: 5'-TATTTGATCAATCCCCAGGG-3', SEQ ID NO: 3 , and 5'-CTCCCTCTCCCAGTTACCGT-3', SEQ ID NO: 4. The human specific primers used for MECP2-e1 were 5'-AGGAGAGACTGGAAGAAAAGTC-3', SEQ ID NO: 5, and 5'-CTTGAGGGGTTTGTCCTTGA-3', SEQ ID NO: 6. The human specific primers used for MECP2-e2 were 5'-CTCACCAGTTCCTGCTTTGATGT-3', SEQ ID NO: 7, and 5'-CTTGAGGGGTTTGTCCTTGA-3', SEQ ID NO: 6. The mouse specific primers used for MECP2-e1 were 5'-AGGAGAGACTGGAGGAAAAGTC-3', SEQ ID NO: 8, and 5'-CTTAAACTTCAGTGGCTTGTCTCTG-3', SEQ ID NO: 9. The mouse specific primers used for MECP2-e2 were 5'-CTCACCAGTTCCTGCTTTGATGT-3', SEQ ID NO: 7, and 5'-CTTAAACTTCAGTGGCTTGTCTCTG-3', SEQ ID NO: 9. MECP2 mRNA levels were normalized to Hprt mRNA levels, which were analyzed using primer 5'-CGGGGGACATAAAAGTTATTG-3', SEQ ID NO: 10, and 5'-TGCATTGTTTTACCAGTGTCAA-3', SEQ ID NO: 11. Results are presented in Table 7 below as average normlized MECP2 mRNA levels relative to saline treated wild type (WT) mice. The results show that all of the antisense oligonucleotides tested inhibited MECP2 mRNA and protein levels in the transgenic mice, and human MECP2 mRNA levels were specifically inhibited, whereas mouse MECP2 mRNA levels were not inhibited. Isis Number 628785 was the most potent in the first experiments and was carried forward. Entries listed as "n/a" indicate that the corresponding experiment was not performed.

**Table 7: MECP2 mRNA and protein levels in transgenic mice following ASO administration**

| Mouse/Isis No. | MECP2 protein level | Total MECP2 mRNA | Human mRNA | | Mouse mRNA | | SEQ ID NO. |
|---|---|---|---|---|---|---|---|
| | | | MECP2-e1 isoform | MECP2-e2 isoform | MECP2-e 1 isoform | MECP2-e2 isoform | |
| WT/PBS | 1.0 | 1.0 | 0.0 | 0.0 | 1.0 | 1.3 | |
| TG/PBS | 2.0 | 3.3 | 0.9 | 8.3 | 1.2 | 1.4 | |
| TG/628724 | 1.5 | 2.0 | n/a | n/a | n/a | n/a | 324 |
| TG/628749 | 1.6 | 2.5 | n/a | n/a | n/a | n/a | 103 |
| TG/628772 | 1.7 | 2.7 | n/a | n/a | n/a | n/a | 264 |
| TG/628775 | 1.4 | 2.1 | n/a | n/a | n/a | n/a | 31 |
| TG/628785 | 1.3 | 1.6 | 0.3 | 2.3 | 1.0 | 1.3 | 112 |

### Example 4: Effect of gradual infusion of antisense oligonucleotide targeting MECP2 in vivo

In order to gradually infuse antisense oligonucleotide into the right ventricle of the brain, micro-osmotic pumps (Alzet model 1004, Durect, Cupertino, CA) were filled with 500µg of Isis No. 628785 or a control oligonucleotide that is not targeted to MECP2, dissolved in 100µl saline. The pump was then connected through a plastic catheter to a cannula (Alzet Brain Infusion Kit 3, Durect, Cupertino, CA). The pump was designed to deliver the drug at a rate of 0.11µl per hour for 28 days. The cannula and pump assembly was primed in sterile saline for two days at 37°C. Mice were anesthetized with isoflurane and placed on a computer-guided stereotaxic instrument (Angle Two Stereotaxic Instrument, Leica Microsystems, Bannockburn, IL). Anesthesia (isoflurane 3%) was continuously delivered via a small face mask. Ketoprofen 5mg/kg was administered subcutaneously at the initiation of the surgery. After sterilizing the surgical site with betadine and 70% alcohol, a midline incision was made over the skull and a subcutaneous pocket was generated on the back of the animal. Next, the pump was inserted into the pocket and the cannula was stereotactically implanted to deliver the drug in the right ventricle using the following coordinates: AP = -0.2 mm, ML = 1 mm, DV = -3 mm. The incision was sutured shut. Carprofen-containing food pellets were provided for 5 days after the surgery. 28 days after the initiation of the treatment the pump was disconnected from the cannula and removed. Two additional weeks were given to the animals to recover.

Isis No. 628785 was gradually infused into the right ventricles of the brains of 7-week old WT or TG mice using the micro-osmotic pumps. Each treatment group consisted of 4 or 5 animals. At the end of the four-week treatment period, western blot was performed as described in Example 3 to analyze MECP2 protein levels at 4, 8, and 12 weeks following the initiation of antisense oligonucleotide treatment. The results are shown in Table 8 below.

**Table 8: MECP2 protein levels following antisense oligonucleotide infusion**

| Mouse/Isis No. | MECP2 protein level (relative to WT/Control) | | |
|---|---|---|---|
| | 4 weeks | 8 weeks | 12 weeks |
| WT/Control | 1.0 | 1.0 | 1.0 |
| TG/Control | 2.9 | 2.7 | 2.3 |
| TG/628785 | 1.6 | 1.8 | 2.2 |

### Example 5: Behavioral effects of antisense oligonucleotide targeting human MECP2 in vivo

Following infusion of antisense oligonucleotide as described in Example 4, a battery of behavioral assays were performed to assess phenotypic effects of oligonucleotide treatment in TG mice treated with Isis No. 628785 or a control oligonucleotide and WT mice treated with a control oligonucleotide. Each treatment group contained at least 15 animals.

An open field test was performed two weeks and six weeks after the completion of the 4 week infusion by placing mice into the center of an open arena after habituation in the test room (40 x 40 x 30 cm). Their behavior was tracked by laser photobeam breaks for 30 min. Horizontal locomotor activity, rearing activity, time spent in the center of the arena, and entries to the center were analyzed using AccuScan Fusion software (Omnitech, Columbus, OH). The results are reported in table 9 below. The results show that the TG mice displayed hypoactivity in the open field test relative to WT mice at both time points, and treatment of TG mice with Isis No. 628785 restored activity close to WT levels.

**Table 9: Open field test**

| Mouse/Isis No. | Horizontal activity (activity counts) | | Rearing episodes | | Time in center (s) | | Entries to center | |
|---|---|---|---|---|---|---|---|---|
| | 2 weeks | 6 weeks | 2 weeks | 6 weeks | 2 weeks | 6 weeks | 2 weeks | 6 weeks |
| WT/Control | 7134 | 5632 | 277 | 236 | 179 | n/a | 147 | 103 |
| TG/Control | 4116 | 3493 | 156 | 106 | 105 | n/a | 65 | 45 |
| TG/628785 | 5550 | 6114 | 170 | 205 | 93 | n/a | 75 | 99 |

Mice were tested in an elevated plus maze two weeks and six weeks after the completion of the 4 week infusion. After habituation in the test room, mice were placed in the center part of the maze facing one of the two open arms. Mouse behavior was video-tracked for 10 minutes, and the time the mice spent in the open arms and the entries to the open arms were recorded and analyzed using ANY-maze system (Stoelting, Wood Dale, IL). The results are shown in Table 10 below. The results show that the TG mice displayed hypoactivity in the open field test relative to WT mice at both time points, and treatment of TG mice with Isis No. 628785 restored activity close to WT levels. The results show that the TG mice displayed hypoactivity in the elevated plus maze test relative to WT mice at both time points, and treatment of TG mice with Isis No. 628785 restored activity close to WT levels.

**Table 10: Elevated plus maze**

| Mouse/Isis No. | Time in open arms (s) | | Entries into open arms | |
|---|---|---|---|---|
| | 2 weeks | 6 weeks | 2 weeks | 6 weeks |
| WT/Control | 139 | 81 | 21 | 12 |
| TG/Control | 76 | 13 | 12 | 2 |
| TG/628785 | 91 | 55 | 11 | 7 |

Mice were assessed in a three-chamber social interaction test three weeks and seven weeks after the completion of the 4 week infusion. The apparatus comprised a clear Plexiglas box with removable partitions that separated the box into three chambers: left, central, and right. In the left and right chambers a cylindrical wire cup was placed with the open side down. Age and gender-matched mice were used as novel partners. Two days before the test, the novel partner mice were habituated to the wire cups (3 inches diameter by 4 inches in height) for 1 hour per day. After habituation in the test room, each mouse was placed in the central chamber and allowed to explore the three chambers for 10 minutes (habituation phase). The time spent in each chamber during the habituation phase was recorded automatically and analyzed using ANY-maze system (Stoelting, Wood Dale, IL). Next, a novel partner mouse was placed under a wire cup in either the left or the right chamber. An inanimate object was placed as a control under the wire cup of the opposite chamber. The location of the novel mouse was randomized between the left and right chambers for each test mouse to control for side preference. The mouse tested was allowed to explore again for an additional 10 minutes. The time spent investigating the novel partner (defined by rearing, sniffing or pawing at the wire cup) and the time spent investigating the inanimate object were measured manually. The results are shown in Table 11 below. The results show that the TG mice displayed hypoactivity and decreased social interaction in the three-chamber social interaction test relative to WT mice at both time points, and treatment of TG mice with Isis No. 628785 restored social interaction with a novel partner to WT levels at the 6 week time point.

**Table 11: Three-chamber social interaction test**

| Mouse/Isis No. | Time spent investigating chambers during habituation phase (s) | | | | Time spent investigating novel partner or inanimate object (s) | | | |
|---|---|---|---|---|---|---|---|---|
| | Left | | Right | | Novel partner | | Inanimate object | |
| | 2 weeks | 6 weeks | 2 weeks | 6 weeks | 2 weeks | 6 weeks | 2 weeks | 6 weeks |
| WT/Control | 52.4 | n/a | 44.1 | n/a | 141 | 107 | 38 | 37 |
| TG/Control | 35.5 | n/a | 29.9 | n/a | 106 | 59 | 33 | 28 |
| TG/628785 | 37.7 | n/a | 23.0 | n/a | 94 | 106 | 27 | 28 |

Mice were assessed in an accelerating rotarod test three weeks after the completion of the 4 week infusion. After habituation in the test room, motor coordination was measured using an accelerating rotarod apparatus (Ugo Basile, Varese, Italy). Mice were tested 2 consecutive days, 4 trials each, with an interval of 60 minutes between trials to rest. Each trial lasted for a maximum of 10 minutes; mice that never fell were given a measurement of 600 seconds. The rod accelerated from 4 to 40 r.p.m. in the first 5 minutes. The time that it took for each mouse to fall from the rod (latency to fall) was recorded. Results are shown in Table 12 below. The results show that the TG mice displayed increased performance in the rotarod test relative to WT mice, and treatment of TG mice with Isis No. 628785 restored performance to WT levels.

**Table 12: Accelerating rotarod test**

| Mouse/Isis No. | Latency to fall (s) | |
|---|---|---|
| | Day 1 | Day 2 |
| WT/Control | 174 | 300 |
| TG/Control | 275 | 400 |
| TG/628785 | 183 | 282 |

The results in tables 9-12 above show that treatment with Isis No. 628785 targeting MECP2 reversed behavioral phenotypes of the TG mice. The TG mice treated with Isis No. 628785 performed similarly to WT mice in the rotarod test 3-4 weeks after completion of the infusion. By 6-7 weeks after completion of the infusion, the hypoactivity, anxiety-like behaviors and social behavior of the TG mice were reversed, as evidenced by the open field, three-chamber, and elevated plus maze tests.

### Example 6: Dose response of antisense oligonucleotide targeting human MECP2 in patient cells

In order to test for a dose dependent effect of Isis No. 628785 on human cells, B-lymphoblast cells from two individuals affected with *MECP*2-duplication symdrome and age-matched control cells were cultured in suspension in RPMI 1640 medium with L-glutamine, penicillin-streptomycin, and 10% (v/v) fetal bovice serum. A day before transfection, cells were seeded in triplicate for each treatment in 6-well plates at 10⁶ cells per well in a total volume of 2 mL medium. Cells were transfected with Isis No. 628785 or control oligonucleotide at a concentration listed in Table 13 below with TurboFect transfection reagent (Thermo Scientific, Carlsbad, CA). Cells were harvested and RNA was extracted 48 hours after transfection, and MECP2 mRNA levels were analyzed as described in Example 1. Results are presented in Table 13 below as average normlized MECP2 mRNA levels for both patients' cells relative to untreated control cells. The results show that Isis No. 628785 inhibited MECP2 expression in human MECP2 duplication patient cells.

**Table 13: Antisense oligonucleotide treatment of patient lymphoblasts**

| Cell type/Isis No. | Concentration (nM) | Total relative MECP2 mRNA |
|---|---|---|
| Control/Control | 600 | 1.0 |
| Patient/Control | 600 | 3.1 |
| Patient/628785 | 150 | 2.2 |
| Patient/628785 | 300 | 1.6 |
| Patient/628785 | 600 | 1.3 |

### Example 7: Reduction of seizure activity with an antisense oligonucleotide targeting human MECP2 in vivo

Without treatment, seizures and accompanying abnormal electrographic discharges occur in MECP2-TG1 mice as they age. In order to test the effect of antisense oligonucleotide treatment on seizure activity in MECP2-TG1 mice, electrocephalography recordings were performed and behavioral seizure activity was observed.

25-35 week old MECP2-TG1 mice that had been treated as described in Example 4 were anaesthetized with isoflurane and mounted in a stereotaxic frame for the surgical implantation of three recording electrodes (Teflon-coated silver wire, 125 µm in diameter) in the subdural space of the left frontal cortex, the left parietal cortex, and the right parietal cortex, with a reference electrode placed in the occipital region of the skull. After 3-5 days of surgical recovery, cortical EEG activity and behavior were recorded for 2h per day over 3-5 days. Strong electrographic seizure events were typically accompanied by behavioral seizures. Figure 1 displays representative EEG traces for WT mice, MECP2-TG1 mice without Isis No. 628785 treatment, and MECP2-TG1 mice that received treatment with Isis No. 628785. Treatment of MECP2-TG1 mice with Isis No. 628785 eliminated both behavioral seizures and abnormal EEG discharges.

### EMBODIMENTS

What is embodied is:
1. A compound, comprising a modified antisense oligonucleotide consisting of 12 to 30 linked nucleosides and having a nucleobase sequence comprising at least 8, at least 9, at least 10, at least 11, at least 12, at least 13, at least 14, at least 15, at least 16, at least 17, at least 18, at least 19, or at least 20 consecutive nucleobases of any of the nucleobase sequences of SEQ ID NOs: 16-327.
2. The compound of embodiment 2, wherein the nucleobase sequence of the modified antisense oligonucleotide is at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% complementary to SEQ ID NO: 1 or SEQ ID NO: 2.
3. The compound of any preceding embodiment, consisting of a single-stranded modified antisense oligonucleotide.
4. The compound of any preceding embodiment, wherein at least one internucleoside linkage is a modified internucleoside linkage.
5. The compound of embodiment 4, wherein at least one modified internucleoside linkage is a phosphorothioate internucleoside linkage.
6. The compound of embodiment 4, wherein each modified internucleoside linkage is a phosphorothioate internucleoside linkage.
7. The compound of any preceding embodiment, wherein at least one internucleoside linkage is a phosphodiester internucleoside linkage.
8. The compound of any preceding embodiment, wherein at least one internucleoside linkage is a phosphorothioate linkage and at least one internucleoside linkage is a phosphodiester linkage.
9. The compound of any preceding embodiment, wherein at least one nucleoside comprises a modified nucleobase.
10. The compound of embodiment 9, wherein the modified nucleobase is a 5-methylcytosine.
11. The compound of any preceding embodiment, wherein at least one nucleoside of the modified antisense oligonucleotide comprises a modified sugar.
12. The compound of embodiment 11, wherein the at least one modified sugar is a bicyclic sugar.
13. The compound of embodiment 12, wherein the bicyclic sugar comprises a 4'-CH(R)-O-2' bridge wherein R is, independently, H, C₁-C₁₂ alkyl, or a protecting group.
14. The compound of embodiment 13, wherein R is methyl.
15. The compound of embodiment 13, wherein R is H.
16. The compound of embodiment 11, wherein the at least one modified sugar comprises a 2'-O-methoxyethyl group.
17. The compound of any preceding embodiment, wherein the modified antisense oligonucleotide comprises:
   a gap segment consisting of 10 linked deoxynucleosides;
   a 5' wing segment consisting of 5 linked nucleosides; and
   a 3' wing segment consisting of 5 linked nucleosides;
   wherein the gap segment is positioned between the 5' wing segment and the 3' wing segment and wherein each nucleoside of each wing segment comprises a modified sugar.
18. The compound of any preceding embodiment, wherein the modified antisense oligonucleotide consists of 20 linked nucleosides.
19. A composition comprising the compound of any preceding embodiment or salt thereof and at least one of a pharmaceutically acceptable carrier or diluent.
20. A method comprising administering to an animal the compound or composition of any preceding embodiment.
21. The method of embodiment 20, wherein the animal is a human.
22. The method of embodiment 20, wherein administering the compound prevents, treats, ameliorates, or slows progression of a MECP2 associated disorder or syndrome.
23. The method of embodiment 22, wherein the disease, disorder or condition is MECP2 duplication syndrome.
24. Use of the compound or composition of any preceding embodiment for the manufacture of a medicament for treating a neurological disorder.

## Claims

1. A compound comprising a modified antisense oligonucleotide consisting of 12 to 30 linked nucleosides and having a nucleobase sequence at least 70% complementary to nucleobases 7300-7418 of SEQ ID NO: 1.

2. A compound comprising a modified antisense oligonucleotide consisting of 12 to 30 linked nucleosides and having a nucleobase sequence comprising at least 8, at least 9, at least 10, at least 11, at least 12, at least 13, at least 14, at least 15, at least 16, at least 17, at least 18, at least 19, or at least 20 consecutive nucleobases of any of the nucleobase sequences of SEQ ID NO: 257, 22-24, 100-102, 177-179, 256, 258.

3. The compound of claim 1 or claim 2, consisting of a single-stranded modified antisense oligonucleotide.

4. The compound of any preceding claim, wherein the nucleobase sequence of the modified antisense oligonucleotide is at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% complementary to SEQ ID NO: 1 or SEQ ID NO: 2.

5. The compound of any preceding claim, wherein at least one internucleoside linkage is a modified internucleoside linkage, wherein optionally (a) at least one modified internucleoside linkage is a phosphorothioate internucleoside linkage; or (b) each modified internucleoside linkage is a phosphorothioate internucleoside linkage.

6. The compound of any preceding claim, wherein:
(a) at least one internucleoside linkages is a phosphodiester linkage; and/or
(b) at least one internucleoside linkage is a phosphorothioate linkage and at least one internucleoside linkage is a phosphodiester linkage.

7. The compound of any preceding claim, wherein at least one nucleoside comprises a modified nucleobase, wherein optionally the modified nucleobase is a 5-methylcytosine.

8. The compound of any preceding claim, wherein at least one nucleoside of the modified antisense oligonucleotide comprises a modified sugar.

9. The compound of claim 8, wherein:
(a) the at least one modified sugar is a bicyclic sugar, wherein optionally the bicyclic sugar comprises a 4'-CH(R)-O-2' bridge wherein R is, independently, H, C₁-C₁₂ alkyl, or a protecting group, preferably methyl or H;
(b) the at least one modified sugar comprises a 5'-vinyl, 5'-methyl (R or S), 4'-S, 2'-F, 2'-OCH₃, 2'-OCH₂CH₃, or 2'-OCH₂CH₂F substituent group; or
(c) the at least one modified sugar comprises a 2'-O-methoxyethyl substituent group.

10. The compound of any preceding claim, wherein the modified antisense oligonucleotide consists of 20 linked nucleosides.

11. A composition comprising the compound of any preceding claim or salt thereof and at least one pharmaceutically acceptable carrier or diluent.
